(19) 

**Europäisches Patentamt**
**European Patent Office**
**Office européen des brevets**

(11) **EP 4 467 562 A1**

(12) **EUROPEAN PATENT APPLICATION**
published in accordance with Art. 153(4) EPC

(43) Date of publication:
**27.11.2024   Bulletin 2024/48**

(21) Application number: **23742959.2**

(22) Date of filing: **19.01.2023**

(51) International Patent Classification (IPC):
*C07K 14/00* (2006.01)      *C07K 5/00* (2006.01)
*C07K 7/00* (2006.01)       *C07K 11/00* (2006.01)
*A61K 38/00* (2006.01)      *A61P 1/00* (2006.01)
*A61P 1/04* (2006.01)

(52) Cooperative Patent Classification (CPC):
**A61K 38/00; A61P 1/00; A61P 1/04; C07K 5/00;
C07K 7/00; C07K 11/00; C07K 14/00**

(86) International application number:
**PCT/CN2023/073068**

(87) International publication number:
**WO 2023/138644 (27.07.2023 Gazette 2023/30)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **21.01.2022   CN 202210070418**

(71) Applicant: **Sichuan Gooddoctor Panxi
Pharmaceutical Co. Ltd
Sichuan 615000 (CN)**

(72) Inventor: **GENG, Funeng
Xichang City 615000 (CN)**

(74) Representative: **Mewburn Ellis LLP
Aurora Building
Counterslip
Bristol BS1 6BX (GB)**

(54) **POLYPEPTIDE COMPOUND AND USE THEREOF IN TREATMENT OF ENTERITIS**

(57)     Provided is a new polypeptide. The new polypeptide shows a significant effect of relieving intestinal lumen distension in pharmacodynamic tests of a Brachydanio Rerio inflammatory bowel disease model and a rat inflammatory bowel disease model, and can be used for preparing a drug for treating enteritis, especially ulcerative colitis.

EP 4 467 562 A1

**Description**

**FIELD OF THE INVENTION**

The present invention relates to a novel polypeptide and use thereof. The polypeptide of the present invention has a significant therapeutic effect on inflammatory bowel disease, especially has a therapeutic effect on ulcerative colitis.

**BACKGROUND OF THE INVENTION**

Enteritis is an inflammatory response of the intestine caused by various reasons, such as infection by intestinal bacteria, viruses and other pathogenic microorganisms, or immune damage, radiation damage, dietary stimulation, drug stimulation and other factors. Its main clinical manifestations include fever, nausea, vomiting, abdominal pain, diarrhea, watery stools or mucus, pus and blood in the stools, and some patients may also have a feeling of tenesmus.

Enteritis can be divided into specific enteritis and nonspecific enteritis according to the manifestations of inflammation. Specific enteritis includes inflammatory bowel disease, necrotizing enteropathy and dysbacteriosis enteritis. Among them, inflammatory bowel disease, as a more common one, is an idiopathic, non-infectious, intestinal, chronic, persistent, recurrent, and inflammatory disease that involves the intestines. Its clinical manifestations are abdominal pain and diarrhea, pus and blood stool, and even fever, abdominal mass, emaciation and malnutrition. Inflammatory bowel disease is a chronic, long-term disease that requires long-term active treatment.

Generally, specific enteritis has a clear cause, such as bacterial enteritis, pseudomembranous enteritis, viral enteritis, radiation enteritis and the like, which are all enteritis with clear causes, while non-specific enteritis has no clear cause and is considered to be caused by immune system diseases, such as ulcerative colitis, Crohn's disease and the like. Inflammatory bowel disease includes ulcerative colitis and Crohn's disease. Ulcerative colitis is a chronic nonspecific intestinal inflammatory disease with an unclear etiology, which most often occurs in young and middle-aged people. Its clinical manifestations are persistent or recurrent diarrhea, mucus, pus and blood in the stool with abdominal pain, tenesmus and varying degrees of systemic symptoms. There may be manifestations outside the intestines, such as in skin, mucous membranes, joints, eyes, liver, gallbladder and the like. Complications include toxic megacolon, intestinal perforation, massive lower gastrointestinal bleeding, intraepithelial neoplasia, and carcinoma. There is a lack of accurate data on the morbidity of UC in China. Regional epidemiological surveys suggest that the morbidity of UC in China is 0.42-2.22/100,000. Although the morbidity is still low compared with Western countries, it has shown a clear upward trend compared with the last two decades. The lesions mainly affect the colon mucosa and submucosa, starting from the colon distal to the rectum, developing retrogradely to the proximal end, and even affecting the entire colon. In 5% of cases, the lesions may affect the terminal ileum, showing a continuous distribution. The main clinical manifestations of ulcerative colitis are diarrhea, abdominal pain, and mucus, pus and blood in the stool. The cause of the disease is not yet fully understood, but it is currently believed to be related to infection, immune abnormalities, genetics and mental factors. In addition, patients with enteritis usually have symptoms such as intestinal lumen dilatation (also known as intestinal dilatation or intestinal canal dilatation), the cause of which may be that inflammation destroys the nerve and muscle regulation mechanism that controls the normal intestine, and the pressure in the intestinal lumen causes the intestinal wall to expand beyond its normal range of motion. Alternatively, it may be caused by excessive growth of microorganisms and the toxins they produce, mucus exudation, etc. Relieving or inhibiting intestinal lumen intestinal lumen dilatation is helpful in the treatment of enteritis.

Although the drugs currently commonly used to treat ulcerative colitis have certain preventive and therapeutic effects, they still have disadvantages such as slow absorption, long treatment cycle, and unclear treatment effect.

**SUMMARY OF THE INVENTION**

In order to overcome the deficiencies and defects of the prior art, an objective of the present invention is to provide a new polypeptide compound and use thereof. The inventors of the present invention have found that the polypeptide compound of the present invention can effectively or significantly alleviate or inhibit intestinal lumen dilatation, thereby playing a role in treating enteritis.

In a first aspect, the present invention relates to a compound of formula (I) or a physiologically compatible salt thereof, wherein the compound of formula (I) is as follows:

$$\text{H-}X_a\text{-}Z_1\text{-}Z_2\text{-}Z_3\text{-}Z_4\text{-}X_b\text{-OH} \qquad (I)$$

wherein

$Z_1$ is Pro, Ala, Gly, D-Pro or is absent;
$Z_2$ is Val, Ala, D-Val, Ile or is absent;

$Z_3$ is Pro, Ala, Gly, D-Pro or is absent; and

$Z_4$ is Gln, Ala, Glu, Ile, D-Gln or is absent;

provided that at most 2 of $Z_1$, $Z_2$, $Z_3$ and $Z_4$ are absent.

$X_a$ is a sequence containing 0-10 amino acid residues; and

$X_b$ is a sequence containing 0-9 amino acid residues.

In one embodiment, 2 of $Z_1$, $Z_2$, $Z_3$ and $Z_4$ are absent. In one embodiment, $Z_1$ and $Z_2$ are absent. In one embodiment, $Z_3$ and $Z_4$ are absent. In one embodiment, 1 of $Z_1$, $Z_2$, $Z_3$ and $Z_4$ is absent. In one embodiment, $Z_1$ is absent. In one embodiment, $Z_4$ is absent. In one embodiment, 0 of $Z_1$, $Z_2$, $Z_3$ and $Z_4$ is absent.

In one embodiment, $Z_1$ is Pro; $Z_2$ is Val; $Z_3$ is Pro; and $Z_4$ is Gln. In one embodiment, $Z_1$ is D-Pro; $Z_2$ is Val; $Z_3$ is Pro; and $Z_4$ is Gln. In one embodiment, $Z_1$ is Pro; $Z_2$ is Val; $Z_3$ is Ala; and $Z_4$ is Gln. In one embodiment, $Z_1$ is Pro; $Z_2$ is Ile; $Z_3$ is Pro; and $Z_4$ is Gln. In one embodiment, $Z_1$ is Pro; $Z_2$ is Val; $Z_3$ is Pro; and $Z_4$ is absent. In one embodiment, $Z_1$ is Pro; $Z_2$ is Ala; $Z_3$ is Pro; and $Z_4$ is Gln. In one embodiment, $Z_1$ is Gly; $Z_2$ is Val; $Z_3$ is Pro; and $Z_4$ is Gln. In one embodiment, $Z_1$ is Pro; $Z_2$ is Val; $Z_3$ is Gly; and $Z_4$ is Gln. In one embodiment, $Z_1$ is Ala; $Z_2$ is Val; $Z_3$ is Pro; and $Z_4$ is Gln. In one embodiment, $Z_1$ is Pro; $Z_2$ is Val; $Z_3$ is Pro; and $Z_4$ is Glu. In one embodiment, $Z_1$ is Pro; $Z_2$ is Val; $Z_3$ is D-Pro; and $Z_4$ is Gln. In one embodiment, $Z_1$ is Pro; $Z_2$ is Val; $Z_3$ is Pro; and $Z_4$ is D-Gln.

In one embodiment, $X_a$ is $X_{a10}$-$X_{a9}$-$X_{a8}$-$X_{a7}$-$X_{a6}$-$X_{a5}$-$X_{a4}$-$X_{a3}$-$X_{a2}$-$X_{a1}$-*, wherein * indicates the position linked to $Z_1$-$Z_2$-$Z_3$-$Z_4$, wherein

$X_{a10}$ is Gly or is absent,

$X_{a9}$ is Gly, Pro, Ser or is absent,

$X_{a8}$ is Pro, Glu, Ser or is absent,

$X_{a7}$ is Arg, Glu, Thr, Ser or is absent,

$X_{a6}$ is Lys, Thr, Ala, Asp, Glu, Arg, Ser or is absent,

$X_{a5}$ is Asp, Ala, Val, Arg, Phe, Ile, Pro, Lys, Glu or is absent,

$X_{a4}$ is Val, Phe, Pro, Pyro-Glu, Lys, Leu, Ile, Ala, Asp or is absent,

$X_{a3}$ is Tyr, Leu, Pro, Asp, Arg, Glu, Lys, Tys, Val, Ile or is absent,

$X_{a2}$ is D-Lys, Lys, Ala, Arg, Val, Glu, Tyr or is absent, and

$X_{a1}$ is Glu, Ala, D-Glu, Tyr, Gln, Asp, Asn or is absent.

In one embodiment, $X_a$ is $X_{aa}$-$X_{a2}$-$X_{a1}$-*, wherein * indicates the position linked to $Z_1$-$Z_2$-$Z_3$-$Z_4$, $X_{a2}$-$X_{a1}$ is Lys-Glu-*, Arg-Glu-*, Val-Tyr-*, Glu-Glu-*, Lys-Gln-*, Lys-D-Glu-* or Tyr-Glu-*, and $X_{aa}$ is $X_{a10}$-$X_{a9}$-$X_{a8}$-$X_{a7}$-$X_{a6}$-$X_{a5}$-$X_{a4}$-$X_{a3}$-, wherein $X_{a10}$ is Gly or is absent, $X_{a9}$ is Gly, Pro, Ser or is absent, $X_{a8}$ is Pro, Glu, Ser or is absent, $X_{a7}$ is Arg, Glu, Thr, Ser or is absent, $X_{a6}$ is Lys, Thr, Ala, Asp, Glu, Arg, Ser or is absent, $X_{a5}$ is Asp, Ala, Val, Arg, Phe, Ile, Pro, Lys, Glu or is absent, $X_{a4}$ is Val, Phe, Pro, Pyro-Glu, Lys, Leu, Ile, Ala, Asp or is absent, and $X_{a3}$ is Tyr, Leu, Pro, Asp, Arg, Glu, Lys, Tys, Val, Ile or is absent. In one embodiment, $X_{aa}$ is Arg-Lys-Asp-Val-Tyr-, Gly-Pro-Glu-Thr-Ala-Phe-Leu-, Val-Pro-Pro-, Pyro-Glu-Leu-, Arg-Lys-Asp-, Gly-Pro-Glu-Thr-Ala-Phe-Leu-Arg-, Ser-Ser-Glu-Asp-Ile-Lys-Glu-, Ser-Ser-Glu-Asp-Ile-Lys-, Val-Pro-Tys-, Pro-Ala-Tys-, Arg-Lys-Asp-Val-, Ser-Ser-Glu-Asp-Ile-, or is absent, wherein the rightmost connector - indicates linkage to -$X_{a2}$-$X_{a1}$. In one embodiment, $X_a$ is $X_{aa}$-$X_{a2}$-$X_{a1}$-*, wherein * indicates the position linked to $Z_1$-$Z_2$-$Z_3$-$Z_4$, wherein $X_{a2}$-$X_{a1}$ is Lys-Glu-*, and $X_{aa}$ is as defined above.

In one embodiment, $X_a$ is $X_{a1}$-*, wherein * indicates the position linked to $Z_1$-$Z_2$-$Z_3$-$Z_4$, wherein $X_{a1}$ is Glu, Ala, D-Glu, Asp, Asn or is absent.

In one embodiment, $X_a$ is $X_{a2}$-$X_{a1}$-*, wherein * indicates the position linked to $Z_1$-$Z_2$-$Z_3$-$Z_4$, wherein $X_{a2}$ is Lys, and $X_{a1}$ is Glu, D-Glu or Gln.

In one embodiment, $X_a$ is Glu-*, Ala-*, D-Glu-*, Asp-*, Asn-*, Lys-Glu-*, D-Lys-Glu-*, Ala-Glu-*, Lys-Gln-*, Lys-D-Glu-*, Lys-Ala-*, Arg-Lys-Asp-Val-Tyr-Lys-Glu-*, Gly-Pro-Glu-Thr-Ala-Phe-Leu-Arg-Glu-*, Val-Pro-Pro-Lys-Glu-*, Pyro-Glu-Leu-Lys-Glu-*, Arg-Lys-Asp-Val-Tyr-Lys-Glu-*, yr-*, Gly-Pro-Glu-Thr-Ala-Phe-Leu-Arg-Lys-Glu-*, Ser-Ser-Glu-Asp-Ile-Lys-Glu-Lys-Glu-*, Ser-Ser-Glu-Asp-Ile-Lys-Glu-Glu-*, Val-Pro-Tys-Lys-Glu-*, Pro-Ala-Tys-Lys-Glu-*, Arg-Lys-Asp-Val-Tyr-Glu-*, Ser-Ser-Glu-Asp-Ile-Lys-Glu-* or is absent, wherein * indicates the position linked to $Z_1$-$Z_2$-$Z_3$-$Z_4$.

In one embodiment, $X_b$ is **-$X_{b1}$-$X_{b2}$-$X_{b3}$-$X_{b4}$-$X_{b6}$-$X_{b6}$-$X_{b7}$-$X_{b8}$-$X_{b9}$, wherein ** indicates the position linked to $Z_1$-$Z_2$-$Z_3$-$Z_4$, wherein

$X_{b1}$ is Ala, D-Ala or is absent,

$X_{b2}$ is Lys, Ala, D-Lys, Arg or is absent,

$X_{b3}$ is Pro, Gly, D-Pro, Ser, Val, Ala or is absent,

$X_{b4}$ is Arg, Ala, Ser, Pro, D-Arg or is absent,

$X_{b5}$ is Lys, Ala, D-Lys, Glu, Tyr or is absent,

$X_{b6}$ is Val, Asp, D-Val, Ala or is absent,

$X_{b7}$ is Ala, D-Ala, Ile or is absent,
$X_{b8}$ is Ala, D-Ala, Lys or is absent, and
$X_{b9}$ is Gln, Ala, Glu, Asn, D-Gln or is absent.

In one embodiment, $X_b$ is **-$X_{b1}$-$X_{b2}$-$X_{b3}$-$X_{b4}$-$X_{b5}$-$X_{b6}$-$X_{b7}$-$X_{b8}$-$X_{b9}$, wherein ** indicates the position linked to $Z_1$-$Z_2$-$Z_3$-$Z_4$, wherein

$X_{b1}$ is Ala or D-Ala,
$X_{b2}$ is Lys, Ala or D-Lys,
$X_{b3}$ is Pro, Gly, or Ala,
$X_{b4}$ is Arg or D-Arg,
$X_{b5}$ is Lys or is absent,
$X_{b6}$ is Val, D-Val, Ala or is absent,
$X_{b7}$ is Ala is absent,
$X_{b8}$ is Ala or is absent, and
$X_{b9}$ is Gln, Asn, D-Gln or is absent.

In one embodiment, $X_b$ is **-$X_{b1}$-$X_{b2}$-$X_{bb}$, wherein ** indicates the position linked to $Z_1$-$Z_2$-$Z_3$-$Z_4$, $X_{b1}$-$X_{b2}$-is **-Ala-Lys-, **-Ala-Lys-, **-Ala-D-Lys-, **-Ala-Ala- or **-D-Ala-Lys-, $X_{bb}$ is -$X_{b3}$-$X_{b4}$-$X_{b5}$-$X_{b6}$-$X_{b7}$-$X_{b8}$-$X_{b9}$, wherein $X_{b3}$ is Pro, Gly, D-Pro, Ser, Val, Ala or is absent, $X_{b4}$ is Arg, Ala, Ser, Pro, D-Arg or is absent, $X_{b5}$ is Lys, Ala, D-Lys, Glu, Tyr or is absent, $X_{b6}$ is Val, Asp, D-Val, Ala or is absent, $X_{b7}$ is Ala, D-Ala, Ile or is absent, $X_{b8}$ is Ala, D-Ala, Lys or is absent, and $X_{b9}$ is Gln, Ala, Glu, Asn, D-Gln or is absent; or $X_{b3}$ is Pro, Gly, or Ala, $X_{b4}$ is Arg or D-Arg, $X_{b5}$ is Lys or is absent, $X_{b6}$ is Val, D-Val, Ala or is absent, $X_{b7}$ is Ala or is absent, $X_{b8}$ is Ala or is absent, and $X_{b9}$ is Gln, Asn, D-Gln or is absent. In one embodiment, $X_{bb}$ is -Pro-Arg-Lys-Val, -Pro-Arg-Lys, -Pro-Arg, -Ala-Gln, -Ala-Ala, -Pro, -Lys-Val, -Val-Pro-Tyr, -Arg-Lys, -Val-Ala, -Pro-Arg-Lys-Val-Ala, -Lys-Val-Ala-Ala-Gln, -Pro-Arg-Lys-Val-Ala-Ala-Gln, -Pro-A rg-Lys-Val-Ala-Ala-Gln, -Pro-Ala-Lys-Val-Ala-Ala-Gln, -Pro-Arg-Ala-Val-Ala-Ala-Gln, -Pro-Arg-Lys-Val-Ala-Ala-Ala, -Gly-Arg-Lys-Val-Ala-Ala-Gln, -D-Pro-Arg-Lys-Val-Ala-Ala-Gln, -Pro-Arg-D-Lys-Val-Ala-Ala-Gln, -Pro-Arg-Lys-Val-D-Ala-Ala-Gln, -Pro-Arg-Lys-Val-Ala-D-Ala-Gln, -Ser-Ser-Glu-Asp-Ile-Lys-Glu, -Pro-Arg-Lys-Val-Ala-Ala-Asn, -Pro-Arg-Lys-Val-Ala-Ala-Gln, - Pro-Arg-Lys-D-Val-Ala-Ala-Gln, -Ala-Arg-Lys-Val-Ala-Ala-Gln, -Pro-Arg-Lys-Ala-Ala-Ala-Gln, -Pro-Arg-Lys-Val-Ala-Ala-Gln, -Pro-D-Arg-Lys-Val-Ala-Ala-Gln, -Pro-Arg-Lys-Val-Ala-Ala-D-Gln or is absent, wherein the leftmost connector - indicates linkage to $X_{b1}$-$X_{b2}$. In one embodiment, $X_b$ is **-$X_{b1}$-$X_{b2}$-$X_{bb}$, wherein ** represents the position linked to $Z_1$-$Z_2$-$Z_3$-$Z_4$, $X_{b1}$-$X_{b2}$- is **-Ala-Lys-, and $X_{bb}$ is as defined above.

In one embodiment, $X_b$ is **-Ala-.

In one embodiment, $X_b$ is **-Ala-, **-Ala-Lys, **-Ala-D-Lys-, **-Ala-Ala-, **-D-Ala-Lys, **-Ala-Arg, **-Ala-Lys-Pro-Arg-Lys-Val, **-Ala-Lys-Pro-Arg-Lys, **-Ala-Lys-Pro-Arg, **-Val-Ala-Ala-Gln, **-Lys-Val-Ala-Ala, **-Ala-Lys-Pro, **-Pro-Arg-Lys-Val, **-Ala-Lys-Val-Pro-Tyr, **-Lys-Pro-Arg-Lys, **-Arg-Lys-Val-Ala, **- Ala-Lys-Pro-Arg-Lys-Val-Ala, **-Pro-Arg-Lys-Val-Ala-Ala-Gln, **-Ala-Lys-Pro-Arg-Lys-Val-Ala-Ala-Gln, **-Ala-Ala-Pro-Arg-Lys-Val-Ala-Ala-Gln, **-Ala-Lys-Pro-Ala-Lys-Val-Ala-Ala-Gln, **-Ala-Lys-Pro-Arg-Ala-Val-Ala-Ala-Gln, **-Ala-Lys-Pro-Arg-Lys-Val-Ala-Ala-Ala, **-Ala-Lys-Gly-Arg-Lys-Val-Ala-Ala-Gln, **-Ala-Lys-D-Pro-Arg-Lys-Val-Ala-Ala-Gln, **-Ala-Lys-Pro-Arg-D-Lys-Val-Ala-Ala-Gln, **-Ala-Lys-Pro-Arg-Lys-Val-D-Ala-Ala-Gln, **-Ala-Lys-Pro-Arg-Lys-Val-Ala-D-Ala-Gln, **-Ala-Lys-Ser-Ser-Glu-Asp-Ile-Lys-Glu, **-Ala-Lys-Pro-Arg-Lys-Val-Ala-Ala-Asn, **-Ala-D-Lys-Pro- Arg-Lys-Val-Ala-Ala-Gln, **-Ala-Lys-Pro-Arg-Lys-D-Val-Ala-Ala-Gln, **-Ala-Lys-Ala-Arg-Lys-Val-Ala-Ala-Gln, **-Ala-Lys-Pro-Arg-Lys-Ala-Ala-Ala-Gln, **-D-Ala-Lys-Pro-Arg-Lys-Val-Ala-Ala-Gln, **-Ala-Lys-Pro-D-Arg-Lys-Val-Ala-Ala-Gln, **-Ala-Lys-Pro-Arg-Lys-Val-Ala-Ala-D-Gln, or is absent, wherein ** indicates the position linked to $Z_1$-$Z_2$-$Z_3$-$Z_4$.

In one embodiment, $Z_1$ is Pro; $Z_2$ is Val; $Z_3$ is Pro; and $Z_4$ is Gln; $X_a$ is $X_{aa}$-$X_{a2}$-$X_{a1}$-*, wherein * indicates the position linked to $Z_1$-$Z_2$-$Z_3$-$Z_4$, wherein $X_{a2}$-$X_{a1}$ is Lys-Glu-*, and $X_b$ is **-$X_{b1}$-$X_{b2}$-$X_{bb}$, wherein ** indicates the position linked to $Z_1$-$Z_2$-$Z_3$-$Z_4$, $X_{b1}$-$X_{b2}$- is **-Ala-Lys-, wherein $X_{aa}$ and $X_{bb}$ are as defined above.

In one embodiment, the compound is selected from:

Lys-Glu-Pro-Val-Pro-Gln-Ala-Lys-Pro-Arg-Lys-Val-Ala-Ala-Gln (Compound 1);

Lys-Glu-Pro-Val-Pro-Gln-Ala-Lys-Pro-Arg-Lys (Compound 2);

Lys-Glu-Pro-Val-Pro-Gln-Ala-Lys-Pro-Arg (Compound 3);

Lys-Glu-Pro-Val-Pro-Gln-Ala-Lys (Compound 4);

Pro-Arg-Lys-Val-Ala-Ala-Gln (Compound 5);

Glu-Pro-Val-Pro-Gln-Ala-Lys           (Compound 6);

Lys-Glu-Pro-Val          (Compound 7);

Val-Ala-Ala-Gln          (Compound 8);

Glu-Pro-Val-Pro          (Compound 9);

Lys-Pro-Arg-Lys          (Compound 10);

Ala-Lys-Pro-Arg          (Compound 11);

Pro-Val-Pro-Gln          (Compound 12);

Val-Pro-Gln-Ala          (Compound 13);

Pro-Gln-Ala-Lys          (Compound 14);

Arg-Lys-Val-Ala          (Compound 15);

Lys-Val-Ala-Ala          (Compound 16);

Gln-Ala-Lys-Pro          (Compound 17);

Pro-Arg-Lys-Val          (Compound 18);

Ala-Glu-Pro-Val-Pro-Gln-Ala-Lys-Pro-Arg-Lys-Val-Ala-Ala-Gln          (Compound 19);

Lys-Ala-Pro-Val-Pro-Gln-Ala-Lys-Pro-Arg-Lys-Val-Ala-Ala-Gln          (Compound 20);

Lys-Glu-Ala-Val-Pro-Gln-Ala-Lys-Pro-Arg-Lys-Val-Ala-Ala-Gln          (Compound 21);

Lys-Glu-Pro-Val-Ala-Gln-Ala-Lys-Pro-Arg-Lys-Val-Ala-Ala-Gln          (Compound 22);

Lys-Glu-Pro-Val-Pro-Ala-Ala-Lys-Pro-Arg-Lys-Val-Ala-Ala-Gln          (Compound 23);

Lys-Glu-Pro-Val-Pro-Gln-Ala-Ala-Pro-Arg-Lys-Val-Ala-Ala-Gln          (Compound 24);

Lys-Glu-Pro-Ala-Pro-Gln-Ala-Lys-Pro-Arg-Lys-Val-Ala-Ala-Gln          (Compound 25);

Lys-Glu-Pro-Val-Pro-Gln-Ala-Lys-Ala-Arg-Lys-Val-Ala-Ala-Gln          (Compound 26);

Lys-Glu-Pro-Val-Pro-Gln-Ala-Lys-Pro-Ala-Lys-Val-Ala-Ala-Gln          (Compound 27);

Lys-Glu-Pro-Val-Pro-Gln-Ala-Lys-Pro-Arg-Ala-Val-Ala-Ala-Gln          (Compound 28);

Lys-Glu-Pro-Val-Pro-Gln-Ala-Lys-Pro-Arg-Lys-Ala-Ala-Ala-Gln          (Compound 29);

Lys-Glu-Pro-Val-Pro-Gln-Ala-Lys-Pro-Arg-Lys-Val-Ala-Ala-Ala          (Compound 30);

Lys-Glu-Gly-Val-Pro-Gln-Ala-Lys-Pro-Arg-Lys-Val-Ala-Ala-Gln          (Compound 31);

Lys-Glu-Pro-Val-Gly-Gln-Ala-Lys-Pro-Arg-Lys-Val-Ala-Ala-Gln          (Compound 32);

Lys-Glu-Pro-Val-Pro-Gln-Ala-Lys-Gly-Arg-Lys-Val-Ala-Ala-Gln          (Compound 33);

Lys-Glu-Pro-Val-Gly-Gln-Ala-Lys-Gly-Arg-Lys-Val-Ala-Ala-Gln          (Compound 34);

Lys-Glu-Gly-Val-Gly-Gln-Ala-Lys-Gly-Arg-Lys-Val-Ala-Ala-Gln (Compound 35);

Lys-Glu-Gly-Val-Pro-Gln-Ala-Lys-Gly-Arg-Lys-Val-Ala-Ala-Gln (Compound 36);

Lys-Glu-Gly-Val-Gly-Gln-Ala-Lys-Pro-Arg-Lys-Val-Ala-Ala-Gln (Compound 37);

Glu-Pro-Val-Pro-Gln-Ala-Lys-Pro-Arg-Lys (Compound 38);

Pro-Val-Pro-Gln-Ala (Compound 39);

Pro-Val-Pro-Glu-Ala (Compound 40);

Pro-Val-Pro-Ile-Ala (Compound 41);

Pro-Val-Pro-Ala (Compound 42);

Glu-Pro-Val-Pro-Gln-Ala (Compound 43);

Pro-Val-Pro-Gln-Ala-Lys (Compound 44);

Glu-Pro-Val-Pro-Gln-Ala-Arg (Compound 45);

Ala-Pro-Val-Pro-Gln-Ala-Lys (Compound 46);

Glu-Pro-Val-Pro-Ala-Ala-Lys (Compound 47);

Glu-Pro-Val-Pro-Gln-Ala-Ala (Compound 48);

Asp-Pro-Val-Pro-Gln-Ala-Lys (Compound 49);

Asn-Pro-Val-Pro-Gln-Ala-Lys (Compound 50);

Glu-Pro-Val-Pro-Gln-Ala-Lys-Pro-Arg-Lys-Val-Ala (Compound 51);

Glu-Pro-Ala-Pro-Gln-Ala-Lys (Compound 52);

Glu-Ala-Val-Pro-Gln-Ala-Lys (Compound 53);

Glu-Pro-Val-Ala-Gln-Ala-Lys (Compound 54);

Glu-Pro-Val-Pro-Glu-Ala-Lys (Compound 55);

Glu-Pro-Val-Pro-Gln-Ala-Lys-Pro-Arg-Lys-Val (Compound 56);

Glu-Pro-Val-Pro-Gln-Ala-Lys-Pro-Arg (Compound 57);

Glu-Pro-Val-Pro-Gln-Ala-Lys-Pro (Compound 58);

Pro-Val-Pro-Gln-Ala-Lys-Pro-Arg (Compound 59);

Pro-Val-Pro-Gln-Ala-Lys-Pro (Compound 60);

D-Lys-Glu-Pro-Val-Pro-Gln-Ala-Lys-Pro-Arg-Lys-Val-Ala-Ala-Gln (Compound 61);

Lys-D-Glu-Pro-Val-Pro-Gln-Ala-Lys-Pro-Arg-Lys-Val-Ala-Ala-Gln (Compound 62);

Lys-Glu-D-Pro-Val-Pro-Gln-Ala-Lys-Pro-Arg-Lys-Val-Ala-Ala-Gln (Compound 63);

Lys-Glu-Pro-D-Val-Pro-Gln-Ala-Lys-Pro-Arg-Lys-Val-Ala-Ala-Gln          (Compound 64);

Lys-Glu-Pro-Val-D-Pro-Gln-Ala-Lys-Pro-Arg-Lys-Val-Ala-Ala-Gln          (Compound 65);

Lys-Glu-Pro-Val-Pro-D-Gln-Ala-Lys-Pro-Arg-Lys-Val-Ala-Ala-Gln          (Compound 66);

Lys-Glu-Pro-Val-Pro-Gln-D-Ala-Lys-Pro-Arg-Lys-Val-Ala-Ala-Gln          (Compound 67);

Lys-Glu-Pro-Val-Pro-Gln-Ala-D-Lys-Pro-Arg-Lys-Val-Ala-Ala-Gln          (Compound 68);

Lys-Glu-Pro-Val-Pro-Gln-Ala-Lys-D-Pro-Arg-Lys-Val-Ala-Ala-Gln          (Compound 69);

Lys-Glu-Pro-Val-Pro-Gln-Ala-Lys-Pro-D-Arg-Lys-Val-Ala-Ala-Gln          (Compound 70);

Lys-Glu-Pro-Val-Pro-Gln-Ala-Lys-Pro-Arg-D-Lys-Val-Ala-Ala-Gln          (Compound 71);

Lys-Glu-Pro-Val-Pro-Gln-Ala-Lys-Pro-Arg-Lys-D-Val-Ala-Ala-Gln          (Compound 72);

Lys-Glu-Pro-Val-Pro-Gln-Ala-Lys-Pro-Arg-Lys-Val-D-Ala-Ala-Gln          (Compound 73);

Lys-Glu-Pro-Val-Pro-Gln-Ala-Lys-Pro-Arg-Lys-Val-Ala-D-Ala-Gln          (Compound 74);

Lys-Glu-Pro-Val-Pro-Gln-Ala-Lys-Pro-Arg-Lys-Val-Ala-Ala-D-Gln          (Compound 75);

Glu-Gly-Val-Pro-Gln-Ala-Lys          (Compound 76);

Glu-Pro-Val-Gly-Gln-Ala-Lys          (Compound 77);

D-Glu-Pro-Val-Pro-Gln-Ala-Lys          (Compound 78);

Glu-D-Pro-Val-Pro-Gln-Ala-Lys          (Compound 79);

Glu-Pro-D-Val-Pro-Gln-Ala-Lys          (Compound 80);

Glu-Pro-Val-D-Pro-Gln-Ala-Lys          (Compound 81);

Glu-Pro-Val-Pro-D-Gln-Ala-Lys          (Compound 82);

Glu-Pro-Val-Pro-Gln-D-Ala-Lys          (Compound 83);

Glu-Pro-Val-Pro-Gln-Ala-D-Lys          (Compound 84);

Lys-Glu-Pro-Val-Pro          (Compound 85);

Glu-Pro-Val-Pro-Gln          (Compound 86);

Lys-Glu-Pro-Val-Pro-Gln          (Compound 87);

Val-Pro-Tyr-Lys-Glu-Pro-Val-Pro-Gln-Ala-Lys-Pro-Arg-Lys-Val-Ala-Ala-Gln          (Compound 88);

Pyro-Glu-Leu-Lys-Glu-Pro-Val-Pro-Gln-Ala-Lys-Pro-Arg-Lys-Val-Ala-Ala-Gln          (Compound 89);

Pro-Ala-Tyr-Lys-Glu-Pro-Val-Pro-Gln-Ala-Lys-Pro-Arg-Lys-Val-Ala-Ala-Gln          (Compound 90);

Arg-Lys-Asp-Val-Tyr-Lys-Glu-Pro-Val-Pro-Gln-Ala-Lys-Pro-Arg-Lys-Val-Ala-Ala-Gln (Compound 91);

Arg-Lys-Asp-Val-Tyr-Lys-Glu-Pro-Val-Pro-Gln-Ala-Lys (Compound 92);

Arg-Lys-Asp-Val-Tyr-Glu-Pro-Val-Pro-Gln-Ala-Lys (Compound 93);

Arg-Lys-Asp-Val-Tyr-Pro-Val-Pro-Gln (Compound 94);

Gly -Pro-Glu- Thr-Ala-Phe-Leu-Arg-L ys-Glu-Pro- V al-Pro-Gln-Ala-L ys-Pro-Arg-L ys- Val-Ala-Ala-Gln (Compound 95);

Gly-Pro-Glu-Thr-Ala-Phe-Leu-Arg-Glu-Pro-Val-Pro-Gln-Ala-Lys (Compound 96);

Ser-Ser-Glu-Asp-Ile-Lys-Glu-Lys-Glu-Pro-Val-Pro-Gln-Ala-Lys-Pro-Arg-Lys-Val-Ala-Ala-Gln (Compound 97);

Ser-Ser-Glu-Asp-Ile-Lys-Glu-Glu-Pro-Val-Pro-Gln-Ala-Lys (Compound 98);

Ser-Ser-Glu-Asp-Ile-Lys-Glu-Pro-Val-Pro-Gln (Compound 99);

Val-Pro-Pro-Lys-Glu-Pro-Val-Pro-Gln-Ala-Lys-Pro-Arg-Lys-Val-Ala-Ala-Gln (Compound 100);

Glu-Pro-Val-Pro-Gln-Ala-Lys-Ser-Ser-Glu-Asp-Ile-Lys-Glu (Compound 101);

Glu-Pro-Val-Pro-Gln-Ala-Lys-Val-Pro-Tyr (Compound 102);

Lys-Glu-Pro-Val-Pro-Gln-Ala-Lys-Pro-Arg-Lys-Val-Ala-Ala-Glu (Compound 103);

Lys-Glu-Pro-Val-Pro-Gln-Ala-Lys-Pro-Arg-Lys-Val-Ala-Ala-Asn (Compound 104);

Lys-Gln-Pro-Val-Pro-Gln-Ala-Lys-Pro-Arg-Lys-Val-Ala-Ala-Gln (Compound 105);

Lys-Glu-Pro-Ile-Pro-Gln-Ala-Lys-Pro-Arg-Lys-Val-Ala-Ala-Gln (Compound 106);

Val-Pro-Gln-Ala (Compound 107);

or

Val-Pro-Gln-Ala-Lys (Compound 108).

In one embodiment, the compound is selected from:

Lys-Glu-Pro-Val-Pro-Gln-Ala-Lys-Pro-Arg-Lys-Val-Ala-Ala-Gln (Compound 1);

Lys-Glu-D-Pro-Val-Pro-Gln-Ala-Lys-Pro-Arg-Lys-Val-Ala-Ala-Gln (Compound 63);

Gly-Pro-Glu-Thr-Ala-Phe-Leu-Arg-Glu-Pro-Val-Pro-Gln-Ala-Lys (Compound 96);

Val-Pro-Pro-Lys-Glu-Pro-Val-Pro-Gln-Ala-Lys-Pro-Arg-Lys-Val-Ala-Ala-Gln (Compound 100);

Lys-Glu-Pro-Val-Pro-Gln-Ala-Lys-Pro-Arg-Lys-Val-Ala-Ala-Asn (Compound 104);

Glu-Pro-Val-Pro-Gln-Ala-Lys (Compound 6);

Pro-Val-Pro-Gln (Compound 12);

Glu-Pro-Val-Ala-Gln-Ala-Lys        (Compound 54);

Glu-Pro-Val-Pro-Gln-Ala-Lys-Pro-Arg        (Compound 57);

Lys-Glu-Pro-Val-Pro-Gln-Ala-D-Lys-Pro-Arg-Lys-Val-Ala-Ala-Gln        (Compound 68);

Lys-Glu-Pro-Val-Pro-Gln-Ala-Lys-Pro-Arg-Lys-D-Val-Ala-Ala-Gln        (Compound 72);

Pyro-Glu-Leu-Lys-Glu-Pro-Val-Pro-Gln-Ala-Lys-Pro-Arg-Lys-Val-Ala-Ala-Gln        (Compound 89);

Arg-Lys-Asp-Val-Tyr-Lys-Glu-Pro-Val-Pro-Gln-Ala-Lys        (Compound 92);

Arg-Lys-Asp-Val-Tyr-Pro-Val-Pro-Gln        (Compound 94);

Gly -Pro-Glu- Thr-Ala-Phe-Leu-Arg-L ys-Glu-Pro- V al-Pro-Gln-Ala-L ys-Pro-Arg-L ys- Val-Ala-Ala-Gln        (Compound 95);

Ser-Ser-Glu-Asp-Ile-Lys-Glu-Lys-Glu-Pro-Val-Pro-Gln-Ala-Lys-Pro-Arg-Lys-Val-Ala-Ala-Gln        (Compound 97);

Ser-Ser-Glu-Asp-Ile-Lys-Glu-Glu-Pro-Val-Pro-Gln-Ala-Lys        (Compound 98);

Lys-Gln-Pro-Val-Pro-Gln-Ala-Lys-Pro-Arg-Lys-Val-Ala-Ala-Gln        (Compound 105);

Lys-Glu-Pro-Ile-Pro-Gln-Ala-Lys-Pro-Arg-Lys-Val-Ala-Ala-Gln        (Compound 106);

Lys-Glu-Pro-Val-Pro-Gln-Ala-Lys        (Compound 4);

Pro-Arg-Lys-Val-Ala-Ala-Gln        (Compound 5);

Glu-Pro-Val-Pro        (Compound 9);

Lys-Pro-Arg-Lys        (Compound 10);

Arg-Lys-Val-Ala        (Compound 15);

Lys-Glu-Pro-Ala-Pro-Gln-Ala-Lys-Pro-Arg-Lys-Val-Ala-Ala-Gln        (Compound 25);

Lys-Glu-Pro-Val-Pro-Gln-Ala-Lys-Ala-Arg-Lys-Val-Ala-Ala-Gln        (Compound 26);

Lys-Glu-Pro-Val-Pro-Gln-Ala-Lys-Pro-Arg-Lys-Ala-Ala-Ala-Gln        (Compound 29);

Lys-Glu-Gly-Val-Pro-Gln-Ala-Lys-Pro-Arg-Lys-Val-Ala-Ala-Gln        (Compound 31);

Lys-Glu-Pro-Val-Gly-Gln-Ala-Lys-Gly-Arg-Lys-Val-Ala-Ala-Gln        (Compound 34);

Glu-Pro-Val-Pro-Gln-Ala-Lys-Pro-Arg-Lys        (Compound 38);

Glu-Pro-Val-Pro-Gln-Ala-Ala        (Compound 48);

Asp-Pro-Val-Pro-Gln-Ala-Lys        (Compound 49);

Asn-Pro-Val-Pro-Gln-Ala-Lys        (Compound 50);

Glu-Pro-Val-Pro-Gln-Ala-Lys-Pro-Arg-Lys-Val-Ala        (Compound 51);

Glu-Ala-Val-Pro-Gln-Ala-Lys          (Compound 53);

Glu-Pro-Val-Pro-Glu-Ala-Lys          (Compound 55);

Lys-D-Glu-Pro-Val-Pro-Gln-Ala-Lys-Pro-Arg-Lys-Val-Ala-Ala-Gln          (Compound 62);

Lys-Glu-Pro-Val-D-Pro-Gln-Ala-Lys-Pro-Arg-Lys-Val-Ala-Ala-Gln          (Compound 65);

Lys-Glu-Pro-Val-Pro-D-Gln-Ala-Lys-Pro-Arg-Lys-Val-Ala-Ala-Gln          (Compound 66);

Lys-Glu-Pro-Val-Pro-Gln-D-Ala-Lys-Pro-Arg-Lys-Val-Ala-Ala-Gln          (Compound 67);

Lys-Glu-Pro-Val-Pro-Gln-Ala-Lys-Pro-D-Arg-Lys-Val-Ala-Ala-Gln          (Compound 70);

Lys-Glu-Pro-Val-Pro-Gln-Ala-Lys-Pro-Arg-Lys-Val-Ala-Ala-D-Gln          (Compound 75);

Glu-Pro-Val-Pro-Gln-D-Ala-Lys          (Compound 83);

Lys-Glu-Pro-Val-Pro          (Compound 85);

Glu-Pro-Val-Pro-Gln          (Compound 86);

Val-Pro-Tyr-Lys-Glu-Pro-Val-Pro-Gln-Ala-Lys-Pro-Arg-Lys-Val-Ala-Ala-Gln          (Compound 88);

Pro-Ala-Tyr-Lys-Glu-Pro-Val-Pro-Gln-Ala-Lys-Pro-Arg-Lys-Val-Ala-Ala-Gln          (Compound 90);

Arg-Lys-Asp-Val-Tyr-Glu-Pro-Val-Pro-Gln-Ala-Lys          (Compound 93);

Ser-Ser-Glu-Asp-Ile-Lys-Glu-Pro-Val-Pro-Gln          (Compound 99);

or

Lys-Glu-Pro-Val-Pro-Gln-Ala-Lys-Pro-Arg-Lys-Val-Ala-Ala-Glu          (Compound 103).

In a second aspect, the present invention provides the use of the polypeptide compound or a physiologically compatible salt thereof in the preparation of a drug for treating enteritis; or a method for treating enteritis, the method comprising administering a therapeutically effective amount of the polypeptide compound or a physiologically compatible salt thereof to a subject; or the polypeptide compound or a physiologically compatible salt thereof for treating enteritis.

Furthermore, enteritis includes specific enteritis and nonspecific enteritis. Furthermore, specific enteritis includes inflammatory bowel disease, necrotizing enteropathy and flora imbalance enteritis.

Furthermore, inflammatory bowel disease includes ulcerative colitis.

Furthermore, the present invention provides a pharmaceutical composition comprising the polypeptide compound of the present invention or a physiologically compatible salt thereof, and a pharmaceutically acceptable excipient.

Furthermore, the dosage forms of the drug include tablets, capsules, solutions, powders and pills.

The beneficial effects of the novel polypeptide provided by the present invention and use thereof:

The polypeptide of the present invention has a short peptide chain, so it is absorbed faster and better. The experimental results show that the polypeptide provided in the present application showed a significant effect of alleviating intestinal lumen dilatation in the test of a rat model of acute inflammatory bowel disease; and had a significant therapeutic effect in the test of the rat model of acute inflammatory bowel disease. The polypeptide of the present invention has a significant effect of alleviating colitis and can be used to prepare drugs for treating enteritis, especially drugs for ulcerative colitis.

**BRIEF DESCRIPTION OF THE DRAWINGS**

FIG. 1 shows a schematic diagram of the steps of solid phase synthesis of polypeptides; and

FIG. 2 shows the mass spectrum of the polypeptide.

**DETAILED DESCRIPTION OF THE INVENTION**

The following is an explanation of the present invention in combination with specific experiments, which is not intended to limit the scope of protection of the present invention.

As used in the present application (including the attached claims), unless the context otherwise clearly indicates, singular forms of words such as "one/a, an" and "the" include their corresponding plural referents.

Unless otherwise clearly indicated by the context, the term "or" is used to refer to the term "and/or" and may be used interchangeably with the term "and/or".

The term "effective amount" or "therapeutically effective amount" refers to the amount of an active ingredient (such as a compound) which when administered to a subject to treat a disease, or at least one clinical symptom of a disease or disorder, is sufficient to influence the treatment of the disease, disorder or symptom. The "therapeutically effective amount" may vary depending on the compound, the disease, disorder and/or symptoms of the disease or disorder, the severity of the disease, disorder and/or symptoms of the disease or disorder, the age of the subject to be treated and/or the weight of the subject to be treated. In any given case, the appropriate amount will be apparent to those skilled in the art, or can be determined by routine experimentation. In some embodiments, a "therapeutically effective amount" is an amount of at least one compound and/or at least one stereoisomer thereof, and/or at least one pharmaceutically acceptable salt thereof, disclosed in the present application, that is effective for the "treatment" (as defined above) of a disease or disorder in a subject. In the case of a combination therapy, "therapeutically effective amount" is the total amount of the combined objects used to effectively treat a disease, disorder or condition.

The term "physiologically compatible salt" refers to a salt form which is physiologically compatible (i.e., pharmacologically acceptable), and is substantially non-toxic to the individual to which the compounds of the invention will be administered. Physiologically compatible salts of the compounds of this invention include conventional and stoichiometric acid addition salts or base addition salts formed from suitable non-toxic organic or inorganic acids or inorganic bases.

The term "intestinal lumen dilatation" refers to intestinal dilatation or intestinal canal dilatation. In patients with enteritis, due to various factors such as intestinal damage, mucus exudation, intestinal gas or fluid accumulation, microbial overgrowth and the like, the intestinal lumen is dilated and the intestinal canal is thicker than normal. Recurrent enteritis such as inflammatory bowel disease leading to intestinal canal dilatation, or tumors causing intestinal canal dilatation and intestinal obstruction are common in clinic.

The abbreviations for amino acid used in the present application are as follows.

**Table 1 English names and abbreviations of solvents and reagents, and their corresponding Chinese names**

| Chinese name | English name | Abbreviation |
|---|---|---|
| 丙氨酸 | Alanine | Ala |
| 精氨酸 | Arginine | Arg |
| 天冬酰胺 | Asparagine | Asn |
| 天冬氨酸 | Aspartic acid | Asp |
| 半胱氨酸 | Cysteine | Cys |
| 谷氨酰胺 | Glutamine | Gln |
| 谷氨酸 | Glutamic acid | Glu |
| 甘氨酸 | Glycine | Gly |
| 组氨酸 | Histidine | His |
| 异亮氨酸 | Isoleucine | Ile |
| 亮氨酸 | Leucine | Leu |
| 赖氨酸 | Lysine | Lys |
| 蛋氨酸 | Methionine | Met |
| 苯丙氨酸 | Phenylalanine | Phe |
| 脯氨酸 | Proline | Pro |

(continued)

| Chinese name | English name | Abbreviation |
|---|---|---|
| 丝氨酸 | Serine | Ser |
| 苏氨酸 | Threonine | Thr |
| 色氨酸 | Tryptophan | Trp |
| 酪氨酸 | Tyrosine | Tyr |
| 缬氨酸 | Valine | Val |

| English name and abbreviation | Chinese name |
|---|---|
| 2-Chlorotrityl Chloride Resin | 2-chlorotrityl chloride Resin |
| DMF | *N, N*-dimethyl formamide |
| DCM | Dichloromethane |
| PIP | Piperidine |
| HOBt | 1-hydroxybenzotriazole |
| DIPEA | *N,N*-diisopropylethylamine |
| Methanol | Methyl alcohol |
| *tert*-Butyl methyl ether | Methyl tertiary butyl ether |
| TFA | Trifluoroacetic acid |
| TIPS | Triisopropylsilane |
| DIC | *N,N*'-diisopropyl carbodiimide |
| Ethanol | Ethyl alcohol |
| Aa | Amino acid |

**Example 1: Chemical synthesis of polypeptides**

The synthesis of polypeptide compounds adopts the conventional solid phase synthesis method using a fully automatic polypeptide synthesizer, and the chemical synthesis of the polypeptide is carried out through the processes of resin swelling, deprotection, washing, amino acid activation and condensation.

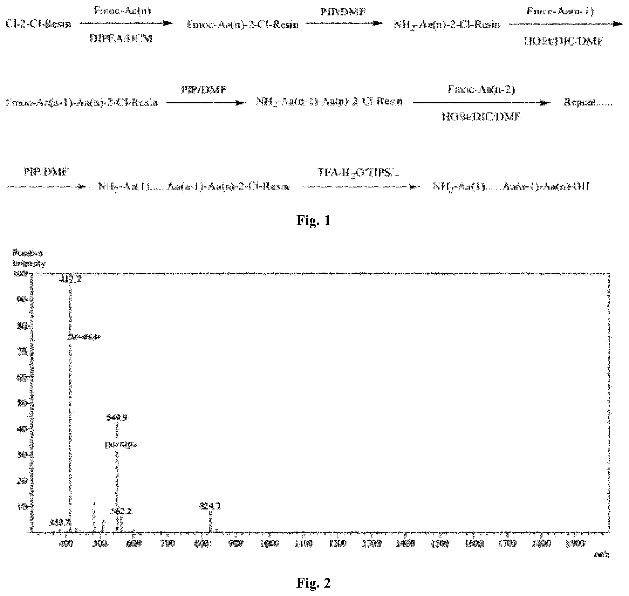

Taking Lys-Glu-Pro-Val-Pro-Gln-Ala-Lys-Pro-Arg-Lys-Val-Ala-Ala-Gln (Compound 1) as an example, the schematic diagram of the solid phase synthesis steps of the polypeptide is shown in FIG. 1.

Step 1: Preparation of fully protected peptide resin for polypeptide

(1) Resin swelling: 7.71 g (SD = 0.73 mmol/g) of 2-Chlorotrityl Chloride Resin was weighed, added into a synthesis tube with a sieve plate, and swollen with 100 ml of dichloromethane (DCM).

(2) Preparation of Fmoc-Gln (Trt)-resin: Fmoc-Gln(Trt)-OH and DIPEA were weighed in a molar ratio of 1:1.78:4.31 of resin, Fmoc-Gln(Trt)-OH and DIPEA respectively and added into the synthesis tube. Nitrogen ($N_2$) bubbling and shaking were performed at room temperature for 1-3 h, and then the mixture was pumped dry; then the mixture was washed with dimethylformamide (DMF) 5 times, 100 ml each time, and the resin was pumped dry.

(3) Removal of Fmoc protecting group: 100 ml of a 20% piperidine-DMF (v/v) solution was added into the reactor, nitrogen was bubbled, and the system was allowed to react for 20 min, pumped dry, then washed with DMF 5 times, at 100 ml/time, 3 min/time, pumped dry, and detected for the Fmoc removal result by the ninhydrin method.

(4) Amino acid pre-activation: 15 mmol of Fmoc-protected amino acid, 15 mmol of HOBt, and 15 mmol of DIC was added into a 250 ml beaker, dissolved in 100 ml of DMF, and stirred at room temperature for later use.

(5) Amino acid linkage: The activated protected amino acid solution was poured into the reactor, and an appropriate amount of DCM was additionally added to clean the apparatus. Nitrogen was bubbled at room temperature and the system was allowed to react for 1-3 h, and whether the amino acid linkage is complete was detected using the ninhydrin method. If so, the system was pumped dry. The resin was washed with DMF 5 times, at 100 ml/time, 3 min/time, and then pumped dry. The amount of each of the amino acids and condensing agents used is shown in Table 2.

(6) After the condensation of the first amino acid was completed, steps (4) and (5) were repeated to extend the peptide chain in the order of amino acids until the coupling of last amino acid was completed.

(7) The resin peptide was washed with DMF 4 times, at 150 ml/time, 3 min/time; then washed with DCM 5 times, at 150 ml/time, 3 min/time, and pumped dry.

**Table 2 Usages of amino acids and condensing agents**

| Amino acid name | Aa/eq | Amino acid usage/g | HOBt/g | DIPEA/g | DIC/g |
|---|---|---|---|---|---|
| Fmoc-L-Gln(Trt)-OH | 10 | 6.107 | / | 3.128 | / |
| Fmoc-L-Ala-OH·$H_2O$ | 15 | 4.941 | 2.027 | / | 2.379 |
| Fmoc-L-Ala-OH·$H_2O$ | 15 | 4.941 | 2.027 | / | 2.379 |
| Fmoc-L-Val-OH | 15 | 5.091 | 2.027 | / | 2.379 |
| Fmoc-L-Lys(Boc)-OH | 15 | 7.029 | 2.027 | / | 2.379 |
| Fmoc-L-Arg(Pbf)-OH | 15 | 9.732 | 2.027 | / | 2.379 |
| Fmoc-L-Pro-OH·$H_2O$ | 15 | 5.061 | 2.027 | / | 2.379 |
| Fmoc-L-Lys(Boc)-OH | 15 | 7.029 | 2.027 | / | 2.379 |
| Fmoc-L-Ala-OH·$H_2O$ | 15 | 4.941 | 2.027 | / | 2.379 |
| Fmoc-L-Gln(Trt)-OH | 15 | 9.161 | 2.027 | / | 2.379 |
| Fmoc-L-Pro-OH·$H_2O$ | 15 | 5.061 | 2.027 | / | 2.379 |
| Fmoc-L-Val-OH | 15 | 5.091 | 2.027 | / | 2.379 |
| Fmoc-L-Pro-OH·$H_2O$ | 15 | 5.061 | 2.027 | / | 2.379 |
| Fmoc-L-Glu(OtBu)-OH·$H_2O$ | 15 | 6.653 | 2.027 | / | 2.379 |
| Fmoc-L-Lys(Boc)-OH | 15 | 7.029 | 2.027 | / | 2.379 |

Step 2: Cleaving

(1) 100 ml of a cleavage agent (TFA: TIPS:$H_2O$= 95: 2.5: 2.5, v/v) was added into the synthesis tube, nitrogen was bubbled, the system was allowed to react for 1.5-3 h.

(2) After the cleavage reaction was completed, the cleavage agent was suction-filtered into a 250 ml roundbottom flask. After vacuum concentration to one-fourth of the original volume of the cleavage agent, 10 times the existing volume of methyl tert-butyl ether was added to precipitate a white solid. The obtained mixture was filtered and washed three times with 50 ml of methyl tert-butyl ether, and then the obtained crude peptide product was placed in a sand core funnel and blowed dry with nitrogen in a fume hood to evaporate the solvent until the crude peptide was in powder form. 9.04 g of crude peptide was obtained with a crude yield of 74.7%.

Step 3: Purification, salt exchange and freeze-drying

Polypeptide HPLC direct salt exchange (acetate salt)

A. Chromatographic parameters

Chromatographic column: dynamic axial compression column 80*250 mm, filler: Daisogel C18 (SP-100-8-ODS-P)
Eluent A1: 0.1 M acetic acid
Eluent A2: 0.025 M acetic acid-0.1 M ammonium acetate
Eluent B: acetonitrile

Flow rate: 180ml/min
UV detection wavelength: 220nm

B. Operating steps

a) Dissolving the crude peptide in water and/or acetonitrile and filtering through a 0.45 $\mu$m filter membrane
b) Equilibrating the chromatographic column with 95% A1 + 5% B
c) Sample injection
d) Equilibrating the chromatographic column with 95% A2 + 5% B
e) Gradient elution with A1 and B
f) Collecting the target peptide eluate
g) Rotary evaporation for concentration
h) Freeze drying

8.26 g of crude peptide was purified to obtain 4.92 g of pure peptide, with a purification yield of 59.5%.

In a similar manner to the synthesis of compound 1, other compounds were synthesized. The results are shown in Table 3 and other parts of the specification.

### Table 3 Synthesized polypeptide compounds

| Serial No. | Compound sequence | Crude product (g) | Purification yield | Acetate purity | MS |
|---|---|---|---|---|---|
| 1 | Lys-Glu-Pro-Val-Pro-Gln-Ala-Lys-Pro-Arg-Lys-Val-Ala-Ala-Gln | 9.04 | 59.5% | 99.44% | 412.70 (quadruply charged)<br>549.9 (triply charged) |
| 2 | Lys-Glu-Pro-Val-Pro-Gln-Ala-Lys-Pro-Arg-Lys | 2.04 | 14.7% | 97.05% | 426.60 (triply charged)<br>639.30 (doubly charged) |
| 3 | Lys-Glu-Pro-Val-Pro-Gln-Ala-Lys-Pro-Arg | 2.11 | 25.7% | 97.47% | 383.90 (triply charged)<br>575.20 (doubly charged) |
| 4 | Lys-Glu-Pro-Val-Pro-Gln-Ala-Lys | 1.55 | 26.1% | 98.22% | 448.70 (doubly charged)<br>896.20 [M+H]+ |
| 5 | Pro-Arg-Lys-Val-Ala-Ala-Gln | 1.06 | 30.6% | 96.82% | 385.60 (doubly charged)<br>769.20 [M+H]+ |
| 6 | Glu-Pro-Val-Pro-Gln-Ala-Lys | 1.64 | 20.8% | 98.32% | 384.70 (doubly charged)<br>768.20 [M+H]+ |
| 7 | Lys-Glu-Pro-Val | 4.30 | 52.0% | 97.22% | 472.20 [M+H]+ |
| 8 | Val-Ala-Ala-Gln | 3.60 | 53.0% | 100% | 388.10 [M+H]+ |
| 9 | Glu-Pro-Val-Pro | N/A | 57.0% | 99.01% | 441.30 [M+H]+ |
| 10 | Lys-Pro-Arg-Lys | 0.50 | 13.5% | 98.39% | 264.70 doubly charged<br>528.20 [M+H]+ |
| 11 | Ala-Lys-Pro-Arg | 3.79 | 17.3% | 98.79% | 471.20 [M+H]+ |
| 12 | Pro-Val-Pro-Gln | 0.20 | 48.7% | 100% | 440.30 [M+H]+ |
| 13 | Val-Pro-Gln-Ala | 0.35 | 57.5% | 99.42% | 414.30 [M+H]+ |

(continued)

| Serial No. | Compound sequence | Crude product (g) | Purification yield | Acetate purity | MS |
|---|---|---|---|---|---|
| 14 | Pro-Gln-Ala-Lys | 3.81 | 22.0% | 98.20% | 443.10 [M+H]+ |
| 15 | Arg-Lys-Val-Ala | 4.23 | 32.5% | 97.48% | 237.10 (doubly charged) 473.20 [M+H]+ |
| 16 | Lys-Val-Ala-Ala | 3.81 | 27.0% | 98.17% | 388.10 [M+H]+ |
| 17 | Gln-Ala-Lys-Pro | N/A | 22.9% | 96.01% | 443.20 [M+H]+ |
| 18 | Pro-Arg-Lys-Val | N/A | 17.7% | 97.47% | 250.20 (doubly charged) 499.30 [M+H]+ |
| 19 | Ala-Glu-Pro-Val-Pro-Gln-Ala-Lys-Pro-Arg-Lys-Val-Ala-Ala-Gln | 1.73 | 43.7% | 100% | 795.60 (doubly charged) 530.80 (triply charged) |
| 20 | Lys-Ala-Pro-Val-Pro-Gln-Ala-Lys-Pro-Arg-Lys-Val-Ala-Ala-Gln | 1.96 | 45.8% | 99.36% | 795.10 (doubly charged) 530.50 (triply charged) |
| 21 | Lys-Glu-Ala-Val-Pro-Gln-Ala-Lys-Pro-Arg-Lys-Val-Ala-Ala-Gln | 1.24 | 54.9% | 99.59% | 811.10 (doubly charged) 541.20 (triply charged) |
| 22 | Lys-Glu-Pro-Val-Ala-Gln-Ala-Lys-Pro-Arg-Lys-Val-Ala-Ala-Gln | 1.5 | 46.5% | 99.54% | 811.10 (doubly charged) 541.20 (triply charged) |
| 23 | Lys-Glu-Pro-Val-Pro-Ala-Ala-Lys-Pro-Arg-Lys-Val-Ala-Ala-Gln | 1.67 | 51.3% | 99.42% | 795.50 (doubly charged) 530.70 (triply charged) |
| 24 | Lys-Glu-Pro-Val-Pro-Gln-Ala-Ala-Pro-Arg-Lys-Val-Ala-Ala-Gln | 2.65 | 52.6% | 99.72% | 795.50 (doubly charged) 530.70 (triply charged) |
| 25 | Lys-Glu-Pro-Ala-Pro-Gln-Ala-Lys-Pro-Arg-Lys-Val-Ala-Ala-Gln | 3.21 | 51.8% | 99.90% | 810.20 (doubly charged) 540.50 (triply charged) |
| 26 | Lys-Glu-Pro-Val-Pro-Gln-Ala-Lys-Ala-Arg-Lys-Val-Ala-Ala-Gln | 3.09 | 53.6% | 99.82% | 811.00 (doubly charged) 541.00 (triply charged) |

(continued)

| Serial No. | Compound sequence | Crude product (g) | Purification yield | Acetate purity | MS |
|---|---|---|---|---|---|
| 27 | Lys-Glu-Pro-Val-Pro-Gln-Ala-Lys-Pro-Ala-Lys-Val-Ala-Ala-Gln | 3.07 | 42.0% | 98.06% | 781.50 (doubly charged)<br>521.40 (triply charged) |
| 28 | Lys-Glu-Pro-Val-Pro-Gln-Ala-Lys-Pro-Arg-Ala-Val-Ala-Ala-Gln | 2.74 | 42.3% | 99.73% | 795.50 (doubly charged)<br>530.70 (triply charged) |
| 29 | Lys-Glu-Pro-Val-Pro-Gln-Ala-Lys-Pro-Arg-Lys-Ala-Ala-Ala-Gln | 2.61 | 39.2% | 99.10% | 810.00 (doubly charged)<br>540.40 (triply charged) |
| 30 | Lys-Glu-Pro-Val-Pro-Gln-Ala-Lys-Pro-Arg-Lys-Val-Ala-Ala-Ala | 1.82 | 53.5% | 99.68% | 795.60 (doubly charged)<br>530.80 (triply charged) |
| 31 | Lys-Glu-Gly-Val-Pro-Gln-Ala-Lys-Pro-Arg-Lys-Val-Ala-Ala-Gln | 2.38 | 50.3% | 100% | 804.10 (doubly charged)<br>536.50 (triply charged) |
| 32 | Lys-Glu-Pro-Val-Gly-Gln-Ala-Lys-Pro-Arg-Lys-Val-Ala-Ala-Gln | 1.93 | 38.5% | 99.12% | 804.10 (doubly charged)<br>536.40 (triply charged) |
| 33 | Lys-Glu-Pro-Val-Pro-Gln-Ala-Lys-Gly-Arg-Lys-Val-Ala-Ala-Gln | 2.95 | 55.5% | 100% | 536.50 (triply charged)<br>804.00 (doubly charged) |
| 34 | Lys-Glu-Pro-Val-Gly-Gln-Ala-Lys-Gly-Arg-Lys-Val-Ala-Ala-Gln | 3.37 | 53.2% | 100% | 523.10 (triply charged)<br>784.10 (doubly charged) |
| 35 | Lys-Glu-Gly-Val-Gly-Gln-Ala-Lys-Gly-Arg-Lys-Val-Ala-Ala-Gln | 2.51 | 59.1% | 100% | 509.7 (triply charged)<br>764.10 (doubly charged) |
| 36 | Lys-Glu-Gly-Val-Pro-Gln-Ala-Lys-Gly-Arg-Lys-Val-Ala-Ala-Gln | 2.93 | 53.4% | 100% | 523.10 (triply charged)<br>784.10 (doubly charged) |

(continued)

| Serial No. | Compound sequence | Crude product (g) | Purification yield | Acetate purity | MS |
|---|---|---|---|---|---|
| 37 | Lys-Glu-Gly-Val-Gly-Gln-Ala-Lys-Pro-Arg-Lys-Val-Ala-Ala-Gln | 2.65 | 59.4% | 100% | 523.10 (triply charged) 784.10 (doubly charged) |
| 38 | Glu-Pro-Val-Pro-Gln-Ala-Lys-Pro-Arg-Lys | 1.85 | 51.1% | 99.00% | 575.50 (doubly charged) 384.00 (triply charged) |
| 39 | Pro-Val-Pro-Gln-Ala | | N/A | 99.93% | 511.20 [M+H]+ |
| 40 | Pro-Val-Pro-Glu-Ala | 4.50 | 47.9% | 100% | 512.40 [M+H]+ |
| 41 | Pro-Val-Pro-Ile-Ala | 4.77 | 60.8% | 99.87% | 496.50 [M+H]+ |
| 42 | Pro-Val-Pro-Ala | 3.48 | 60.7% | 100% | 383.30 [M+H]+ |
| 43 | Glu-Pro-Val-Pro-Gln-Ala | 0.66 | 60.1% | 97.77% | 640.20 [M+H]+ |
| 44 | Pro-Val-Pro-Gln-Ala-Lys | 0.61 | 45.2% | 99.87% | 639.30 [M+H]+ |
| 45 | Glu-Pro-Val-Pro-Gln-Ala-Arg | 0.73 | 42.3% | 98.98% | 796.30 [M+H]+ |
| 46 | Ala-Pro-Val-Pro-Gln-Ala-Lys | 0.70 | 52.8% | 99.78% | 710.30 [M+H]+ |
| 47 | Glu-Pro-Val-Pro-Ala-Ala-Lys | 1.02 | 39.7% | 98.55% | 711.40 [M+H]+ |
| 48 | Glu-Pro-Val-Pro-Gln-Ala-Ala | 1.03 | 63.3% | 97.75% | 711.40 [M+H]+ |
| 49 | Asp-Pro-Val-Pro-Gln-Ala-Lys | N/A | N/A | 99.69% | 754.30 [M+H]+ |
| 50 | Asn-Pro-Val-Pro-Gln-Ala-Lys | 1.03 | 58.3% | 99.53% | 753.30 [M+H]+ |
| 51 | Glu-Pro-Val-Pro-Gln-Ala-Lys-Pro-Arg-Lys-Val-Ala | N/A | N/A | 97.89% | 660.50 (doubly charged) |
| 52 | Glu-Pro-Ala-Pro-Gln-Ala-Lys | 1.04 | 56.9% | 99.14% | 740.30 [M+H]+ |
| 53 | Glu-Ala-Val-Pro-Gln-Ala-Lys | N/A | N/A | 98.45% | 371.80 doubly charged 742.40 [M+H]+ |

(continued)

| Serial No. | Compound sequence | Crude product (g) | Purification yield | Acetate purity | MS |
|---|---|---|---|---|---|
| 54 | Glu-Pro-Val-Ala-Gln-Ala-Lys | 0.66 | 63.2% | 98.35% | 742.40 [M+H]+ |
| 55 | Glu-Pro-Val-Pro-Glu-Ala-Lys | N/A | N/A | 97.91% | 385.30 (doubly charged) 769.30 [M+H]+ |
| 56 | Glu-Pro-Val-Pro-Gln-Ala-Lys-Pro-Arg-Lys-Val | N/A | N/A | 98.46% | 417.10 (triply charged) 625.10 [M+H]+ |
| 57 | Glu-Pro-Val-Pro-Gln-Ala-Lys-Pro-Arg | N/A | N/A | 98.76% | 511.40 (doubly charged) |
| 58 | Glu-Pro-Val-Pro-Gln-Ala-Lys-Pro | N/A | N/A | 98.17% | 433.30 (doubly charged) 865.50 [M+H]+ |
| 59 | Pro-Val-Pro-Gln-Ala-Lys-Pro-Arg | N/A | N/A | 99.47% | 298.20 (triply charged) 446.90 (doubly charged) 892.50 [M+H]+ |
| 60 | Pro-Val-Pro-Gln-Ala-Lys-Pro | N/A | N/A | 99.42% | 368.90 (doubly charged) 736.40 [M+H]+ |
| 61 | D-Lys-Glu-Pro-Val-Pro-Gln-Ala-Lys-Pro-Arg-Lys-Val-Ala-Ala-Gln | 2.35 | 52.4% | 100% | 824.2 doubly charged<br><br>549.8 triply charged |
| 62 | Lys-D-Glu-Pro-Val-Pro-Gln-Ala-Lys-Pro-Arg-Lys-Val-Ala-Ala-Gln | 2.21 | 53.8% | 100% | 824.2 doubly charged<br><br>549.8 triply charged |
| 63 | Lys-Glu-D-Pro-Val-Pro-Gln-Ala-Lys-Pro-Arg-Lys-Val-Ala-Ala-Gln | 2.1 | 54.3% | 100% | 824.2 doubly charged<br><br>549.9 triply charged |
| 64 | Lys-Glu-Pro-D-Val-Pro-Gln-Ala-Lys-Pro-Arg-Lys-Val-Ala-Ala-Gln | 2.1 | 54.8% | 99.74% | 824.2 doubly charged<br><br>549.7 triply charged |

(continued)

| Serial No. | Compound sequence | Crude product (g) | Purification yield | Acetate purity | MS |
|---|---|---|---|---|---|
| 65 | Lys-Glu-Pro-Val-D-Pro-Gln-Ala-Lys-Pro-Arg-Lys-Val-Ala-Ala-Gln | 1.97 | 48.6% | 100% | 824.1 doubly charged<br><br>549.8 triply charged |
| 66 | Lys-Glu-Pro-Val-Pro-D-Gln-Ala-Lys-Pro-Arg-Lys-Val-Ala-Ala-Gln | 1.99 | 59.8% | 100% | 824.1 doubly charged<br><br>549.8 triply charged |
| 67 | Lys-Glu-Pro-Val-Pro-Gln-D-Ala-Lys-Pro-Arg-Lys-Val-Ala-Ala-Gln | 1.71 | 86.5% | 100% | 824.2 doubly charged<br><br>549.8 triply charged |
| 68 | Lys-Glu-Pro-Val-Pro-Gln-Ala-D-Lys-Pro-Arg-Lys-Val-Ala-Ala-Gln | 2.21 | 71.5% | 100% | 824.2 doubly charged<br><br>549.9 triply charged |
| 69 | Lys-Glu-Pro-Val-Pro-Gln-Ala-Lys-D-Pro-Arg-Lys-Val-Ala-Ala-Gln | 1.87 | 74.3% | 99.59% | 824.3 doubly charged<br><br>549.8 triply charged |
| 70 | Lys-Glu-Pro-Val-Pro-Gln-Ala-Lys-Pro-D-Arg-Lys-Val-Ala-Ala-Gln | 1.99 | 63.4% | 100% | 824.3 doubly charged<br><br>549.9 triply charged |
| 71 | Lys-Glu-Pro-Val-Pro-Gln-Ala-Lys-Pro-Arg-D-Lys-Val-Ala-Ala-Gln | 2.15 | 67.1% | 100% | 824.2 doubly charged<br><br>549.9 triply charged |
| 72 | Lys-Glu-Pro-Val-Pro-Gln-Ala-Lys-Pro-Arg-Lys-D-Val-Ala-Ala-Gln | 1.95 | 73.3% | 100% | 824.2 doubly charged<br><br>550.0 triply charged |

(continued)

| Serial No. | Compound sequence | Crude product (g) | Purification yield | Acetate purity | MS |
|---|---|---|---|---|---|
| 73 | Lys-Glu-Pro-Val-Pro-Gln-Ala-Lys-Pro-Arg-Lys-Val-D-Ala-Ala-Gln | 2.94 | 46.3% | 97.94% | 824.0 doubly charged<br><br>549.7 triply charged |
| 74 | Lys-Glu-Pro-Val-Pro-Gln-Ala-Lys-Pro-Arg-Lys-Val-Ala-D-Ala-Gln | 3.32 | 37.3% | 99.12% | 824.1 doubly charged<br><br>549.8 triply charged |
| 75 | Lys-Glu-Pro-Val-Pro-Gln-Ala-Lys-Pro-Arg-Lys-Val-Ala-Ala-D-Gln | 3.73 | 44.2% | 99.03% | 824.1 doubly charged<br><br>549.7 triply charged |
| 76 | Glu-Gly-Val-Pro-Gln-Ala-Lys | N/A | N/A | 97.45% | 364.80 doubly charged<br>728.50 [M+H]$^+$ |
| 77 | Glu-Pro-Val-Gly-Gln-Ala-Lys | N/A | N/A | 97.89% | 364.80 doubly charged<br>728.50 [M+H]$^+$ |
| 78 | D-Glu-Pro-Val-Pro-Gln-Ala-Lys | N/A | N/A | 96.92% | 768.50 [M+H]$^+$<br>384.90 doubly charged |
| 79 | Glu-D-Pro-Val-Pro-Gln-Ala-Lys | N/A | N/A | 96.65% | 768.60 [M+H]$^+$<br>384.70 doubly charged |
| 80 | Glu-Pro-D-Val-Pro-Gln-Ala-Lys | 3.86 | 48.5% | 98.51% | 768.5 [M+H]$^+$<br>384.9 doubly charged |
| 81 | Glu-Pro-Val-D-Pro-Gln-Ala-Lys | 3.39 | 45.6% | 98.06% | 768.6 [M+H]$^+$<br>384.8 doubly charged |
| 82 | Glu-Pro-Val-Pro-D-Gln-Ala-Lys | 3.76 | 41.2% | 98.34% | 768.6 [M+H]$^+$<br>384.9 doubly charged |
| 83 | Glu-Pro-Val-Pro-Gln-D-Ala-Lys | 3.83 | 45.2% | 97.85% | 768.3 [M+H]$^+$<br>384.7 doubly charged |
| 84 | Glu-Pro-Val-Pro-Gln-Ala-D-Lys | N/A | 55.0% | 97.06% | 768.2 [M+H]$^+$ |
| 85 | Lys-Glu-Pro-Val-Pro | 3.38 | 42.8% | 99.94% | 569.1 [M+H]$^+$ |
| 86 | Glu-Pro-Val-Pro-Gln | 330m | 38.4% | 97.14% | 569.2 [M+H]$^+$ |

(continued)

| Serial No. | Compound sequence | Crude product (g) | Purification yield | Acetate purity | MS |
|---|---|---|---|---|---|
| 87 | Lys-Glu-Pro-Val-Pro-Gln | 560m | 91.8% | 98.83% | 697.3 [M+H]$^+$ <br> 349.1 doubly charged |
| 88 | Val-Pro-Tyr-Lys-Glu-Pro-Val-Pro-Gln-Ala-Lys-Pro-Arg-Lys-Val-Ala-Ala-Gln | 3.57 | 39.7% | 99.40% | 669.6 triply charged <br><br> 1003.7 doubly charged |
| 89 | *Pyro*-Glu-Leu-Lys-Glu-Pro-Val-Pro-Gln-Ala-Lys-Pro-Arg-Lys-Val-Ala-Ala-Gln | 2.66 | 48.6% | 99.86% | 624.5 triply charged <br><br> 936.1 doubly charged |
| 90 | Pro-Ala-Tyr-Lys-Glu-Pro-Val-Pro-Gln-Ala-Lys-Pro-Arg-Lys-Val-Ala-Ala-Gln | 3.15 | 49.3% | 98.66% | 660.2 triply charged <br><br> 990.2 doubly charged |
| 91 | Arg-Lys-Asp-Val-Tyr-Lys-Glu-Pro-Val-Pro-Gln-Ala-Lys-Pro-Arg-Lys-Val-Ala-Ala-Gln | 3.37 | 27.4% | 100% | 770.2 triply charged <br><br> 1154.8 doubly charged |
| 92 | Arg-Lys-Asp-Val-Tyr-Lys-Glu-Pro-Val-Pro-Gln-Ala-Lys | 1.82 | 44.1% | 98.42% | 779.4 doubly charged <br><br> 520.0 triply charged |
| 93 | Arg-Lys-Asp-Val-Tyr-Glu-Pro-Val-Pro-Gln-Ala-Lys | 1.3 | 48.5% | 98.83% | 715.3 doubly charged <br><br> 477.3 triply charged |
| 94 | Arg-Lys-Asp-Val-Tyr-Pro-Val-Pro-Gln | 0.77 | 57.1% | 98.08% | 551.3 doubly charged <br><br> 1101.5 [M+H]$^+$ |
| 95 | Gly-Pro-Glu-Thr-Ala-Phe-Leu-Arg-Lys-Glu-Pro-Val-Pro-Gln-Ala-Lys-Pro-Arg-Lys-Val-Ala-Ala-Gln | 1.58 | 52.7% | 94.96% | 840.2 triply charged <br><br> 1260.2 doubly charged |

(continued)

| Serial No. | Compound sequence | Crude product (g) | Purification yield | Acetate purity | MS |
|---|---|---|---|---|---|
| 96 | Gly-Pro-Glu-Thr-Ala-Phe-Leu-Arg-Glu-Pro-Val-Pro-Gln-Ala-Lys | 0.98 | 55.1% | 96.59% | 820.4 doubly charged<br><br>547.2 triply charged |
| 97 | Ser-Ser-Glu-Asp-Ile-Lys-Glu-Lys-Glu-Pro-Val-Pro-Gln-Ala-Lys-Pro-Arg-Lys-Val-Ala-Ala-Gln | 4.04 | 39.1% | 94.51% | 812.7 triply charged<br><br>1218.4 doubly charged |
| 98 | Ser-Ser-Glu-Asp-Ile-Lys-Glu-Glu-Pro-Val-Pro-Gln-Ala-Lys | 2.42 | 43.6% | 99.27% | 779.0 doubly charged<br><br>519.7 triply charged |
| 99 | Ser-Ser-Glu-Asp-Ile-Lys-Glu-Pro-Val-Pro-Gln | 1.58 | 49.9% | 98.08% | 614.9 doubly charged<br><br>1228.6 [M+H]$^+$ |
| 100 | Val-Pro-Pro-Lys-Glu-Pro-Val-Pro-Gln-Ala-Lys-Pro-Arg-Lys-Val-Ala-Ala-Gln | 2.81 | 37.9% | 100% | 647.5 triply charged<br><br>970.8 doubly charged |
| 101 | Glu-Pro-Val-Pro-Gln-Ala-Lys-Ser-Ser-Glu-Asp-Ile-Lys-Glu | 4.65 | 11.0% | 98.63% | 778.9 doubly charged<br><br>519.6 triply charged |
| 102 | Glu-Pro-Val-Pro-Gln-Ala-Lys-Val-Pro-Tyr | 4.3 | 49.8% | 97.73% | 564.1 doubly charged<br>1127.5 [M+H]$^+$ |
| 103 | Lys-Glu-Pro-Val-Pro-Gln-Ala-Lys-Pro-Arg-Lys-Val-Ala-Ala-Glu | 5.9 | 26.3% | 99.84% | 824.4 doubly charged<br><br>550.0 triply charged |
| 104 | Lys-Glu-Pro-Val-Pro-Gln-Ala-Lys-Pro-Arg-Lys-Val-Ala-Ala-Asn | 6.04 | 42.8% | 98.79% | 817.0 doubly charged<br><br>545.1 triply charged |

(continued)

| Serial No. | Compound sequence | Crude product (g) | Purification yield | Acetate purity | MS |
|---|---|---|---|---|---|
| **105** | Lys-Gln-Pro-Val-Pro-Gln-Ala-Lys-Pro-Arg-Lys-Val-Ala-Ala-Gln | 6.4 | 39.3% | 98.66% | 823.6 doubly charged<br><br>549.5 triply charged |
| **106** | Lys-Glu-Pro-Ile-Pro-Gln-Ala-Lys-Pro-Arg-Lys-Val-Ala-Ala-Gln | 5.98 | 39.7% | 99.54% | 831.1 doubly charged<br><br>554.5 triply charged |
| **107** | Val-Pro-Gln-Ala | 0.47 | 47.2% | 100% | 414.2 [M+H]+ |
| **108** | Val-Pro-Gln-Ala-Lys | 0.50 | 51.3% | 99.63% | 542.3 [M+H]+ |

**Note:** A doubly charged peak indicates that the target molecule is bound to 2 protons, and a triply charged peak indicates that the target molecule is bound to 3 protons; and N/A means that the weighing is difficult and the actual weight is not considered.

**Compound 1:** Lys-Glu-Pro-Val-Pro-Gln-Ala-Lys-Pro-Arg-Lys-Val-Ala-Ala-Gln (acetate salt)

High resolution mass spectrometry (TOF-HRMS), molecular formula: $C_{73}H_{127}N_{23}O_{20}$; m/z:m/z: 549.65904 ($[M+3H]^{3+}$), 823.98493 ($[M+2H]^{2+}$), 1646.96346 ($[M+H]^+$).
$^1$H NMR (600 MHz, D$_2$O+CD$_3$CN) δ 4.48 (dd, $J$ = 9.5, 4.1 Hz, 1H), 4.42 (dd, $J$ = 9.2, 4.5 Hz, 1H), 4.32 - 4.27 (m, 1H), 4.27 - 4.21 (m, 3H), 4.20 - 4.07 (m, 6H), 4.00 (dd, $J$ = 8.3, 4.8 Hz, 1H), 3.95 (d, $J$ = 7.5 Hz, 1H), 3.86 (t, $J$ = 6.7 Hz, 1H), 3.77 - 3.62 (m, 3H), 3.59 - 3.43 (m, 3H), 3.04 (t, $J$= 6.7 Hz, 2H), 2.87 - 2.78 (m, 6H), 2.28 - 2.08 (m, 9H), 2.00 - 1.80 (m, 31H, AcOH), 1.80 - 1.41 (m, 23H), 1.40 - 1.24 (m, 6H), 1.24 - 1.18 (m, 9H), 0.87 - 0.73 (m, 12H).

**Compound 2:** Lys-Glu-Pro-Val-Pro-Gln-Ala-Lys-Pro-Arg-Lys (acetate salt)

High resolution mass spectrometry (TOF-HRMS), molecular formula: $C_{57}H_{100}N_{18}O_{15}$; m/z: 426.59680 ($[M+3H]^{3+}$), 639.39021 ($[M+2H]^{2+}$), 1277.77023 ($[M+H]^+$).
1H NMR (600 MHz, DMSO-d6) δ 4.57 (dd, $J$ = 8.7, 4.9 Hz, 1H), 4.41 - 4.11 (m, 10H), 3.83 (s, 1H), 3.78 (t, $J$ = 6.5 Hz, 1H), 3.54 (d, $J$ = 73.5 Hz, 7H), 3.08 (d, $J$ = 7.0 Hz, 2H), 2.74 - 2.68 (m, 7H), 2.32 (d, $J$ = 9.1 Hz, 2H), 2.23 - 2.17 (m, 2H), 2.05 - 1.46 (m, AcOH, 56H), 1.43 - 1.22 (m, 7H), 1.17 (d, $J$= 7.0 Hz, 3H), 0.82 (dd, $J$ = 19.6, 6.7 Hz, 6H).

**Compound 3:** Lys-Glu-Pro-Val-Pro-Gln-Ala-Lys-Pro-Arg (acetate salt)

High resolution mass spectrometry (TOF-HRMS), molecular formula: $C_{51}H_{88}N_{16}O_{14}$; m/z: 383.89927 ($[M+3H]^{3+}$), 575.34276 ($[M+2H]^{2+}$), 1149.67551 ($[M+H]^+$).
$^1$H NMR (600 MHz, DMSO-$d_6$) δ 4.58 - 4.55 (m, 1H), 4.44 - 4.37 (m, 2H), 4.33 (dd, $J$ = 8.4, 4.2 Hz, 1H), 4.30 - 4.22 (m, 3H), 4.16 - 4.12 (m, 2H), 3.79 (d, $J$ = 6.1 Hz, 1H), 3.64 - 3.48 (m, 6H), 3.08 (t, $J$ = 7.0 Hz, 2H), 2.71 (d, $J$ = 8.2 Hz, 5H), 2.36 - 2.30 (m, 2H), 2.15 (t, $J$ = 7.6 Hz, 2H), 2.09 - 1.41 (m, AcOH, 46H), 1.37 - 1.29 (m, 4H), 1.16 (d, $J$ = 7.1 Hz, 3H), 0.85 (dd, $J$ = 20.7, 6.7 Hz, 6H).

**Compound 4:** Lys-Glu-Pro-Val-Pro-Gln-Ala-Lys (acetate salt)

High resolution mass spectrometry (TOF-HRMS), molecular formula: $C_{40}H_{59}N_{11}O_{12}$; m/z: 448.76653 ($[M+2H]^{2+}$), 896.52058 ($[M+H]^+$).
$^1$H NMR (600 MHz, DMSO-$d_6$) δ 4.56 (dd, $J$ = 9.0, 4.9 Hz, 1H), 4.39 (dd, $J$ = 8.3, 4.4 Hz, 1H), 4.31 - 4.24 (m, 3H), 4.13 (dd, $J$ = 9.1, 5.1 Hz, 2H), 3.78 (dd, $J$ = 7.9, 4.3 Hz, 1H), 3.67 - 3.51 (m, 4H), 2.75 - 2.70 (m, 4H), 2.38 - 2.29 (m, 2H), 2.14 (t, $J$ = 7.8 Hz, 2H), 2.04 - 1.47 (m, AcOH, 33H), 1.35 - 1.28 (m, 4H), 1.19 (d, $J$ = 7.2 Hz, 3H), 0.85 (dd, $J$ = 22.1, 6.8 Hz, 6H).

**Compound 5:** Pro-Arg-Lys-Val-Ala-Ala-Gln (acetate salt)

High resolution mass spectrometry (TOF-HRMS), molecular formula: $C_{33}H_{60}N_{12}O_9$; m/z: 385.24021 ([M+2H]$^{2+}$), 769.46971 ([M+H]$^+$).

$^1$H NMR (600 MHz, $D_2O$) δ 4.23 - 4.17 (m, 1H), 4.15 - 4.07 (m, 4H), 3.95 (dd, $J$ = 8.5, 4.8 Hz, 1H), 3.89 (d, $J$ = 7.7 Hz, 1H), 3.25 - 3.15 (m, 2H), 3.00 (t, $J$ = 7.0 Hz, 2H), 2.78 (t, $J$ = 7.7 Hz, 2H), 2.30 - 2.22 (m, 1H), 2.12 -2.05 (m, 2H), 1.93 - 1.80 (m, 5H), 1.75 (s, AcOH, 10H), 1.62 - 1.40 (m, 8H), 1.19 (d, $J$ = 7.2 Hz, 8H), 0.73 (dd, $J$ = 6.7, 4.0 Hz, 6H).

**Compound 6:** Glu-Pro-Val-Pro-Gln-Ala-Lys (acetate salt)

High resolution mass spectrometry (TOF-HRMS), molecular formula: $C_{34}H_{57}N_9O_{11}$; m/z: 768.42641 ([M+H]$^+$).

$^1$H NMR (600 MHz, $D_2O$) δ 4.46 (dd, $J$ = 8.4, 6.4 Hz, 1H), 4.35 - 4.27 (m, 3H), 4.26 - 4.18 (m, 2H), 4.09 (dd, $J$ = 8.2, 5.1 Hz, 1H), 3.84 - 3.77 (m, 1H), 3.68 - 3.52 (m, 3H), 2.91 (t, $J$ = 7.5 Hz, 2H), 2.39 (t, $J$ = 7.2 Hz, 2H), 2.33 (t, $J$ = 7.6 Hz, 2H), 2.27 - 2.17 (m, 2H), 2.12 - 1.87 (m, AcOH, 13H), 1.83 - 1.72 (m, 3H), 1.66 - 1.57 (m, 3H), 1.32 (dd, $J$ = 11.8, 7.5 Hz, 5H), 0.92 (dd, $J$ = 12.6, 6.7 Hz, 6H).

**Compound 7:** Lys-Glu-Pro-Val (acetate salt)

High resolution mass spectrometry (TOF-HRMS), molecular formula: $C_{21}H_{37}N_5O_7$; m/z: 472.27768 ([M+H]$^+$).

$^1$H NMR (600 MHz, $D_2O$) δ 4.43 (dd, $J$ = 10.2, 4.0 Hz, 1H), 4.27 (dd, $J$ = 8.3, 5.3 Hz, 1H), 3.85 - 3.80 (m, 2H), 3.68 - 3.60 (m, 1H), 3.59 - 3.46 (m, 1H), 2.80 (t, $J$ = 7.6 Hz, 2H), 2.22 - 2.14 (m, 2H), 2.10 - 2.06 (m, 1H), 1.96 - 1.64 (m, AcOH, 11H), 1.52 - 1.47 (m, 2H), 1.29 - 1.22 (m, 2H), 0.71 (dd, $J$ = 15.3, 6.9 Hz, 6H).

**Compound 8:** Val-Ala-Ala-Gln (acetate salt)

High resolution mass spectrometry (Orbitrap-HRMS), molecular formula: $C_{16}H_{29}N_5O_6$; m/z: 388.2187 ([M+H]$^+$).

$^1$H NMR (600 MHz, $D_2O$) δ 4.25 (q, $J$ = 7.2 Hz, 1H), 4.17 (q, $J$ = 7.2 Hz, 1H), 4.05 (dd, $J$ = 8.5, 4.8 Hz, 1H), 3.67 (d, $J$ = 6.0 Hz, 1H), 2.23 - 2.14 (m, 2H), 2.13 - 2.05 (m, 1H), 2.04 - 1.95 (m, 1H), 1.85 - 1.76 (m, 1H), 1.28 (dd, $J$ = 7.2, 5.0 Hz, 6H), 0.90 (dd, $J$ = 10.3, 6.9 Hz, 6H).

**Compound 9:** Glu-Pro-Val-Pro (acetate salt)

High resolution mass spectrometry (TOF-HRMS), molecular formula: $C_{20}H_{32}N_4O_7$; 441.23562 ([M+H]$^+$).

$^1$H NMR (600 MHz, DMSO-$d_6$) δ 4.42 (dd, $J$ = 8.4, 4.6 Hz, 1H), 4.28 (t, $J$ = 8.3 Hz, 1H), 4.17 (dd, $J$ = 8.5, 4.7 Hz, 1H), 3.78 (dd, $J$ = 8.3, 4.2 Hz, 1H), 3.64 - 3.45 (m, 4H), 2.37 - 2.27 (m, 2H), 2.08 - 1.58 (m, AcOH, 16H), 0.87 (dd, $J$ = 25.7, 6.7 Hz, 6H).

**Compound 10:** Lys-Pro-Arg-Lys(acetate salt)

High resolution mass spectrometry (TOF-HRMS), molecular formula: $C_{23}H_{45}N_9O_5$; 264.68527 ([M+2H]$^{2+}$), 528.36210 ([M+H]$^+$).

$^1$H NMR (600 MHz, DMSO-$d_6$) δ 4.42 (dd, $J$ = 8.2, 5.2 Hz, 1H), 4.23 (t, $J$ = 6.7 Hz, 1H), 4.14 (dd, $J$ = 8.9, 5.2 Hz, 1H), 4.09 (d, $J$ = 7.9 Hz, 1H), 3.64 (dd, $J$ = 9.9, 6.6 Hz, 1H), 3.45 - 3.40 (m, 1H), 3.07 (s, 2H), 2.72 (t, $J$ = 6.3 Hz, 4H), 2.11 - 2.04 (m, 1H), 1.92 - 1.66 (m, AcOH, 18H), 1.56 - 1.48 (m, 8H), 1.45 - 1.38 (m, 2H), 1.33 - 1.29 (m, 2H).

**Compound 11:** Ala-Lys-Pro-Arg (acetate salt)

High resolution mass spectrometry (TOF-HRMS), molecular formula: $C_{20}H_{38}N_8O_5$; 236.15638 ([M+2H]$^{2+}$), 471.30462 ([M+H]$^+$).

$^1$H NMR (600 MHz, $D_2O$)) δ 4.55 (dd, $J$ = 8.3, 5.6 Hz, 1H), 4.34 (dd, $J$ = 8.4, 5.9 Hz, 1H), 4.09 - 3.96 (m, 2H), 3.82 - 3.74 (m, 1H), 3.64 - 3.55 (m, 1H), 3.12 (t, $J$ = 6.9 Hz, 2H), 2.93 (t, $J$ = 7.6 Hz, 2H), 2.28 - 2.20 (m, 1H), 2.04 - 1.83 (m, 3H), 1.82 (s, AcOH, 6H), 1.79 - 1.60 (m, 6H), 1.58 - 1.51 (m, 2H), 1.42 (d, $J$ = 7.1 Hz, 5H).

**Compound 12:** Pro-Val-Pro-Gln (acetate salt)

High resolution mass spectrometry (TOF-HRMS), molecular formula: $C_{20}H_{33}N_5O_6$; m/z: 440.25122 ([M+H]$^+$).

$^1$H NMR (600 MHz, $D_2O$) δ 4.33 - 4.18 (m, 3H), 3.97 (dd, $J$ = 8.9, 4.8 Hz, 1H), 3.74 - 3.65 (m, 1H), 3.53 - 3.46 (m, 1H), 3.25 - 3.16 (m, 2H), 2.29 - 2.22 (m, 1H), 2.17 - 2.10 (m, 3H), 1.99 - 1.66 (m, AcOH, 12H), 0.82 (d, $J$ = 6.8 Hz, 3H), 0.76 (d, *J* = 6.7 Hz, 3H).

**Compound 13:** Val-Pro-Gln-Ala (acetate salt)

High resolution mass spectrometry (TOF-HRMS), molecular formula: $C_{18}H_{31}N_5O_6$; m/z: 414.23575 ([M+H]+).

$^1$H NMR (600 MHz, $D_2O$) δ 4.37 (t, *J* = 7.5 Hz, 1H), 4.15 (dd, *J* = 8.6, 60 Hz, 1H), 4.07 (d, *J* = 5.5 Hz, 1H), 4.03 (t, *J* = 7.2 Hz, 1H), 3.68 - 3.61 (m, 1H), 3.55 - 3.48 (m, 1H), 2.35 - 2.26 (m, 2H), 2.26 - 2.15 (m, 2H), 2.04 - 1.74 (m, AcOH, 7H), 1.22 (d, *J* = 7.2 Hz, 3H), 0.98 (d, *J* = 7.0 Hz, 3H), 0.87 (d, *J* = 6.8 Hz, 3H).

**Compound 14:** Pro-Gln-Ala-Lys (acetate salt)

High resolution mass spectrometry (TOF-HRMS), molecular formula: $C_{19}H_{34}N_6O_6$; m/z: 222.13480([M+2H]2+), 443.26183([M+H]+).

$^1$H NMR (600 MHz, $D_2O$) δ 4.28 (dd, *J* = 8.7, 6.1 Hz, 1H), 4.25 - 4.15 (m, 2H), 4.01 (dd, *J* = 8.1, 5.2 Hz, 1H), 3.34 - 3.22 (m, 2H), 2.86 (t, *J* = 7.5 Hz, 2H), 2.37 - 2.29 (m, 1H), 2.26 (t, *J* = 7.6 Hz, 2H), 2.00 - 1.84 (m, 5H), 1.73 - 1.64 (m, 1H), 1.62 - 1.50 (m, 3H), 1.30 - 1.24 (m, 5H).

**Compound 15:** Arg-Lys-Val-Ala (acetate salt)

High resolution mass spectrometry (TOF-HRMS), molecular formula: $C_{20}H_{40}N_8O_5$; m/z: 237.16465 ([M+2H]2+), 473.32087 ([M+H]+).

$^1$H NMR (600 MHz, $D_2O$) δ 4.28 (t, *J*= 7.3 Hz, 1H), 4.00 - 3.94 (m, 2H), 3.90 (t, *J*= 6.4 Hz, 1H), 3.07 (t, *J* = 7.0 Hz, 2H), 2.85 (t, *J*= 7.6 Hz, 2H), 2.01 - 1.92 (m, 1H), 1.80 - 1.75 (m, AcOH, 8H), 1.73 - 1.59 (m, 2H), 1.59 1.40 (m, 4H), 1.35 - 1.22 (m, 2H), 1.19 (d, *J* = 7.2 Hz, 3H), 0.81 (t, *J* = 6.9 Hz, 6H).

**Compound 16:** Lys-Val-Ala-Ala (acetate salt)

High resolution mass spectrometry (TOF-HRMS), molecular formula: $C_{17}H_{33}N_5O_5$; m/z: 388.25660 ([M+H]+).

$^1$H NMR (600 MHz, $D_2O$) δ 4.18 (q, *J*= 7.1 Hz, 1H), 4.03 - 3.91 (m, 3H), 2.87 (t, *J*= 7.7 Hz, 2H), 1.98 - 1.89 (m, 1H), 1.83 - 1.74 (m, AcOH, 6H), 1.60 - 1.53 (m, 2H), 1.36 - 1.27 (m, 2H), 1.26 (d, *J*= 7.2 Hz, 3H), 1.19 (d, *J*= 7.2 Hz, 3H), 0.83 (d, *J* = 6.8 Hz, 6H).

**Compound 17:** Gln-Ala-Lys-Pro (acetate salt)

High resolution mass spectrometry (TOF-HRMS), molecular formula: $C_{19}H_{34}N_6O_6$; m/z: 443.26161([M+H]+).

$^1$H NMR (600 MHz, $D_2O$) δ 4.48 (dd, *J*= 8.2, 5.6 Hz, 1H), 4.26 - 4.20 (m, 1H), 4.10 (dd, *J*= 8.5, 5.4 Hz, 1H), 3.91 (t, *J* = 6.6 Hz, 1H), 3.68 - 3.61 (m, 1H), 3.55 - 3.43 (m, 1H), 2.88 (t, *J*= 7.5 Hz, 2H), 2.39 - 2.27 (m, 2H), 2.15 - 1.96 (m, 3H), 1.92 - 1.69 (m, 8H), 1.66 - 1.49 (m, 3H), 1.43 - 1.29 (m, 2H), 1.28 - 1.22 (m, 3H).

**Compound 18:** Pro-Arg-Lys-Val (acetate salt)

High resolution mass spectrometry (TOF-HRMS), molecular formula: $C_{22}H_{42}N_8O_5$; m/z: 250.17158 ([M+2H]2+), 499.33464 ([M+H]+).

$^1$H NMR (600 MHz, $D_2O$) δ 4.27 (dd, *J* = 8.7, 6.0 Hz, 1H), 4.24 - 4.17 (m, 2H), 3.88 (d, *J* = 6.2 Hz, 1H), 3.33 - 3.20 (m, 2H), 3.09 - 3.02 (m, 2H), 2.85 (t, *J*= 7.5 Hz, 2H), 2.37 - 2.28 (m, 1H), 1.98 - 1.86 (m, 4H), 1.78 (s, AcOH, 6H), 1.72 - 1.42 (m, 8H), 1.38 - 1.23 (m, 2H), 0.75 (dd, *J*= 11.2, 6.8 Hz, 6H).

**Compound 19:** Ala-Glu-Pro-Val-Pro-Gln-Ala-Lys-Pro-Arg-Lys-Val-Ala-Ala-Gln (acetate salt)

High resolution mass spectrometry (TOF-HRMS), molecular formula: $C_{70}H_{120}N_{22}O_{20}$; m/z: 530.64499 ([M+3H]3+), 795.46224 ([M+2H]2+), 1589.91350 ([M+H]+).

$^1$H NMR (600 MHz, $D_2O$) δ 4.53 - 4.43 (m, 2H), 4.33 (dd, *J*= 8.3, 6.0 Hz, 1H), 4.27 (t, *J*= 7.7 Hz, 3H), 4.24 - 4.12 (m, 6H), 4.06 - 3.94 (m, 3H), 3.77 (q, *J* = 7.6 Hz, 1H), 3.73 - 3.67 (m, 2H), 3.63 - 3.53 (m, 2H), 3.51 (q, *J* = 7.6 Hz, 1H), 3.08 (t, *J* = 6.9 Hz, 2H), 2.91 - 2.83 (m, 4H), 2.29 (t, *J* = 7.6 Hz, 2H), 2.26 - 2.12 (m, 7H), 2.03 - 1.46 (m, AcOH, 38H), 1.39 (d, *J* = 7.1 Hz, 4H), 1.36 - 1.30 (m, 2H), 1.30 - 1.22 (m, 10H), 0.92 - 0.77 (m, 12H).

**Compound 20:** Lys-Ala-Pro-Val-Pro-Gln-Ala-Lys-Pro-Arg-Lys-Val-Ala-Ala-Gln (acetate salt)

High resolution mass spectrometry (TOF-HRMS), molecular formula: $C_{71}H_{125}N_{23}O_{18}$; m/z: 530.32804 ($[M+3H]^{3+}$), 794.98683 ($[M+2H]^{2+}$), 1588.96546 ($[M+H]^+$).

$^1$H NMR (600 MHz, $D_2O$) δ 4.51 (q, $J = 7.1$ Hz, 1H), 4.48 - 4.43 (m, 1H), 4.31 - 4.25 (m, 3H), 4.24 - 4.11 (m, 6H), 4.03 (dd, $J = 8.4$, 4.9 Hz, 1H), 3.98 (d, $J = 7.6$ Hz, 1H), 3.87 (t, $J = 6.7$ Hz, 1H), 3.79 - 3.67 (m, 3H), 3.60 - 3.47 (m, 3H), 3.08 (t, $J = 6.9$ Hz, 2H), 2.91 - 2.83 (m, 6H), 2.28 (t, $J = 7.6$ Hz, 2H), 2.21 - 2.13 (m, 5H), 2.03 - 1.64 (m, AcOH, 33H), 1.64 - 1.47 (m, 11H), 1.44 - 1.28 (m, 6H), 1.28 - 1.21 (m, 12H), 0.89 - 0.76 (m, 12H).

**Compound 21:** Lys-Glu-Ala-Val-Pro-Gln-Ala-Lys-Pro-Arg-Lys-Val-Ala-Ala-Gln (acetate salt)

High resolution mass spectrometry (TOF-HRMS), molecular formula: $C_{71}H_{125}N_{23}O_{20}$; m/z: 540.99125 ($[M+3H]^{3+}$), 810.98235 ($[M+2H]^{2+}$), 1620.94722 ($[M+H]^+$).

$^1$H NMR (600 MHz, $D_2O$) δ 4.45 (dd, $J = 9.4$, 4.9 Hz, 1H), 4.30 - 4.24 (m, 3H), 4.24 - 4.11 (m, 8H), 4.02 (dd, $J = 8.4$, 4.9 Hz, 1H), 3.97 (d, $J = 7.6$ Hz, 1H), 3.89 (t, $J = 6.7$ Hz, 1H), 3.79 - 3.66 (m, 2H), 3.60 - 3.47 (m, 2H), 3.08 (t, $J = 6.9$ Hz, 2H), 2.91 - 2.83 (m, 6H), 2.28 (t, $J = 7.6$ Hz, 2H), 2.22 - 2.12 (m, 6H), 2.02 - 1.46 (m, AcOH, 41H), 1.43 - 1.28 (m, 6H), 1.28 - 1.20 (m, 12H), 0.88 - 0.75 (m, 12H).

**Compound 22:** Lys-Glu-Pro-Val-Ala-Gln-Ala-Lys-Pro-Arg-Lys-Val-Ala-Ala-Gln (acetate salt)

High resolution mass spectrometry (TOF-HRMS), molecular formula: $C_{71}H_{125}N_{23}O_{20}$; m/z: 540.99088($[M+3H]^{3+}$), 810.98168($[M+2H]^{2+}$), 1620.95237($[M+H]^+$).

$^1$H NMR (600 MHz, $D_2O$) δ 4.51 (dd, $J = 9.6$, 4.4 Hz, 1H), 4.46 (dd, $J = 9.4$, 4.8 Hz, 1H), 4.35 (dd, $J= 8.4$, 5.8 Hz, 1H), 4.28 (dd, $J = 8.3$, 6.1 Hz, 1H), 4.25 - 4.13 (m, 7H), 4.03 (dd, $J = 8.3$, 4.9 Hz, 1H), 3.98 (d, $J = 7.6$ Hz, 1H), 3.94 - 3.88 (m, 2H), 3.75 - 3.67 (m, 2H), 3.63 - 3.56 (m, 1H), 3.51 (q, $J = 7.3$ Hz, 1H), 3.09 (t, $J = 6.9$ Hz, 2H), 2.91 - 2.83 (m, 6H), 2.29 - 2.21 (m, 4H), 2.21 - 2.13 (m, 4H), 2.03 - 1.65 (m, AcOH, 31H), 1.64 - 1.46 (m, 11H), 1.45 - 1.28 (m, 6H), 1.28 - 1.22 (m, 12H), 0.89 - 0.78 (m, 12H).

**Compound 23:** Lys-Glu-Pro-Val-Pro-Ala-Ala-Lys-Pro-Arg-Lys-Val-Ala-Ala-Gln (acetate salt)

High resolution mass spectrometry (Orbitrap-HRMS), molecular formula: $C_{71}H_{124}N_{22}O_{19}$; m/z: 398.24191 ($[M+4H]^{4+}$), 530.65344 ($[M+3H]^{3+}$), 795.97791 ($[M+2H]^{2+}$).

$^1$H NMR (600 MHz, $D_2O$) δ 4.51 (dd, $J = 9.6$, 4.4 Hz, 1H), 4.44 (dd, $J = 9.4$, 4.9 Hz, 1H), 4.33 (dd, $J = 8.4$, 5.8 Hz, 1H), 4.31 - 4.08 (m, 9H), 4.03 (dd, $J = 8.4$, 4.9 Hz, 1H), 3.97 (d, $J = 7.6$ Hz, 1H), 3.92 - 3.87 (m, 1H), 3.79 - 3.73 (m, 1H), 3.73 - 3.66 (m, 2H), 3.62 - 3.52 (m, 2H), 3.51 - 3.46 (m, 1H), 3.07 (t, $J = 6.9$ Hz, 2H), 2.90 - 2.81 (m, 6H), 2.29 - 2.21 (m, 2H), 2.21 - 2.10 (m, 5H), 2.02 - 1.64 (m, AcOH, 37H), 1.64 - 1.45 (m, 11H), 1.44 - 1.27 (m, 6H), 1.27 - 1.20 (m, 12H), 0.91 - 0.74 (m, 12H).

**Compound 24:** Lys-Glu-Pro-Val-Pro-Gln-Ala-Ala-Pro-Arg-Lys-Val-Ala-Ala-Gln (acetate salt)

High resolution mass spectrometry (Orbitrap-HRMS), molecular formula: $C_{70}H_{120}N_{22}O_{20}$; m/z: 398.23245($[M+4H]^{4+}$), 530.64075($[M+3H]^{3+}$), 795.45752($[M+2H]^{2+}$).

$^1$H NMR (600 MHz, $D_2O$) δ 4.51 (dd, $J = 9.7$, 4.4 Hz, 1H), 4.45 (q, $J = 7.1$ Hz, 1H), 4.33 (dd, $J = 8.4$, 5.8 Hz, 1H), 4.31 - 4.24 (m, 3H), 4.23 - 4.12 (m, 6H), 4.03 (dd, $J = 8.4$, 4.8 Hz, 1H), 3.97 (d, $J = 7.6$ Hz, 1H), 3.92 - 3.87 (m, 1H), 3.79 - 3.73 (m, 1H), 3.73 - 3.64 (m, 2H), 3.62 - 3.48 (m, 3H), 3.08 (t, $J= 6.9$ Hz, 2H), 2.91 - 2.83 (m, 4H), 2.32 - 2.21 (m, 4H), 2.20 - 2.11 (m, 5H), 2.03 - 1.71 (m, AcOH, 32H), 1.71 - 1.45 (m, 10H), 1.37 - 1.27 (m, 4H), 1.27 - 1.17 (m, 12H), 0.90 - 0.76 (m, 12H).

**Compound 25:** Lys-Glu-Pro-Ala-Pro-Gln-Ala-Lys-Pro-Arg-Lys-Val-Ala-Ala-Gln (acetate salt)

High resolution mass spectrometry (Orbitrap-HRMS), molecular formula: $C_{71}H_{123}N_{23}O_{20}$; m/z: 405.48935 ($[M+4H]^{4+}$), 540.31677 ($[M+3H]^{3+}$), 809.97144 ($[M+2H]^{2+}$).

$^1$H NMR (600 MHz, $D_2O$) δ 4.51 (dd, $J = 9.5$, 4.4 Hz, 1H), 4.48 - 4.41 (m, 2H), 4.32 - 4.25 (m, 3H), 4.21 (dd, $J = 8.1$, 6.7 Hz, 1H), 4.19 - 4.12 (m, 5H), 4.02 (dd, $J = 8.4$, 4.9 Hz, 1H), 3.97 (d, $J = 7.6$ Hz, 1H), 3.92 - 3.85 (m, 1H), 3.74 - 3.64 (m, 3H), 3.60 - 3.46 (m, 3H), 3.07 (t, $J = 6.9$ Hz, 2H), 2.90 - 2.83 (m, 6H), 2.30 - 2.20 (m, 4H), 2.20 - 2.12 (m, 5H), 2.02 - 1.45 (m, AcOH, 48H), 1.43 - 1.27 (m, 6H), 1.27 - 1.21 (m, 12H), 0.80 (dd, $J = 6.8$, 4.4 Hz, 6H).

**Compound 26:** Lys-Glu-Pro-Val-Pro-Gln-Ala-Lys-Ala-Arg-Lys-Val-Ala-Ala-Gln (acetate salt)

High resolution mass spectrometry (Orbitrap-HRMS), molecular formula: $C_{71}H_{125}N_{23}O_{20}$; m/z: 405.99350 ($[M+4H]^{4+}$), 540.98883 ($[M+3H]^{3+}$), 810.97961 ($[M+2H]^{2+}$).

$^1$H NMR (600 MHz, $D_2O$) δ 4.51 (dd, $J$ = 9.6, 4.4 Hz, 1H), 4.33 (dd, $J$ = 8.4, 5.8 Hz, 1H), 4.31 - 4.23 (m, 2H), 4.23 - 4.09 (m, 8H), 4.03 (dd, $J$ = 8.4, 4.9 Hz, 1H), 3.97 (d, $J$ = 7.6 Hz, 1H), 3.93 - 3.86 (m, 1H), 3.81 - 3.66 (m, 2H), 3.62 - 3.52 (m, 2H), 3.07 (t, $J$ = 6.9 Hz, 2H), 2.86 (m, 6H), 2.31 - 2.11 (m, 8H), 2.03 - 1.41 (m, AcOH, 48H), 1.40 - 1.27 (m, 6H), 1.25 (dd, $J$ = 13.7, 7.3 Hz, 12H), 0.90 - 0.76 (m, 12H).

**Compound 27:** Lys-Glu-Pro-Val-Pro-Gln-Ala-Lys-Pro-Ala-Lys-Val-Ala-Ala-Gln (acetate salt)

High resolution mass spectrometry (Orbitrap-HRMS), molecular formula: $C_{70}H_{120}N_{20}O_{20}$ ; m/z: 391.23177 ($[M+4H]^{4+}$), 781.45496 ($[M+2H]^{2+}$).

$^1$H NMR (600 MHz, $D_2O$) δ 4.52 - 4.43 (m, 2H), 4.33 (dd, $J$ = 8.4, 5.8 Hz, 1H), 4.31 - 4.22 (m, 3H), 4.21 - 4.10 (m, 6H), 4.02 (dd, $J$ = 8.5, 4.8 Hz, 1H), 3.96 (d, $J$ = 7.7 Hz, 1H), 3.92 - 3.84 (m, 1H), 3.81 - 3.73 (m, 1H), 3.73 - 3.65 (m, 2H), 3.61 - 3.52 (m, 2H), 3.52 - 3.45 (m, 1H), 2.91 - 2.81 (m, 6H), 2.30 - 2.10 (m, 9H), 2.02 - 1.62 (m, AcOH, 31H), 1.62 - 1.51 (m, 8H), 1.41 - 1.27 (m, 6H), 1.27 - 1.20 (m, 12H), 0.90 - 0.75 (m, 12H).

**Compound 28:** Lys-Glu-Pro-Val-Pro-Gln-Ala-Lys-Pro-Arg-Ala-Val-Ala-Ala-Gln (acetate salt)

High resolution mass spectrometry (Orbitrap-HRMS), molecular formula: $C_{70}H_{120}N_{22}O_{20}$; m/z: 398.23303($[M+4H]^{4+}$), 530.64142($[M+3H]^{3+}$), 795.45850($[M+2H]^{2+}$).

$^1$H NMR (600 MHz, $D_2O$) δ 4.52 - 4.42 (m, 2H), 4.33 (dd, $J$ = 8.4, 5.8 Hz, 1H), 4.30 - 4.24 (m, 3H), 4.23 - 4.09 (m, 6H), 4.02 (dd, $J$ = 8.5, 4.8 Hz, 1H), 3.95 (d, $J$ = 7.5 Hz, 1H), 3.92 - 3.85 (m, 1H), 3.79 - 3.73 (m, 1H), 3.72 - 3.67 (m, 2H), 3.62 - 3.42 (m, 3H), 3.08 (t, $J$ = 6.9 Hz, 2H), 2.87 (t, $J$ = 7.6 Hz, 4H), 2.30 - 2.25 (m, 2H), 2.24 - 2.10 (m, 7H), 2.02 - 1.65 (m, AcOH, 29H), 1.64 - 1.48 (m, 8H), 1.42 - 1.29 (m, 4H), 1.28 - 1.19 (m, 12H), 0.90 - 0.75 (m, 12H).

**Compound 29:** Lys-Glu-Pro-Val-Pro-Gln-Ala-Lys-Pro-Arg-Lys-Ala-Ala-Ala-Gln (acetate salt)

High resolution mass spectrometry (Orbitrap-HRMS), molecular formula: $C_{71}H_{123}N_{23}O_{20}$; m/z: 405.48969($[M+4H]^{4+}$), 540.31714($[M+3H]^{3+}$), 809.97205($[M+2H]^{2+}$).

$^1$H NMR (600 MHz, $D_2O$) δ 4.49 (dd, $J$ = 9.6, 4.3 Hz, 1H), 4.45 (dd, $J$ = 9.3, 4.9 Hz, 1H), 4.33 (dd, $J$ = 8.4, 5.8 Hz, 1H), 4.29 - 4.24 (m, 3H), 4.19 - 4.13 (m, 7H), 4.02 (dd, $J$ = 8.4, 4.8 Hz, 1H), 3.89 (t, $J$ = 6.8 Hz, 1H), 3.79 - 3.66 (m, 3H), 3.61 - 3.47 (m, 3H), 3.08 (t, $J$ = 6.9 Hz, 2H), 2.86 (q, $J$ = 8.0 Hz, 6H), 2.28 (t, $J$ = 7.6 Hz, 2H), 2.23 - 2.13 (m, 7H), 2.02 - 1.46 (m, AcOH, 47H), 1.42 - 1.18 (m, 20H), 0.85 (dd, $J$ = 14.2, 6.7 Hz, 6H).

**Compound 30:** Lys-Glu-Pro-Val-Pro-Gln-Ala-Lys-Pro-Arg-Lys-Val-Ala-Ala-Ala (acetate salt)

High resolution mass spectrometry (TOF-HRMS), molecular formula: $C_{71}H_{124}N_{22}O_{19}$; m/z: 398.24493 ($[M+4H]^{4+}$), 530.65551 ($[M+3H]^{3+}$), 795.47815 ($[M+2H]^{2+}$), 1589.94194 ($[M+H]^+$).

$^1$H NMR (600 MHz, $D_2O$) δ 4.51 (dd, $J$ = 9.6, 4.4 Hz, 1H), 4.46 (dd, $J$ = 9.4, 4.9 Hz, 1H), 4.34 (dd, $J$ = 8.4, 5.8 Hz, 1H), 4.31 - 4.24 (m, 3H), 4.24 - 4.20 (m, 1H), 4.20 - 4.09 (m, 5H), 4.02 - 3.96 (m, 2H), 3.93 - 3.86 (m, 1H), 3.80 - 3.74 (m, 1H), 3.74 - 3.67 (m, 2H), 3.63 - 3.43 (m, 3H), 3.08 (t, $J$ = 6.9 Hz, 2H), 2.91 - 2.82 (m, 6H), 2.31 - 2.12 (m, 7H), 2.01 - 1.65 (m, AcOH, 34H), 1.64 - 1.46 (m, 11H), 1.35 (dd, $J$ = 16.0, 8.1 Hz, 6H), 1.27 - 1.20 (m, 12H), 0.92 - 0.77 (m, 12H).

**Compound 31:** Lys-Glu-Gly-Val-Pro-Gln-Ala-Lys-Pro-Arg-Lys-Val-Ala-Ala-Gln (acetate salt)

High resolution mass spectrometry (TOF-HRMS), molecular formula: $C_{70}H_{123}N_{23}O_{20}$; m/z: 402.49240 ($[M+4H]^{4+}$), 536.31955 ($[M+3H]^{3+}$), 803.97442 ($[M+2H]^{2+}$), 1606.91471 ($[M+H]^+$).

$^1$H NMR (600 MHz, $D_2O$) δ 4.45 (dd, $J$ = 9.4, 4.9 Hz, 1H), 4.32 (d, $J$ = 8.1 Hz, 1H), 4.30 - 4.25 (m, 2H), 4.24 - 4.19 (m, 2H), 4.19 - 4.11 (m, 5H), 4.02 (dd, $J$ = 8.4, 4.9 Hz, 1H), 3.97 (d, $J$ = 7.6 Hz, 1H), 3.91 - 3.84 (m, 2H), 3.82 - 3.73 (m, 2H), 3.69 (q, $J$ = 7.7, 7.0 Hz, 1H), 3.59 - 3.52 (m, 1H), 3.52 - 3.46 (m, 1H), 3.07 (t, $J$ = 6.9 Hz, 2H), 2.90 - 2.82 (m, 6H), 2.28 (t, $J$ = 7.6 Hz, 2H), 2.23 - 2.12 (m, 6H), 2.02 - 1.64 (m, AcOH, 35H), 1.63 - 1.45 (m, 11H), 1.44 - 1.27 (m, 6H), 1.27 - 1.21 (m, 9H), 0.88 - 0.73 (m, 12H).

**Compound 32:** Lys-Glu-Pro-Val-Gly-Gln-Ala-Lys-Pro-Arg-Lys-Val-Ala-Ala-Gln (acetate salt)

High resolution mass spectrometry (TOF-HRMS), molecular formula: $C_{70}H_{123}N_{23}O_{20}$; m/z:

402.49278([M+4H]$^{4+}$), 536.31975([M+3H]$^{3+}$), 803.97422([M+2H]$^{2+}$), 1606.92969([M+H]$^+$).

$^1$H NMR (600 MHz, D$_2$O) δ 4.51 (dd, $J$ = 9.7, 4.4 Hz, 1H), 4.46 (dd, $J$ = 9.4, 4.9 Hz, 1H), 4.36 (dd, $J$ = 8.3, 5.9 Hz, 1H), 4.28 (dd, $J$ = 8.3, 6.1 Hz, 1H), 4.24 - 4.14 (m, 6H), 4.03 (dd, $J$ = 8.3, 4.9 Hz, 1H), 3.97 (dd, $J$ = 10.0, 7.5 Hz, 2H), 3.90 (t, $J$ = 6.8 Hz, 1H), 3.87 - 3.78 (m, 2H), 3.76 - 3.67 (m, 2H), 3.61 - 3.54 (m, 1H), 3.54 - 3.47 (m, 1H), 3.08 (t, $J$ = 6.9 Hz, 2H), 2.91 - 2.83 (m, 6H), 2.26 - 2.13 (m, 8H), 2.03 - 1.87 (m, 9H), 1.87 - 1.45 (m, AcOH, 33H), 1.44 - 1.28 (m, 6H), 1.26 (dd, $J$ = 18.4, 7.2 Hz, 9H), 0.89 - 0.79 (m, 12H).

**Compound 33:** Lys-Glu-Pro-Val-Pro-Gln-Ala-Lys-Gly-Arg-Lys-Val-Ala-Ala-Gln (acetate salt)

High resolution mass spectrometry (Orbitrap-HRMS), molecular formula: C$_{70}$H$_{123}$N$_{23}$O$_{20}$; m/z: 402.48917 ([M+4H]$^{4+}$), 536.31647 ([M+3H]$^{3+}$), 803.97101 ([M+2H]$^{2+}$).

$^1$H NMR (600 MHz, D$_2$O) δ 4.51 (dd, $J$ = 9.6, 4.4 Hz, 1H), 4.34 (dd, $J$ = 8.4, 5.8 Hz, 1H), 4.31 - 4.11 (m, 9H), 4.03 (dd, $J$ = 8.4, 4.9 Hz, 1H), 3.97 (d, $J$ = 7.6 Hz, 1H), 3.92 - 3.86 (m, 1H), 3.85 - 3.74 (m, 3H), 3.74 - 3.66 (m, 1H), 3.62 - 3.52 (m, 2H), 3.06 (t, $J$ = 7.0 Hz, 2H), 2.91 - 2.83 (m, 6H), 2.31 - 2.11 (m, 8H), 2.03 - 1.43 (m, AcOH, 45H), 1.40 - 1.27 (m, 6H), 1.27 - 1.23 (m, 9H), 0.91 - 0.77 (m, 12H).

**Compound 34:** Lys-Glu-Pro-Val-Gly-Gln-Ala-Lys-Gly-Arg-Lys-Val-Ala-Ala-Gln (acetate salt)

High resolution mass spectrometry (Orbitrap-HRMS), molecular formula: C$_{67}$H$_{119}$N$_{23}$O$_{20}$; m/z: 392.48178 ([M+4H]$^{4+}$), 522.97314 ([M+3H]$^{3+}$), 784.45752 ([M+2H]$^{2+}$).

$^1$H NMR (600 MHz, D$_2$O) δ 4.51 (dd, $J$ = 9.6, 4.4 Hz, 1H), 4.35 (dd, $J$ = 8.3, 5.9 Hz, 1H), 4.25 - 4.12 (m, 7H), 4.03 (dd, $J$ = 8.4, 4.8 Hz, 1H), 3.96 (dd, $J$ = 10.6, 7.5 Hz, 2H), 3.92 - 3.86 (m, 1H), 3.85 - 3.77 (m, 4H), 3.75 - 3.68 (m, 1H), 3.60 - 3.53 (m, 1H), 3.06 (t, $J$ = 7.0 Hz, 2H), 2.92 - 2.83 (m, 6H), 2.28 - 2.12 (m, 7H), 2.05 - 1.41 (m, AcOH, 42H), 1.40 - 1.27 (m, 6H), 1.27 - 1.24 (m, 9H), 0.85 (dd, $J$ = 11.0, 6.7 Hz, 6H), 0.80 (dd, $J$ = 6.8, 5.1 Hz, 6H).

**Compound 35:** Lys-Glu-Gly-Val-Gly-Gln-Ala-Lys-Gly-Arg-Lys-Val-Ala-Ala-Gln (acetate salt)

High resolution mass spectrometry (Orbitrap-HRMS), molecular formula: C$_{64}$H$_{115}$N$_{23}$O$_{20}$; m/z: 382.47363 ([M+4H]$^{4+}$), 509.62903 ([M+3H]$^{3+}$), 763.93982 ([M+2H]$^{2+}$).

$^1$H NMR (600 MHz, D$_2$O) δ 4.27 - 4.13 (m, 8H), 4.05 - 4.00 (m, 2H), 3.97 (d, $J$ = 7.6 Hz, 1H), 3.93 - 3.87 (m, 2H), 3.85 - 3.76 (m, 5H), 3.06 (t, $J$ = 7.0 Hz, 2H), 2.90 - 2.83 (m, 6H), 2.24 - 2.12 (m, 6H), 2.02 - 1.41 (m, AcOH, 40H), 1.39 - 1.27 (m, 6H), 1.27 - 1.22 (m, 9H), 0.85 - 0.76 (m, 12H).

**Compound 36:** Lys-Glu-Gly-Val-Pro-Gln-Ala-Lys-Gly-Arg-Lys-Val-Ala-Ala-Gln (acetate salt)

High resolution mass spectrometry (Orbitrap-HRMS), molecular formula: C$_{67}$H$_{119}$N$_{23}$O$_{20}$; m/z: 392.48172([M+4H]$^{4+}$), 522.97314([M+3H]$^{3+}$), 783.95605([M+2H]$^{2+}$).

$^1$H NMR (600 MHz, D$_2$O) δ 4.32 (d, $J$ = 8.0 Hz, 1H), 4.28 (dd, $J$ = 8.2, 6.7 Hz, 1H), 4.25 - 4.12 (m, 8H), 4.03 (dd, $J$ = 8.4, 4.8 Hz, 1H), 3.97 (d, $J$ = 7.6 Hz, 1H), 3.92 - 3.83 (m, 2H), 3.83 - 3.73 (m, 4H), 3.60 - 3.53 (m, 1H), 3.06 (t, $J$ = 7.0 Hz, 2H), 2.91 - 2.83 (m, 6H), 2.28 (t, $J$ = 7.5 Hz, 2H), 2.24 - 2.12 (m, 5H), 2.02 - 1.42 (m, AcOH, 43H), 1.41 - 1.27 (m, 6H), 1.27 - 1.22 (m, 9H), 0.91 - 0.72 (m, 12H).

**Compound 37:** Lys-Glu-Gly-Val-Gly-Gln-Ala-Lys-Pro-Arg-Lys-Val-Ala-Ala-Gln (acetate salt)

High resolution mass spectrometry (Orbitrap-HRMS), molecular formula: C$_{67}$H$_{119}$N$_{23}$O$_{20}$; m/z: 392.48172([M+4H]$^{4+}$), 522.97314([M+3H]$^{3+}$), 783.95605([M+2H]$^{2+}$).

$^1$H NMR (600 MHz, D$_2$O) δ 4.44 (dd, $J$ = 9.4, 4.9 Hz, 1H), 4.29 - 4.12 (m, 8H), 4.04 - 3.99 (m, 2H), 3.96 (d, $J$ = 7.6 Hz, 1H), 3.91 - 3.82 (m, 3H), 3.81 (s, 2H), 3.72 - 3.65 (m, 1H), 3.52 - 3.44 (m, 1H), 3.06 (t, $J$ = 6.9 Hz, 2H), 2.89 - 2.81 (m, 6H), 2.23 - 2.12 (m, 7H), 2.01 - 1.45 (m, AcOH, 43H), 1.42 - 1.26 (m, 6H), 1.26 - 1.20 (m, 9H), 0.83 - 0.77 (m, 12H).

**Compound 38:** Glu-Pro-Val-Pro-Gln-Ala-Lys-Pro-Arg-Lys (acetate salt)

High resolution mass spectrometry (Orbitrap-HRMS), molecular formula: C$_{51}$H$_{88}$N$_{16}$O$_{14}$; m/z: 383.89569 ([M+3H]$^{3+}$), 575.33978 ([M+2H]$^{2+}$).

$^1$H NMR (600 MHz, D$_2$O) δ 4.45 (dd, $J$ = 9.2, 5.0 Hz, 1H), 4.39 (dd, $J$ = 8.3, 6.4 Hz, 1H), 4.29 - 4.21 (m, 4H), 4.19 - 4.12 (m, 3H), 4.01 (dd, $J$ = 7.8, 5.5 Hz, 1H), 3.78 - 3.73 (m, 1H), 3.72 - 3.67 (m, 1H), 3.61 - 3.48 (m, 4H), 3.08 (t, $J$ = 6.8 Hz, 2H), 2.88 - 2.83 (m, 4H), 2.30 - 2.26 (m, 4H), 2.20 - 2.13 (m, 3H), 2.08 - 1.46 (m, AcOH, 38H), 1.39 - 1.22 (m, 7H), 0.86 (dd, $J$ = 10.3, 6.7 Hz, 6H).

**Compound 39:** Pro-Val-Pro-Gln-Ala (acetate salt)

High resolution mass spectrometry (Orbitrap-HRMS), molecular formula: $C_{23}H_{38}N_6O_7$; m/z: 511.2871 ([M+H]$^+$).

$^1$H NMR (600 MHz, $D_2O$) $\delta$ 4.41 (d, $J$ = 7.3 Hz, 1H), 4.38 - 4.32 (m, 2H), 4.20 (dd, $J$ = 8.7, 5.9 Hz, 1H), 4.11 - 4.04 (m, 1H), 3.85 - 3.77 (m, 1H), 3.62 (dt, $J$ = 10.3, 7.2 Hz, 1H), 3.39 - 3.29 (m, 2H), 2.39 - 2.31 (m, 3H), 2.28 - 2.20 (m, 1H), 2.08 - 1.90 (m, AcOH, 10H), 1.86 - 1.80 (m, 1H), 1.26 (d, $J$ = 7.3 Hz, 3H), 0.94 (d, $J$ = 6.8 Hz, 3H), 0.88 (d, $J$= 6.8 Hz, 3H).

**Compound 40:** Pro-Val-Pro-Glu-Ala (acetate salt)

High resolution mass spectrometry (Orbitrap-HRMS), molecular formula: $C_{23}H_{37}N_5O_8$; m/z: 512.27081 ([M+H]$^+$).

$^1$H NMR (600 MHz, $D_2O$) $\delta$ 4.41 (d, $J$ = 7.3 Hz, 1H), 4.38 - 4.32 (m, 2H), 4.23 (dd, $J$ = 8.9, 5.6 Hz, 1H), 4.13 - 4.09 (m, 1H), 3.86 - 3.76 (m, 1H), 3.62 (dt, $J$ = 10.2, 7.2 Hz, 1H), 3.38 - 3.28 (m, 2H), 2.43 - 2.35 (m, 3H), 2.26 - 2.16 (m, 1H), 2.12 - 1.72 (m, AcOH, 12H), 1.28 (d, $J$ = 7.2 Hz, 3H), 0.94 (d, $J$ = 6.8 Hz, 3H), 0.87 (d, $J$ = 6.8 Hz, 3H).

**Compound 41:** Pro-Val-Pro-Ile-Ala (acetate salt)

High resolution mass spectrometry (Orbitrap-HRMS), molecular formula: $C_{24}H_{41}N_5O_6$; m/z: 496.31158 ([M+H]$^+$).

$^1$H NMR (600 MHz, $D_2O$) $\delta$ 4.36 - 4.27 (m, 3H), 4.03 (q, $J$ = 7.2 Hz, 1H), 3.98 (d, $J$ = 7.8 Hz, 1H), 3.79 - 3.72 (m, 1H), 3.60 - 3.53 (m, 1H), 3.34 - 3.21 (m, 2H), 2.36 - 2.25 (m, 1H), 2.21 - 2.12 (m, 1H), 2.03 - 1.96 (m, 1H), 1.96 - 1.81 (m, AcOH, 7H), 1.80 - 1.69 (m, 2H), 1.43 - 1.33 (m, 1H), 1.20 (d, $J$ = 7.2 Hz, 3H), 1.13 - 1.02 (m, 1H), 0.88 (d, $J$ = 6.8 Hz, 3H), 0.85 - 0.80 (m, 6H), 0.80 - 0.77 (m, 1H), 0.74 (t, $J$ = 7.4 Hz, 3H).

**Compound 42:** Pro-Val-Pro-Ala

High resolution mass spectrometry (Orbitrap-HRMS), molecular formula: $C_{18}H_{30}N_4O_5$; m/z: 383.22791 ([M+H]$^+$).

$^1$H NMR (600 MHz, $D_2O$) $\delta$ 4.36 (d, $J$ = 7.2 Hz, 1H), 4.30 (dd, $J$ = 8.5, 6.3 Hz, 1H), 4.24 (dd, $J$ = 8.1, 6.5 Hz, 1H), 4.00 (q, $J$ = 7.2 Hz, 1H), 3.79 - 3.72 (m, 1H), 3.60 - 3.52 (m, 1H), 3.31 - 3.23 (m, 2H), 2.33 - 2.26 (m, 1H), 2.20 - 2.13 (m, 1H), 2.09 - 1.69 (m, AcOH, 10H), 1.21 (d, $J$ = 7.3 Hz, 3H), 0.88 (d, $J$ = 6.9 Hz, 3H), 0.81 (d, $J$ = 6.8 Hz, 3H).

**Compound 43:** Glu-Pro-Val-Pro-Gln-Ala (acetate salt)

High resolution mass spectrometry (Orbitrap-HRMS), molecular formula: $C_{28}H_{45}N_7O_{10}$; m/z: 640.32892 ([M+H]$^+$).

$^1$H NMR (600 MHz, $D_2O$) $\delta$ 4.34 (dd, $J$ = 8.3, 6.5 Hz, 1H), 4.25 - 4.15 (m, 3H), 4.08 (dd, $J$ = 8.8, 5.9 Hz, 1H), 4.04 - 4.00 (m, 1H), 3.73 - 3.65 (m, 1H), 3.57 - 3.41 (m, 3H), 2.35 (t, $J$ = 7.3 Hz, 2H), 2.24 - 2.20 (m, 2H), 2.16 - 2.07 (m, 2H), 2.04 - 1.75 (m, AcOH, 11H), 1.73 - 1.63 (m, 2H), 1.17 (d, $J$ = 7.2 Hz, 3H), 0.80 (dd, $J$ = 12.9, 6.8 Hz, 6H).

**Compound 44:** Pro-Val-Pro-Gln-Ala-Lys (acetate salt)

High resolution mass spectrometry (Orbitrap-HRMS), molecular formula: $C_{29}H_{50}N_8O_8$; m/z: 320.19421 ([M+2H]$^{2+}$).

$^1$H NMR (600 MHz, $D_2O$) $\delta$ 4.29 - 4.19 (m, 3H), 4.13 - 4.06 (m, 2H), 3.95 (dd, $J$ = 8.2, 5.1 Hz, 1H), 3.73 - 3.68 (m, 1H), 3.52 - 3.47 (m, 1H), 3.24 - 3.16 (m, 2H), 2.79 (t, $J$ = 7.5 Hz, 2H), 2.25 - 2.19 (m, 3H), 2.14 - 2.08 (m, 1H), 1.97 - 1.57 (m, AcOH, 17H), 1.53 - 1.45 (m, 3H), 1.21 - 1.17 (m, 5H), 0.82 (d, $J$= 6.8 Hz, 3H), 0.75 (d, $J$ = 6.7 Hz, 3H).

**Compound 45:** Glu-Pro-Val-Pro-Gln-Ala-Arg (acetate salt)

High resolution mass spectrometry (Orbitrap-HRMS), molecular formula: $C_{34}H_{57}N_{11}O_{11}$; m/z: 398.71835 ([M+2H]$^{2+}$), 796.42944 ([M+H]$^+$).

$^1$H NMR (600 MHz, $D_2O$) $\delta$ 4.38 (dd, $J$ = 8.3, 6.4 Hz, 1H), 4.27 - 4.19 (m, 3H), 4.19 - 4.10 (m, 2H), 4.03 (dd, $J$= 8.3, 4.9 Hz, 1H), 3.78 - 3.70 (m, 1H), 3.60 - 3.46 (m, 3H), 3.07 - 3.02 (m, 2H), 2.30 (t, $J$ = 7.2 Hz, 2H), 2.25 (t, $J$= 7.6 Hz, 2H), 2.19 - 2.11 (m, 2H), 2.06 - 1.78 (m, AcOH, 12H), 1.76 - 1.67 (m, 3H), 1.60 - 1.53 (m, 1H), 1.48 - 1.42 (m, 2H), 1.24 (d, $J$ = 7.1 Hz, 3H), 0.85 (dd, $J$ = 12.8, 6.7 Hz, 6H).

**Compound 46:** Ala-Pro-Val-Pro-Gln-Ala-Lys (acetate salt)

High resolution mass spectrometry (Orbitrap-HRMS), molecular formula: $C_{32}H_{55}N_9O_9$; m/z: 355.71259 ([M+2H]$^{2+}$), 710.41809 ([M+H]$^+$).

$^1$H NMR (600 MHz, DMSO-$d_6$) $\delta$ 8.23 (d, $J$ = 7.7 Hz, 1H), 8.08 (d, $J$ = 8.1 Hz, 1H), 7.87 (d, $J$ = 8.4 Hz, 1H), 7.68 (s, 1H), 7.38 (d, $J$ = 6.4 Hz, 1H), 6.75 (s, 1H), 4.40 - 4.37 (m, 1H), 4.33 - 4.25 (m, 3H), 4.15 (t, $J$ = 7.2 Hz, 3H), 3.78 (d, $J$= 6.4 Hz, 1H), 3.66 (dd, $J$ = 32.4, 8.0 Hz, 2H), 3.58 - 3.51 (m, 2H), 3.43 (d, $J$ = 7.0 Hz, 1H), 3.36 - 3.27 (m, 1H), 2.67 (t, $J$ = 7.7 Hz, 3H), 2.19 - 2.07 (m, 4H), 2.01 - 1.69 (m, AcOH, 24H), 1.63 - 1.60 (m, 1H), 1.55 - 1.41 (m, 5H), 1.26 (d, 1H), 1.18 (d, $J$ = 7.2 Hz, 6H), 1.11 (d, $J$ = 6.7 Hz, 3H), 1.04 (d, $J$ = 6.6 Hz, 1H), 0.87 (d, $J$ = 6.7 Hz, 3H), 0.83 (d, $J$ = 6.9 Hz, 3H).

**Compound 47:** Glu-Pro-Val-Pro-Ala-Ala-Lys (acetate salt)

High resolution mass spectrometry (Orbitrap-HRMS), molecular formula: $C_{32}H_{54}N_8O_{10}$; m/z: 356.20514 ([M+2H]$^{2+}$), 711.40198 ([M+H]$^+$).

$^1$H NMR (600 MHz, D$_2$O) $\delta$ 4.45 (dd, $J$ = 8.4, 6.4 Hz, 1H), 4.33 - 4.27 (m, 3H), 4.21 (dd, $J$= 15.1, 7.2 Hz, 2H), 4.07 (dd, $J$ = 8.1, 5.1 Hz, 1H), 3.86 - 3.75 (m, 1H), 3.67 - 3.54 (m, 3H), 2.91 (t, $J$ = 7.5 Hz, 2H), 2.37 (t, $J$ = 6.9 Hz, 2H), 2.26 - 2.18 (m, 2H), 2.14 - 1.69 (m, AcOH, 13H), 1.65 - 1.58 (m, 3H), 1.35 - 1.29 (m, 9H), 0.92 (dd, $J$ = 13.6, 6.8 Hz, 6H).

**Compound 48:** Glu-Pro-Val-Pro-Gln-Ala-Ala (acetate salt)

High resolution mass spectrometry (Orbitrap-HRMS), molecular formula: $C_{31}H_{50}N_8O_{11}$; m/z: 356.18692 ([M+2H]$^{2+}$), 711.36597 ([M+H]$^+$).

$^1$H NMR (600 MHz, D$_2$O) $\delta$ 4.34 (dd, $J$ = 8.3, 6.5 Hz, 1H), 4.24 - 4.16 (m, 3H), 4.12 - 4.06 (m, 2H), 4.03 - 3.99 (m, 1H), 3.73 - 3.65 (m, 1H), 3.56 - 3.41 (m, 3H), 2.35 (t, $J$ = 7.3 Hz, 2H), 2.22 - 2.18 (m, 2H), 2.15 - 2.07 (m, 2H), 2.04 - 1.73 (m, AcOH, 11H), 1.71 - 1.63 (m, 2H), 1.18 (dd, $J$ = 11.2, 7.2 Hz, 6H), 0.80 (dd, $J$ = 13.0, 6.8 Hz, 6H).

**Compound 49:** Asp-Pro-Val-Pro-Gln-Ala-Lys (acetate salt)

High resolution mass spectrometry (Orbitrap-HRMS), molecular formula: $C_{33}H_{55}N_9O_{11}$; m/z: 377.70767 ([M+2H]$^{2+}$), 754.40814 ([M+H]$^+$).

$^1$H NMR (600 MHz, D$_2$O) $\delta$ 4.36 - 4.29 (m, 2H), 4.23 - 4.14 (m, 2H), 4.13 - 4.06 (m, 2H), 3.97 (dd, $J$ = 8.3, 5.0 Hz, 1H), 3.74 - 3.65 (m, 1H), 3.56 - 3.46 (m, 2H), 3.44 - 3.39 (m, 1H), 2.79 (t, $J$ = 7.5 Hz, 2H), 2.65 (dd, $J$ = 17.5, 3.3 Hz, 1H), 2.38 (dd, $J$ = 17.5, 10.7 Hz, 1H), 2.21 (t, $J$ = 7.6 Hz, 2H), 2.15 - 2.06 (m, 2H), 1.93 - 1.75 (m, AcOH, 9H), 1.72 - 1.61 (m, 3H), 1.54 - 1.45 (m, 3H), 1.21 (dd, $J$= 13.1, 7.5 Hz, 5H), 0.80 (dd, $J$= 11.0, 6.7 Hz, 6H).

**Compound 50:** Asn-Pro-Val-Pro-Gln-Ala-Lys (acetate salt)

High resolution mass spectrometry (Orbitrap-HRMS), molecular formula: $C_{33}H_{56}N_{10}W_{10}$; m/z: 377.21542 ([M+2H]$^{2+}$), 753.42365 ([M+H]$^+$).

$^1$H NMR (600 MHz, D$_2$O) $\delta$ 4.55 (dd, $J$ = 9.3, 4.0 Hz, 1H), 4.47 (dd, $J$ = 8.4, 6.1 Hz, 1H), 4.34 - 4.29 (m, 2H), 4.26 - 4.19 (m, 2H), 4.07 (dd, $J$= 8.1, 5.1 Hz, 1H), 3.83 - 3.77 (m, 1H), 3.69 - 3.53 (m, 3H), 2.94 - 2.90 (m, 3H), 2.72 (dd, $J$ = 17.0, 9.3 Hz, 1H), 2.33 (t, $J$ = 7.6 Hz, 2H), 2.27 - 2.19 (m, 2H), 2.05 - 1.71 (m, AcOH, 15H), 1.65 - 1.57 (m, 3H), 1.34 - 1.30 (m, 5H), 0.91 (dd, $J$ = 13.4, 6.7 Hz, 6H).

**Compound 51:** Glu-Pro-Val-Pro-Gln-Ala-Lys-Pro-Arg-Lys-Val-Ala (acetate salt)

High resolution mass spectrometry (Orbitrap-HRMS), molecular formula: $C_{59}H_{102}N_{18}W_{16}$; m/z: 440.59735 ([M+3H]$^{3+}$), 660.39252 ([M+2H]$^{2+}$).

$^1$H NMR (600 MHz, D$_2$O) $\delta$ 4.38 (dd, $J$ = 9.3, 4.8 Hz, 1H), 4.33 (dd, $J$ = 8.3, 6.5 Hz, 1H), 4.22 - 4.12 (m, 5H), 4.10 - 4.04 (m, 3H), 3.93 - 3.88 (m, 2H), 3.75 - 3.67 (m, 1H), 3.66 - 3.58 (m, 1H), 3.56 - 3.41 (m, 4H), 3.00 (t, $J$ = 6.9 Hz, 2H), 2.82 - 2.76 (m, 4H), 2.24 - 2.19 (m, 4H), 2.14 - 2.06 (m, 3H), 1.97 - 1.41 (m, AcOH, 37H), 1.33 - 1.12 (m, 10H), 0.80 (dd, $J$ = 9.7, 6.7 Hz, 6H), 0.74 (dd, $J$ = 11.0, 6.8 Hz, 6H).

**Compound 52:** Glu-Pro-Ala-Pro-Gln-Ala-Lys (acetate salt)

High resolution mass spectrometry (Orbitrap-HRMS), molecular formula: $C_{32}H_{53}N_9O_{11}$; m/z: 370.6998 ([M+2H]$^{2+}$), 740.39221 ([M+H]$^+$).

$^1$H NMR (600 MHz, D$_2$O) $\delta$ 4.43 (q, $J$ = 7.1 Hz, 1H), 4.33 (dd, $J$ = 8.4, 6.5 Hz, 1H), 4.26 (dd, $J$ = 8.4, 5.8 Hz, 1H), 4.22 (dd, *J* = 7.6, 4.6 Hz, 1H), 4.18 - 4.11 (m, 2H), 4.01 (dd, *J* = 8.3, 5.0 Hz, 1H), 3.67 (dt, *J* = 10.0, 6.7 Hz, 1H), 3.61 - 3.54 (m, 1H), 3.53 - 3.44 (m, 2H), 2.84 (t, *J* = 7.5 Hz, 2H), 230 (t, *J* = 7.2 Hz, 2H), 2.25 (t, *J* = 7.6 Hz, 2H), 2.21 - 2.12 (m, 2H), 2.08 - 1.77 (m, AcOH, 11H), 1.77 - 1.63 (m, 3H), 1.60 - 1.49 (m, 3H), 1.24 (dd, *J* = 10.4, 7.1 Hz, 6H).

**Compound 53:** Glu-Ala-Val-Pro-Gln-Ala-Lys (acetate salt)

High resolution mass spectrometry (Orbitrap-HRMS), molecular formula: $C_{32}H_{55}N_9O_{11}$; m/z: 371.7081 ($[M+2H]^{2+}$), 742.4089($[M+H]^+$).

$^1$H NMR (600 MHz, $D_2O$) δ 4.23 - 4.18 (m, 3H), 4.13 - 4.06 (m, 2H), 3.95 (dd, *J* = 8.3, 50 Hz, 1H), 3.85 - 3.82 (m, 1H), 3.73 - 3.66 (m, 1H), 3.53 - 3.47 (m, 1H), 2.79 (t, *J* = 7.5 Hz, 2H), 2.22 - 2.17 (m, 4H), 2.13 - 2.07 (m, 1H), 1.99 - 1.70 (m, AcOH, 9H), 1.69 - 1.59 (m, 2H), 1.53 - 1.45 (m, 3H), 1.26 - 1.12 (m, 9H), 0.79 (d, *J* = 6.7 Hz, 3H), 0.76 (d, *J*= 6.7 Hz, 3H).

**Compound 54:** Glu-Pro-Val-Ala-Gln-Ala-Lys (acetate salt)

High resolution mass spectrometry (Orbitrap-HRMS), molecular formula: $C_{32}H_{55}N_9O_{11}$; m/z: 371.70767($[M+2H]^{2+}$), 742.40802($[M+H]^+$).

$^1$H NMR (600 MHz, DMSO-$d_6$ + $D_2O$) δ 4.34 (dd, *J* = 8.3, 6.0 Hz, 1H), 4.15 - 4.05 (m, 4H), 3.97 - 3.87 (m, 2H), 3.51 (d, *J* = 9.8 Hz, 1H), 3.46 - 3.38 (m, 1H), 2.74 (t, *J* = 7.5 Hz, 2H), 2.23 (t, *J* = 7.1 Hz, 2H), 2.17 - 2.06 (m, 3H), 1.95 - 1.67 (m, AcOH, 10H), 1.65 - 1.59 (m, 1H), 1.53 - 1.42 (m, 3H), 1.22 - 1.13 (m, 8H), 0.78 (d, *J* = 6.8 Hz, 6H).

**Compound 55:** Glu-Pro-Val-Pro-Glu-Ala-Lys (acetate salt)

High resolution mass spectrometry (Orbitrap-HRMS), molecular formula: $C_{34}H_{56}N_8O_{12}$; m/z: 385.2083 ($[M+2H]^{2+}$), 769.4092 ($[M+H]^+$).

$^1$H NMR (600 MHz, $D_2O$) δ 4.33 (dd, *J*= 8.3, 6.5 Hz, 1H), 4.21 - 4.16 (m, 3H), 4.11 (t, *J* = 7.1 Hz, 1H), 4.07 (dd, *J* = 8.8, 5.7 Hz, 1H), 3.98 (dd, *J* = 8.5, 4.8 Hz, 1H), 3.71 - 3.66 (m, 1H), 3.55 - 3.41 (m, 3H), 2.78 (t, *J* = 7.5 Hz, 2H), 2.36 - 2.14 (m, 5H), 2.13 - 2.05 (m, 2H), 1.99 - 1.74 (m, AcOH, 11H), 1.70 - 1.61 (m, 3H), 1.53 - 1.45 (m, 3H), 1.20 (dd, *J* = 15.4, 7.5 Hz, 5H), 0.80 (dd, *J* = 12.5, 6.7 Hz, 6H).

**Compound 56:** Glu-Pro-Val-Pro-Gln-Ala-Lys-Pro-Arg-Lys-Val (acetate salt)

High resolution mass spectrometry (Orbitrap-HRMS), molecular formula: $C_{56}H_{97}N_{17}O_{15}$; m/z: 416.91827 ($[M+3H]^{3+}$), 624.87354 ($[M+2H]^{2+}$).

$^1$H NMR (600 MHz, $D_2O$) δ 4.43 (dd, *J* = 9.3, 4.9 Hz, 1H), 4.38 (dd, *J* = 8.4, 6.4 Hz, 1H), 4.27 - 4.19 (m, 5H), 4.16 - 4.10 (m, 3H), 3.88 (d, *J* = 6.2 Hz, 1H), 3.74 (dt, *J* = 10.1, 6.9 Hz, 1H), 3.71 - 3.63 (m, 1H), 3.60 - 3.46 (m, 4H), 3.07 - 3.02 (m, 2H), 2.87 - 2.82 (m, 4H), 2.29 - 2.24 (m, 4H), 2.19 - 2.11 (m, 3H), 2.04 - 1.43 (m, AcOH, 37H), 1.37 - 1.20 (m, 7H), 0.85 (dd, *J*= 10.1, 6.8 Hz, 6H), 0.74 (dd, *J*= 11.8, 6.9 Hz, 6H).

**Compound 57:** Glu-Pro-Val-Pro-Gln-Ala-Lys-Pro-Arg (acetate salt)

High resolution mass spectrometry (Orbitrap-HRMS), molecular formula: $C_{45}H_{76}N_{14}O_{13}$; m/z: 511.29254 ($[M+2H]^{2+}$).

$^1$H NMR (600 MHz, $D_2O$) δ 4.39 (dd, *J* = 8.6, 5.5 Hz, 1H), 4.32 (dd, *J*= 8.3, 6.5 Hz, 1H), 4.22 - 4.13 (m, 4H), 4.10 - 4.03 (m, 2H), 3.93 (dd, *J* = 7.9, 5.2 Hz, 1H), 3.72 - 3.60 (m, 2H), 3.54 - 3.40 (m, 4H), 3.00 (t, *J*= 6.9 Hz, 2H), 2.80 (t, *J*= 7.5 Hz, 2H), 2.24 - 2.18 (m, 4H), 2.14 - 2.06 (m, 3H), 1.98 - 1.70 (m, AcOH, 19H), 1.69 - 1.60 (m, 4H), 1.55 - 1.47 (m, 4H), 1.45 - 1.39 (m, 2H), 1.34 - 1.24 (m, 2H), 1.16 (d, *J* = 7.1 Hz, 3H), 0.80 (dd, *J* = 10.4, 6.7 Hz, 6H).

**Compound 58:** Glu-Pro-Val-Pro-Gln-Ala-Lys-Pro (acetate salt)

High resolution mass spectrometry (Orbitrap-HRMS), molecular formula: $C_{39}H_{64}N_{10}O_{12}$; m/z: 433.24188 ($[M+2H]^{2+}$), 865.47662 ($[M+H]^+$).

$^1$H NMR (600 MHz, $D_2O$) δ 4.41 (dd, *J* = 8.5, 5.5 Hz, 1H), 4.33 (dd, *J* = 8.3, 6.5 Hz, 1H), 4.22 - 4.14 (m, 3H), 4.11 - 4.02 (m, 3H), 3.75 - 3.67 (m, 1H), 3.59 - 3.41 (m, 5H), 2.81 (t, *J* = 7.4 Hz, 2H), 2.25 - 2.19 (m, 4H), 2.15 - 1.61 (m, AcOH, 26H), 1.57 - 1.48 (m, 3H), 1.37 - 1.23 (m, 2H), 1.16 (d, *J* = 7.0 Hz, 3H), 0.80 (dd, *J* = 11.5, 6.7 Hz, 6H).

**Compound 59:** Pro-Val-Pro-Gln-Ala-Lys-Pro-Arg (acetate salt)

High resolution mass spectrometry (Orbitrap-HRMS), molecular formula: $C_{40}H_{69}N_{13}O_{10}$; m/z: 298.18314

([M+3H]$^{3+}$), 446.77118 ([M+2H]$^{2+}$), 892.53497 ([M+H]$^+$).

$^1$H NMR (600 MHz, D$_2$O) δ 4.40 (dd, $J$ = 8.6, 5.5 Hz, 1H), 4.30 - 4.19 (m, 4H), 4.11 - 4.05 (m, 2H), 3.93 (dd, $J$ = 7.9, 5.2 Hz, 1H), 3.73 - 3.63 (m, 2H), 3.52 - 3.45 (m, 2H), 3.26 - 3.17 (m, 2H), 3.01 (t, $J$ = 6.9 Hz, 2H), 2.81 (t, $J$ = 7.5 Hz, 2H), 2.26 - 2.19 (m, 3H), 2.14 - 2.09 (m, 2H), 1.94 - 1.62 (m, AcOH, 24H), 1.57 - 1.40 (m, 7H), 1.34 - 1.26 (m, 2H), 1.16 (d, $J$ = 7.3 Hz, 3H), 0.82 (d, $J$ = 6.8 Hz, 3H), 0.76 (d, $J$ = 6.7 Hz, 3H).

**Compound 60:** Pro-Val-Pro-Gln-Ala-Lys-Pro (acetate salt)

High resolution mass spectrometry (Orbitrap-HRMS), molecular formula: C$_{34}$H$_{57}$N$_9$O$_9$; m/z: 368.7207 ([M+2H]$^{2+}$), 736.4343 ([M+H]$^+$).

$^1$H NMR (600 MHz, D$_2$O) δ 4.46 (dd, $J$ = 8.4, 5.6 Hz, 1H), 4.34 (d, $J$ = 7.4 Hz, 1H), 4.29 (dd, $J$ = 8.5, 6.2 Hz, 1H), 4.27 - 4.24 (m, 1H), 4.16 - 4.07 (m, 3H), 3.78 - 3.70 (m, 1H), 3.66 - 3.60 (m, 1H), 3.58 - 3.45 (m, 2H), 3.30 - 3.22 (m, 2H), 2.86 (t, $J$ = 7.5 Hz, 2H), 2.32 - 2.24 (m, 3H), 2.19 - 2.13 (m, 1H), 2.12 - 2.03 (m, 1H), 2.02 - 1.69 (m, AcOH, 19H), 1.61 - 1.52 (m, 3H), 1.42 - 1.27 (m, 2H), 1.23 - 1.20 (m, 3H), 0.87 (d, $J$ = 6.8 Hz, 3H), 0.81 (d, $J$ = 6.7 Hz, 3H).

**Compound 61:** D-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: C$_{73}$H$_{127}$N$_{23}$O$_{20}$; m/z: 412.4978([M+4H]$^{4+}$), 549.6611([M+3H]$^{3+}$), 823.9882([M+2H]$^{2+}$).

$^1$H NMR (600 MHz, D$_2$O) δ 4.46 - 4.35 (m, 2H), 4.27 (dd, $J$ = 8.3, 6.1 Hz, 1H), 4.22 - 4.04 (m, 9H), 3.96 (dd, $J$ = 8.4, 4.8 Hz, 1H), 3.90 (d, $J$ = 7.7 Hz, 1H), 3.84 (t, $J$ = 6.6 Hz, 1H), 3.74 - 3.59 (m, 3H), 3.56 - 3.38 (m, 3H), 3.01 (t, $J$ = 6.9 Hz, 2H), 2.83 - 2.76 (m, 6H), 2.23 - 2.06 (m, 9H), 1.96 - 1.39 (m, AcOH, 48H), 1.18 (dd, $J$ = 22.0, 7.2 Hz, 15H), 0.83 - 0.67 (m, 12H).

**Compound 62:** Lys-*D*-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: C$_{73}$H$_{127}$N$_{23}$O$_{20}$; m/z: 412.4976([M+4H]$^{4+}$), 549.6608([M+3H]$^{3+}$).

$^1$H NMR (600 MHz, D$_2$O) δ 4.50 (dd, $J$ = 9.8, 4.4 Hz, 1H), 4.37 (dd, $J$ = 9.4, 4.9 Hz, 1H), 4.25 - 4.03 (m, 10H), 3.95 (dd, $J$ = 8.5, 4.8 Hz, 1H), 3.89 (d, $J$ = 7.7 Hz, 1H), 3.83 (t, $J$ = 6.6 Hz, 1H), 3.74 - 3.68 (m, 1H), 3.64 - 3.55 (m, 2H), 3.52 - 3.38 (m, 2H), 3.00 (t, $J$ = 6.9 Hz, 2H), 2.83 - 2.75 (m, 6H), 2.20 (t, $J$ = 7.6 Hz, 2H), 2.12 - 2.06 (m, 6H), 1.92 - 1.41 (m, AcOH, 47H), 1.32 - 1.13 (m, 15H), 0.78 (dd, $J$ = 9.7, 6.7 Hz, 6H), 0.73 (dd, $J$ = 6.8, 4.1 Hz, 6H).

**Compound 63:** Lys-Glu-D-Pro-Val-Pro-Gln-Ala-Lys-Pro-Arg-Lys-Val-Ala-Ala-Gln (acetate salt)

High resolution mass spectrometry (Orbitrap-HRMS), molecular formula: C$_{73}$H$_{127}$N$_{23}$O$_{20}$; m/z: 412.4976([M+4H]$^{4+}$), 549.6609([M+3H]$^{3+}$), 823.9878([M+2H]$^{2+}$).

$^1$H NMR (600 MHz, D$_2$O) δ 4.45 (dd, $J$ = 9.4, 4.4 Hz, 1H), 4.37 (dd, $J$ = 9.4, 4.9 Hz, 1H), 4.29 - 4.25 (m, 1H), 4.21 - 4.04 (m, 8H), 3.96 - 3.94 (m, 1H), 3.89 (d, $J$ = 7.7 Hz, 1H), 3.79 (d, $J$ = 6.9 Hz, 1H), 3.68 - 3.59 (m, 2H), 3.54 - 3.35 (m, 3H), 3.00 (t, $J$ = 6.9 Hz, 2H), 2.82 - 2.74 (m, 6H), 2.23 - 2.19 (m, 2H), 2.15 - 2.02 (m, 6H), 1.95 - 1.41 (m, AcOH, 48H), 1.17 (dd, $J$ = 21.6, 7.2 Hz, 15H), 0.83 - 0.64 (m, 12H).

**Compound 64:** Lys-Glu-Pro-D-Val-Pro-Gln-Ala-Lys-Pro-Arg-Lys-Val-Ala-Ala-Gln (acetate salt)

High resolution mass spectrometry (Orbitrap-HRMS), molecular formula: C$_{73}$H$_{127}$N$_{23}$O$_{20}$; m/z: 412.4978([M+4H]$^{4+}$), 549.6609([M+3H]$^{3+}$), 823.9878([M+2H]$^{2+}$).

$^1$H NMR (600 MHz, D$_2$O) δ 4.46 (dd, $J$ = 10.2, 3.9 Hz, 1H), 4.41 - 4.31 (m, 2H), 4.27 - 4.18 (m, 3H), 4.15 - 4.02 (m, 7H), 3.95 - 3.92 (m, 1H), 3.89 (d, $J$ = 7.7 Hz, 1H), 3.83 (t, $J$ = 6.6 Hz, 1H), 3.67 - 3.50 (m, 5H), 3.47 - 3.25 (m, 2H), 3.00 (t, $J$ = 6.9 Hz, 2H), 2.81 - 2.76 (m, 6H), 2.23 - 2.06 (m, 10H), 1.94 - 1.41 (m, AcOH, 50H), 1.17 (dd, $J$ = 19.9, 7.1 Hz, 17H), 0.76 - 0.68 (m, 12H).

**Compound 65:** Lys-Glu-Pro-Val-D-Pro-Gln-Ala-Lys-Pro-Arg-Lys-Val-Ala-Ala-Gln (acetate salt)

High resolution mass spectrometry (Orbitrap-HRMS), molecular formula: C$_{73}$H$_{127}$N$_{23}$O$_{20}$; m/z: 412.4979([M+4H]$^{4+}$), 549.6612([M+3H]$^{3+}$), 823.9883([M+2H]$^{2+}$).

$^1$H NMR (600 MHz, D$_2$O) δ 4.42 (dd, $J$ = 9.9, 4.2 Hz, 1H), 4.34 (dd, $J$ = 9.2, 5.2 Hz, 1H), 4.31 - 4.23 (m, 2H), 4.22 - 4.18 (m, 2H), 4.15 - 4.05 (m, 6H), 3.94 (dd, $J$ = 8.4, 4.8 Hz, 1H), 3.89 (d, $J$ = 7.7 Hz, 1H), 3.82 (t, $J$ = 6.7 Hz, 1H), 3.65 (d, $J$ = 6.8 Hz, 3H), 3.57 - 3.36 (m, 3H), 3.00 (t, $J$ = 6.9 Hz, 2H), 2.82 - 2.75 (m, 6H), 2.18 - 2.03 (m, 9H), 1.95 - 1.40 (m, AcOH, 46H), 1.37 - 1.08 (m, 16H), 0.80 - 0.68 (m, 12H).

**Compound 66:** Lys-Glu-Pro-Val-Pro-D-Gln-Ala-Lys-Pro-Arg-Lys-Val-Ala-Ala-Gln (acetate salt)

High resolution mass spectrometry (Orbitrap-HRMS), molecular formula: $C_{73}H_{127}N_{23}O_{20}$; m/z: 412.4978([M+4H]$^{4+}$), 549.6611([M+3H]$^{3+}$), 823.9881([M+2H]$^{2+}$).

$^1$H NMR (600 MHz, D$_2$O) δ 4.44 - 4.35 (m, 2H), 4.26 - 4.04 (m, 10H), 3.94 (dd, $J$ = 8.5, 4.8 Hz, 1H), 3.89 (d, $J$ = 7.7 Hz, 1H), 3.82 (t, $J$ = 6.8 Hz, 1H), 3.76 - 3.68 (m, 1H), 3.64 (d, $J$ = 8.7 Hz, 2H), 3.55 - 3.37 (m, 3H), 3.00 (t, $J$ = 6.9 Hz, 2H), 2.83 - 2.75 (m, 6H), 2.19 - 2.05 (m, 9H), 2.02 - 1.38 (m, AcOH, 47H), 1.34 - 1.15 (m, 15H), 0.78 (dd, $J$ = 11.2, 6.7 Hz, 6H), 0.73 (dd, $J$ = 6.7, 3.9 Hz, 6H).

**Compound 67:** Lys-Glu-Pro-Val-Pro-Gln-**D**-Ala-Lys-Pro-Arg-Lys-Val-Ala-Ala-Gln (acetate salt)

High resolution mass spectrometry (Orbitrap-HRMS), molecular formula: $C_{73}H_{127}N_{23}O_{20}$; m/z: 412.4961([M+4H]$^{4+}$), 549.6615([M+3H]$^{3+}$), 823.9886([M+2H]$^{2+}$).

$^1$H NMR (600 MHz, D$_2$O) δ 4.47 - 4.38 (m, 2H), 4.26 (dd, $J$ = 8.4, 5.8 Hz, 1H), 4.22 - 4.04 (m, 9H), 3.95 (dd, $J$ = 8.5, 4.8 Hz, 1H), 3.89 (d, $J$ = 7.7 Hz, 1H), 3.82 (t, $J$ = 6.8 Hz, 1H), 3.74 - 3.56 (m, 3H), 3.54 - 3.35 (m, 3H), 3.00 (t, $J$ = 6.9 Hz, 2H), 2.86 - 2.74 (m, 6H), 2.23 - 2.05 (m, 9H), 1.94 - 1.38 (m, AcOH, 46H), 1.29 - 1.14 (m, 15H), 0.78 (dd, $J$ = 10.5, 6.7 Hz, 6H), 0.73 (dd, $J$ = 6.8, 3.9 Hz, 6H).

**Compound 68:** Lys-Glu-Pro-Val-Pro-Gln-Ala-**D**-Lys-Pro-Arg-Lys-Val-Ala-Ala-Gln (acetate salt)

High resolution mass spectrometry (Orbitrap-HRMS), molecular formula: $C_{73}H_{127}N_{23}O_{20}$; m/z: 412.4977([M+4H]$^{4+}$), 549.6615([M+3H]$^{3+}$), 823.9879([M+2H]$^{2+}$).

$^1$H NMR (600 MHz, D$_2$O) δ 4.47 - 4.40 (m, 2H), 4.26 (dd, $J$ = 8.4, 5.9 Hz, 1H), 4.23 - 4.15 (m, 3H), 4.14 - 4.01 (m, 6H), 3.95 (dd, $J$ = 8.5, 4.8 Hz, 1H), 3.89 (d, $J$ = 7.7 Hz, 1H), 3.82 (t, $J$ = 6.8 Hz, 1H), 3.71 - 3.44 (m, 5H), 3.01 (t, $J$ = 6.9 Hz, 2H), 2.82 - 2.73 (m, 6H), 2.23 - 2.05 (m, 9H), 1.93 - 1.40 (m, AcOH, 46H), 1.30 - 1.14 (m, 15H), 0.79 (dd, $J$ = 12.5, 6.7 Hz, 6H), 0.73 (dd, $J$ = 6.8, 4.6 Hz, 6H).

**Compound 69:** Lys-Glu-Pro-Val-Pro-Gln-Ala-Lys-**D**-Pro-Arg-Lys-Val-Ala-Ala-Gln (acetate salt)

High resolution mass spectrometry (Orbitrap-HRMS), molecular formula: $C_{73}H_{127}N_{23}O_{20}$; m/z: 412.4976([M+4H]$^{4+}$), 549.6608([M+3H]$^{3+}$).

$^1$H NMR (600 MHz, D$_2$O) δ 4.42 (dt, $J$ = 8.2, 4.3 Hz, 2H), 4.26 (dd, $J$ = 8.4, 5.9 Hz, 1H), 4.23 - 4.14 (m, 3H), 4.14 - 4.05 (m, 6H), 3.94 (dd, $J$ = 8.5, 4.8 Hz, 1H), 3.89 (d, $J$ = 7.7 Hz, 1H), 3.81 (q, $J$ = 7.7, 7.2 Hz, 1H), 3.74 - 3.58 (m, 3H), 3.55 - 3.38 (m, 3H), 2.98 (t, $J$ = 7.0 Hz, 2H), 2.82 - 2.75 (m, 6H), 2.23 - 2.05 (m, 9H), 1.93 - 1.13 (m, AcOH, 62H), 0.79 (dd, $J$ = 13.2, 6.7 Hz, 6H), 0.73 (dd, $J$ = 6.8, 4.7 Hz, 6H).

**Compound 70:** Lys-Glu-Pro-Val-Pro-Gln-Ala-Lys-Pro-**D**-Arg-Lys-Val-Ala-Ala-Gln (acetate salt)

High resolution mass spectrometry (Orbitrap-HRMS), molecular formula: $C_{73}H_{127}N_{23}O_{20}$; m/z: 412.4978([M+4H]$^{4+}$), 549.6611([M+3H]$^{3+}$), 823.9883([M+2H]$^{2+}$).

$^1$H NMR (600 MHz, D$_2$O) δ 4.45 - 4.36 (m, 2H), 4.27 - 4.14 (m, 6H), 4.12 - 4.05 (m, 4H), 3.95 (dd, $J$ = 8.4, 4.8 Hz, 1H), 3.90 (d, $J$ = 7.7 Hz, 1H), 3.82 (t, $J$ = 6.8 Hz, 1H), 3.66 (dd, $J$ = 35.9, 8.3 Hz, 3H), 3.55 - 3.39 (m, 3H), 2.99 (t, $J$ = 7.0 Hz, 2H), 2.82 - 2.75 (m, 6H), 2.23 - 2.07 (m, 9H), 1.93 - 1.15 (m, AcOH, 63H), 0.79 (dd, $J$ = 13.9, 6.7 Hz, 6H), 0.74 (dd, $J$ = 6.8, 2.0 Hz, 6H).

**Compound 71:** Lys-Glu-Pro-Val-Pro-Gln-Ala-Lys-Pro-Arg-**D**-Lys-Val-Ala-Ala-Gln (acetate salt)

High resolution mass spectrometry (Orbitrap-HRMS), molecular formula: $C_{73}H_{127}N_{23}O_{20}$; m/z: 412.4976([M+4H]$^{4+}$), 549.6609([M+3H]$^{3+}$), 823.9878([M+2H]$^{2+}$).

$^1$H NMR (600 MHz, D$_2$O) δ 4.45 - 4.36 (m, 2H), 4.26 (dd, $J$ = 8.4, 5.8 Hz, 1H), 4.22 - 4.16 (m, 3H), 4.12 - 4.05 (m, 6H), 3.96 - 3.91 (m, 2H), 3.82 (t, $J$ = 6.8 Hz, 1H), 3.73 - 3.58 (m, 3H), 3.54 - 3.36 (m, 3H), 3.00 (t, $J$ = 7.0 Hz, 2H), 2.82 - 2.75 (m, 6H), 2.22 - 2.06 (m, 9H), 1.93 - 1.41 (m, AcOH, 45H), 1.32 - 1.14 (m, 15H), 0.79 (dd, $J$ = 13.8, 6.7 Hz, 6H), 0.74 (d, $J$ = 6.8 Hz, 3H), 0.70 (d, $J$ = 6.8 Hz, 3H).

**Compound 72:** Lys-Glu-Pro-Val-Pro-Gln-Ala-Lys-Pro-Arg-Lys-**D**-Val-Ala-Ala-Gln (acetate salt)

High resolution mass spectrometry (Orbitrap-HRMS), molecular formula: $C_{73}H_{127}N_{23}O_{20}$; m/z: 412.4976([M+4H]$^{4+}$), 549.6609([M+3H]$^{3+}$), 823.9876([M+2H]$^{2+}$).

$^1$H NMR (600 MHz, D$_2$O $\delta$ 4.45 - 4.35 (m, 2H), 4.26 (dd, $J$ = 8.4, 5.9 Hz, 1H), 4.21 - 4.05 (m, 9H), 3.94 - 3.88 (m, 2H), 3.82 (t, $J$ = 6.8 Hz, 1H), 3.73 - 3.57 (m, 3H), 3.55 - 3.37 (m, 3H), 2.99 (t, $J$ = 6.8 Hz, 2H), 2.82 - 2.75 (m, 6H), 2.23 - 2.06 (m, 9H), 1.96 - 1.36 (m, AcOH, 50H), 1.32 - 1.14 (m, 15H), 0.78 (dd, $J$ = 13.9, 6.7 Hz, 6H), 0.73 (d, $J$ = 6.7 Hz, 6H).

**Compound 73:** Lys-Glu-Pro-Val-Pro-Gln-Ala-Lys-Pro-Arg-Lys-Val-*D*-Ala-Ala-Gln (acetate salt)

High resolution mass spectrometry (Orbitrap-HRMS), molecular formula: $C_{73}H_{127}N_{23}O_{20}$; m/z: 412.4977([M+4H]$^{4+}$), 549.6609([M+3H]$^{3+}$), 823.9879([M+2H]$^{2+}$).

$^1$H NMR (600 MHz, D$_2$O) $\delta$ 4.48 (dd, $J$ = 9.7, 4.3 Hz, 1H), 4.43 (dd, $J$ = 9.3, 4.9 Hz, 1H), 4.32 (dd, $J$= 8.4, 5.8 Hz, 1H), 4.27 - 4.23 (m, 3H), 4.21 - 4.17 (m, 3H), 4.15 - 4.10 (m, 3H), 3.97 (dd, $J$ = 9.2, 4.6 Hz, 1H), 3.89 - 3.85 (m, 2H), 3.76 - 3.66 (m, 3H), 3.59 - 3.45 (m, 3H), 3.05 (t, $J$ = 6.9 Hz, 2H), 2.87 - 2.81 (m, 6H), 2.26 (t, $J$ = 7.7 Hz, 2H), 2.22 - 2.12 (m, 7H), 2.00 - 1.48 (m, AcOH, 47H), 1.37 - 1.20 (m, 16H), 0.86 - 0.77 (m, 12H).

**Compound 74:** Lys-Glu-Pro-Val-Pro-Gln-Ala-Lys-Pro-Arg-Lys-Val-Ala-*D*-Ala-Gln (acetate salt)

High resolution mass spectrometry (Orbitrap-HRMS), molecular formula: $C_{73}H_{127}N_{23}O_{20}$; m/z: 412.4977([M+4H]$^{4+}$), 549.6610([M+3H]$^{3+}$), 823.9879([M+2H]$^{2+}$).

$^1$H NMR (600 MHz, D$_2$O) $\delta$ 4.48 (dd, $J$ = 9.6, 4.3 Hz, 1H), 4.44 (dd, $J$ = 9.3, 4.9 Hz, 1H), 4.32 (dd, $J$ = 8.4, 5.8 Hz, 1H), 4.28 - 4.17 (m, 5H), 4.17 - 4.09 (m, 4H), 4.05 (dd, $J$ = 9.1, 4.7 Hz, 1H), 3.95 (d, $J$ = 7.7 Hz, 1H), 3.88 (t, $J$ = 6.8 Hz, 1H), 3.78 - 3.63 (m, 3H), 3.60 - 3.37 (m, 3H), 3.06 (t, $J$ = 6.9 Hz, 2H), 2.89 - 2.81 (m, 6H), 2.29 - 2.09 (m, 9H), 2.02 - 1.46 (m, AcOH, 45H), 1.39 - 1.19 (m, 15H), 0.84 (dd, $J$ = 14.0, 6.7 Hz, 6H), 0.78 (dd, $J$ = 14.3, 6.8 Hz, 6H).

**Compound 75:** Lys-Glu-Pro-Val-Pro-Gln-Ala-Lys-Pro-Arg-Lys-Val-Ala-Ala-*D*-Gln (acetate salt)

High resolution mass spectrometry (Orbitrap-HRMS), molecular formula: $C_{73}H_{127}N_{23}O_{20}$; m/z: 412.4978([M+4H]$^{4+}$), 549.6611([M+3H]$^{3+}$), 823.9881([M+2H]$^{2+}$).

$^1$H NMR (600 MHz, D$_2$O) $\delta$ 4.49 - 4.42 (m, 2H), 4.32 (dd, $J$ = 8.4, 5.8 Hz, 1H), 4.27 - 4.10 (m, 9H), 4.04 (dd, $J$ = 8.4, 4.6 Hz, 1H), 3.96 (d, $J$ = 7.4 Hz, 1H), 3.90 - 3.84 (m, 1H), 3.73 (d, $J$ = 7.7 Hz, 1H), 3.71 - 3.64 (m, 2H), 3.60 - 3.32 (m, 3H), 3.06 (t, $J$ = 6.9 Hz, 2H), 2.89 - 2.79 (m, 6H), 2.28 - 2.06 (m, 9H), 1.99 - 1.47 (m, AcOH, 44H), 1.39 - 1.18 (m, 15H), 0.84 (dd, $J$ = 13.9, 6.7 Hz, 6H), 0.78 (dd, $J$ = 6.8, 3.3 Hz, 6H).

**Compound 76:** Glu-Gly-Val-Pro-Gln-Ala-Lys (acetate salt)

High resolution mass spectrometry (Orbitrap-HRMS), molecular formula: $C_{31}H_{53}N_9O_{11}$; m/z: 364.7022 ([M+2H]$^{2+}$), 728.3929 ([M+H]$^+$).

$^1$H NMR (600 MHz, D$_2$O) $\delta$ 4.30 (d, $J$ = 7.8 Hz, 1H), 4.26 (dd, $J$ = 8.2, 6.7 Hz, 1H), 4.19 - 4.11 (m, 2H), 4.02 (dd, $J$ = 8.3, 5.0 Hz, 1H), 3.95 (t, $J$ = 6.5 Hz, 1H), 3.91 - 3.82 (m, 2H), 3.78 - 3.72 (m, 1H), 3.58 - 3.51 (m, 1H), 2.84 (t, $J$ = 7.5 Hz, 2H), 2.29 - 2.24 (m, 4H), 2.20 - 2.11 (m, 1H), 2.01 - 1.82 (m, AcOH, 10H), 1.77 - 1.65 (m, 2H), 1.60 - 1.50 (m, 3H), 1.25 (dd, $J$ = 7.5, 4.7 Hz, 5H), 0.84 (d, $J$ = 6.8 Hz, 3H), 0.80 (d, $J$ = 6.7 Hz, 3H).

**Compound 77:** Glu-Pro-Val-Gly-Gln-Ala-Lys (acetate salt)

High resolution mass spectrometry (Orbitrap-HRMS), molecular formula: $C_{31}H_{53}N_9O_{11}$; m/z: 364.7004([M+2H]$^{2+}$), 728.3932([M+H]$^+$).

$^1$H NMR (600 MHz, D$_2$O) $\delta$ 4.40 (dd, $J$ = 8.3, 6.5 Hz, 1H), 4.23 (dd, $J$ = 7.7, 4.4 Hz, 1H), 4.20 - 4.15 (m, 2H), 4.01 (dd, $J$ = 8.3, 5.1 Hz, 1H), 3.95 (d, $J$ = 7.4 Hz, 1H), 3.83 - 3.76 (m, 2H), 3.63 - 3.55 (m, 1H), 3.50 - 3.45 (m, 1H), 2.85 (t, $J$ = 7.5 Hz, 2H), 2.32 (t, $J$ = 7.2 Hz, 2H), 2.23 - 2.15 (m, 3H), 2.05 - 1.67 (m, AcOH, 12H), 1.59 - 1.50 (m, 3H), 1.26 (dd, $J$ = 9.7, 7.5 Hz, 5H), 0.84 (dd, $J$ = 14.3, 6.8 Hz, 6H).

**Compound 78:** *D*-Glu-Pro-Val-Pro-Gln-Ala-Lys (acetate salt)

High resolution mass spectrometry (Orbitrap-HRMS), molecular formula: $C_{34}H_{57}N_9O_{11}$; m/z: 384.716 ([M+2H]$^{2+}$), 768.4245 ([M+H]$^+$).

$^1$H NMR (600 MHz, D$_2$O) $\delta$ 4.36 (dd, $J$ = 8.8, 4.3 Hz, 1H), 4.29 - 4.21 (m, 3H), 4.18 - 4.11 (m, 2H), 4.01 (dd, $J$ = 8.2, 5.1 Hz, 1H), 3.78 - 3.72 (m, 1H), 3.63 - 3.49 (m, 3H), 2.84 (t, $J$ = 7.5 Hz, 2H), 2.28 - 2.23 (m, 4H), 2.19 - 2.12 (m, 2H), 1.98 - 1.66 (m, AcOH, 15H), 1.59 - 1.50 (m, 3H), 1.25 (dd, $J$ = 11.6, 7.4 Hz, 5H), 0.85 (dd, $J$ = 15.5, 6.8 Hz, 6H).

**Compound 79:** Glu-*D*-Pro-Val-Pro-Gln-Ala-Lys (acetate salt)

High resolution mass spectrometry (Orbitrap-HRMS), molecular formula: $C_{34}H_{57}N_9O_{11}$; m/z: 384.7159 ([M+2H]$^{2+}$), 768.4244 ([M+H]$^+$).

$^1$H NMR (600 MHz, D$_2$O) δ 4.36 (dd, $J$ = 8.8, 3.9 Hz, 1H), 4.33 (d, $J$ = 8.1 Hz, 1H), 4.28 (dd, $J$ = 8.3, 6.6 Hz, 1H), 4.23 (dd, $J$ = 7.1, 5.1 Hz, 1H), 4.19 - 4.13 (m, 2H), 4.02 (dd, $J$ = 8.2, 5.1 Hz, 1H), 3.76 - 3.68 (m, 1H), 3.61 - 3.51 (m, 3H), 2.84 (t, $J$ = 7.5 Hz, 2H), 2.31 - 2.06 (m, 7H), 2.00 - 1.66 (m, AcOH, 16H), 1.58 - 1.50 (m, 3H), 1.27 - 1.23 (m, 5H), 0.81 (dd, $J$ = 22.8, 6.7 Hz, 6H).

**Compound 80:** Glu-Pro-*D*-Val-Pro-Gln-Ala-Lys (acetate salt)

High resolution mass spectrometry (Orbitrap-HRMS), molecular formula: $C_{34}H_{57}N_9O_{11}$; m/z: 384.7158 ([M+2H]$^{2+}$), 768.4241 ([M+H]$^+$).

$^1$H NMR (600 MHz, D$_2$O) δ 4.46 (d, $J$ = 7.3 Hz, 1H), 4.36 (dd, $J$ = 8.3, 6.6 Hz, 1H), 4.30 - 4.20 (m, 3H), 4.17 (dd, $J$ = 8.1, 6.1 Hz, 1H), 4.03 - 4.00 (m, 1H), 3.72 - 3.66 (m, 1H), 3.63 - 3.57 (m, 2H), 3.53 - 3.49 (m, 1H), 2.85 (t, $J$ = 7.5 Hz, 2H), 2.36 - 2.21 (m, 6H), 2.18 - 2.13 (m, 1H), 2.05 - 1.78 (m, AcOH, 17H), 1.70 - 1.66 (m, 1H), 1.58 - 1.51 (m, 3H), 1.28 - 1.23 (m, 6H), 0.78 (dd, $J$ = 15.0, 6.7 Hz, 6H).

**Compound 81:** Glu-Pro-Val-*D*-Pro-Gln-Ala-Lys (acetate salt)

High resolution mass spectrometry (Orbitrap-HRMS), molecular formula: $C_{34}H_{57}N_9O_{11}$; m/z: 384.7157 ([M+2H]$^{2+}$), 768.4240 ([M+H]$^+$).

$^1$H NMR (600 MHz, D$_2$O) δ 4.43 - 4.30 (m, 2H), 4.28 - 4.21 (m, 2H), 4.21 - 4.13 (m, 2H), 4.03 - 3.98 (m, 1H), 3.72 - 3.53 (m, 3H), 3.50 - 3.44 (m, 1H), 2.85 (t, $J$ = 7.5 Hz, 2H), 2.33 (t, $J$ = 7.2 Hz, 2H), 2.23 - 2.11 (m, 4H), 2.07 - 1.74 (m, AcOH, 14H), 1.73 - 1.64 (m, 2H), 1.58 - 1.50 (m, 3H), 1.29 - 1.23 (m, 5H), 0.81 (dd, $J$ = 15.1, 6.7 Hz, 6H).

**Compound 82:** Glu-Pro-Val-Pro-*D*-Gln-Ala-Lys (acetate salt)

High resolution mass spectrometry (Orbitrap-HRMS), molecular formula: $C_{31}H_{53}N_9O_{11}$; m/z: 384.7158([M+2H]$^{2+}$), 768.4242([M+H]$^+$).

$^1$H NMR (600 MHz, D$_2$O) δ 4.38 (dd, $J$ = 8.3, 6.4 Hz, 1H), 4.27 - 4.14 (m, 5H), 4.03 (dd, $J$ = 8.4, 5.0 Hz, 1H), 3.80 - 3.75 (m, 1H), 3.62 - 3.45 (m, 3H), 2.85 (t, $J$ = 7.5 Hz, 2H), 2.32 (t, $J$ = 7.3 Hz, 2H), 2.24 - 2.12 (m, 4H), 2.09 - 1.67 (m, AcOH, 15H), 1.60 - 1.50 (m, 3H), 1.28 (d, $J$ = 7.3 Hz, 5H), 0.88 - 0.80 (m, 6H).

**Compound 83:** Glu-Pro-Val-Pro-Gln-*D*-Ala-Lys (acetate salt)

High resolution mass spectrometry (Orbitrap-HRMS), molecular formula: $C_{31}H_{53}N_9O_{11}$; m/z: 384.7159([M+2H]$^{2+}$), 768.4243([M+H]$^+$).

$^1$H NMR (600 MHz, D$_2$O) δ 4.38 (dd, $J$ = 8.3, 6.4 Hz, 1H), 4.28 - 4.20 (m, 3H), 4.19 - 4.12 (m, 2H), 4.05 (dd, $J$ = 8.6, 5.0 Hz, 1H), 3.77 - 3.72 (m, 1H), 3.60 - 3.45 (m, 3H), 2.85 - 2.80 (m, 2H), 2.32 (t, $J$ = 7.2 Hz, 2H), 2.26 (t, $J$ = 8.0 Hz, 2H), 2.20 - 2.12 (m, 2H), 2.08 - 1.77 (m, AcOH, 13H), 1.75 - 1.66 (m, 3H), 1.57 - 1.47 (m, 3H), 1.26 - 1.18 (m, 5H), 0.85 (dd, $J$ = 13.0, 6.7 Hz, 6H).

**Compound 84:** Glu-Pro-Val-Pro-Gln-Ala-*D*-Lys (acetate salt)

High resolution mass spectrometry (Orbitrap-HRMS), molecular formula: $C_{31}H_{53}N_9O_{11}$; m/z: 384.7160([M+2H]$^{2+}$), 768.4246([M+H]$^+$).

$^1$H NMR (600 MHz, D$_2$O) δ 4.39 (dd, $J$ = 8.3, 6.4 Hz, 1H), 4.30 - 4.20 (m, 3H), 4.19 - 4.11 (m, 2H), 4.05 (dd, $J$ = 8.3, 4.8 Hz, 1H), 3.79 - 3.71 (m, 1H), 3.61 - 3.47 (m, 3H), 2.86 - 2.81 (m, 2H), 2.32 (t, $J$ = 7.2 Hz, 2H), 2.27 (t, $J$ = 7.5 Hz, 2H), 2.21 - 2.12 (m, 2H), 2.05 - 1.78 (m, AcOH, 12H), 1.78 - 1.68 (m, 3H), 1.60 - 1.50 (m, 3H), 1.27 - 1.20 (m, 5H), 0.86 (dd, $J$ = 12.5, 6.7 Hz, 6H).

**Compound 85:** Lys-Glu-Pro-Val-Pro (acetate salt)

High resolution mass spectrometry (Orbitrap-HRMS), molecular formula: $C_{26}H_{44}N_6O_8$; m/z: 454.2655 ([M+2H]$^{2+}$), 569.3287 ([M+H]$^+$).

$^1$H NMR (600 MHz, D$_2$O) δ 4.50 (dd, $J$ = 9.9, 4.2 Hz, 1H), 4.36 - 4.22 (m, 3H), 4.09 (dd, $J$ = 8.5, 5.8 Hz, 1H), 3.89 (t, $J$ = 6.7 Hz, 1H), 3.70 - 3.65 (m, 2H), 3.60 - 3.53 (m, 2H), 3.51 - 3.26 (m, 1H), 2.87 (d, $J$ = 7.5 Hz, 2H), 2.28 - 2.09 (m, 5H), 2.00 - 1.68 (m, AcOH, 18H), 1.58 - 1.55 (m, 2H), 1.35 - 1.29 (m, 2H), 0.88 (d, $J$ = 6.8 Hz, 3H), 0.82 (d, $J$ = 6.7 Hz, 3H).

**Compound 86:** Glu-Pro-Val-Pro-Gln (acetate salt)

High resolution mass spectrometry (Orbitrap-HRMS), molecular formula: $C_{25}H_{40}N_6O_9$; m/z: 569.2919 ([M+H]$^+$).

$^1$H NMR (600 MHz, D$_2$O) δ 4.40 (dd, $J$ = 8.3, 6.4 Hz, 1H), 4.31 - 4.23 (m, 3H), 4.09 (dd, $J$ = 9.0, 4.8 Hz, 1H), 3.78 - 3.72 (m, 1H), 3.61 - 3.48 (m, 3H), 2.41 (t, $J$ = 7.3 Hz, 2H), 2.25 - 2.14 (m, 4H), 2.09 - 1.70 (m, AcOH, 13H), 0.86 (dd, $J$ = 15.3, 6.7 Hz, 6H).

**Compound 87:** Lys-Glu-Pro-Val-Pro-Gln (acetate salt)

High resolution mass spectrometry (Orbitrap-HRMS), molecular formula: $C_{31}H_{52}N_8O_{10}$; m/z: 349.1971 ([M+2H]$^{2+}$), 697.3868 ([M+H]$^+$).

$^1$H NMR (600 MHz, D$_2$O) δ 4.51 (dd, $J$ = 9.8, 4.3 Hz, 1H), 4.34 - 4.25 (m, 3H), 4.03 (dd, $J$ = 8.8, 4.7 Hz, 1H), 3.89 (t, $J$ = 6.7 Hz, 1H), 3.79 - 3.65 (m, 2H), 3.63 - 3.52 (m, 2H), 2.86 (t, $J$ = 7.5 Hz, 2H), 2.28 - 2.13 (m, 6H), 2.00 - 1.73 (m, AcOH, 17H), 1.60 - 1.54 (m, 2H), 1.36 - 1.28 (m, 2H), 0.86 (d, $J$ = 6.8 Hz, 3H), 0.83 (d, $J$ = 6.6 Hz, 3H).

**Compound 88:** Val-Pro-Tyr-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{92}H_{152}N_{26}O_{24}$; m/z: 502.2934 ([M+4H]$^{4+}$), 669.3699 ([M+3H]$^{3+}$).

$^1$H NMR (600 MHz, D$_2$O) δ 6.97 - 6.94 (m, 2H), 6.70 - 6.66 (m, 2H), 4.44 (dd, $J$ = 9.4, 4.8 Hz, 1H), 4.35 - 4.08 (m, 17H), 4.05 - 4.00 (m, 2H), 3.96 (d, $J$ = 7.6 Hz, 1H), 3.76 - 3.67 (m, 3H), 3.64 - 3.53 (m, 3H), 3.48 (dd, $J$ = 10.2, 6.3 Hz, 3H), 3.07 (t, $J$ = 6.9 Hz, 2H), 2.94 (dd, $J$ = 13.6, 6.6 Hz, 1H), 2.88 - 2.79 (m, 7H), 2.76 (dd, $J$ = 13.6, 9.2 Hz, 1H), 2.27 (t, $J$ = 7.6 Hz, 2H), 2.21 (t, $J$ = 7.6 Hz, 2H), 2.18 - 2.08 (m, 8H), 2.01 - 1.45 (m, AcOH, 59H), 1.30 - 1.10 (m, 16H), 0.95 (d, $J$ = 6.9 Hz, 3H), 0.87 - 0.77 (m, 18H).

**Compound 89:** *pyro*-Glu-Leu-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{84}H_{145}N_{25}O_{24}$; m/z: 468.7766 ([M+4H]$^{4+}$), 624.6998 ([M+3H]$^{3+}$), 936.5460 ([M+2H]$^{2+}$).

$^1$H NMR (600 MHz, D$_2$O) δ 4.47 - 4.42 (m, 2H), 4.32 - 4.10 (m, 13H), 4.02 (dd, $J$ = 8.4, 4.8 Hz, 1H), 3.96 (d, $J$ = 7.6 Hz, 1H), 3.82 - 3.60 (m, 3H), 3.58 - 3.45 (m, 3H), 3.07 (t, $J$ = 6.9 Hz, 2H), 2.90 - 2.80 (m, 6H), 2.45 - 2.36 (m, 1H), 2.31 - 2.24 (m, 4H), 2.23 - 2.03 (m, 7H), 1.99 - 1.44 (m, AcOH, 47H), 1.39 - 1.19 (m, 15H), 0.85 (dd, $J$ = 12.5, 6.8 Hz, 6H), 0.81 - 0.77 (m, 9H), 0.75 (d, $J$ = 6.0 Hz, 3H).

**Compound 90:** Pro-Ala-Tyr-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{90}H_{148}N_{26}O_{24}$; m/z: 495.2855 ([M+4H]$^{4+}$), 660.0449 ([M+3H]$^{3+}$), 989.5637 ([M+2H]$^{2+}$).

$^1$H NMR (600 MHz, D$_2$O) δ 6.99 - 6.92 (m, 2H), 6.71 - 6.64 (m, 2H), 4.47 - 4.42 (m, 1H), 4.36 (t, $J$ = 7.8 Hz, 1H), 4.32 - 4.06 (m, 15H), 4.04 - 3.99 (m, 1H), 3.96 (d, $J$ = 7.6 Hz, 1H), 3.79 - 3.65 (m, 3H), 3.60 - 3.46 (m, 3H), 3.30 - 3.22 (m, 2H), 3.07 (t, $J$ = 6.9 Hz, 2H), 2.89 - 2.79 (m, 8H), 2.33 - 2.06 (m, 11H), 1.99 - 1.45 (m, AcOH, 51H), 1.39 - 1.14 (m, 19H), 0.87 - 0.77 (m, 12H).

**Compound 91:** Arg-Lys-Asp-Val-Tyr-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{103}H_{174}N_{32}O_{28}$; m/z: 578.0856 ([M+4H]$^{4+}$), 770.4463 ([M+3H]$^{3+}$), 1155.1642 ([M+2H]$^{2+}$).

$^1$H NMR (600 MHz, D$_2$O) δ 6.96 (d, $J$ = 8.5 Hz, 2H), 6.70 - 6.64 (m, 2H), 4.50 - 4.43 (m, 2H), 4.38 (t, $J$ = 8.0 Hz, 1H), 4.30 - 4.11 (m, 13H), 4.02 (dd, $J$ = 8.4, 4.9 Hz, 1H), 3.96 (d, $J$ = 7.6 Hz, 1H), 3.94 - 3.90 (m, 2H), 3.79 - 3.65 (m, 3H), 3.62 - 3.46 (m, 3H), 3.11 - 3.05 (m, 4H), 2.88 - 2.79 (m, 10H), 2.51 (dd, $J$ = 15.9, 5.3 Hz, 1H), 2.42 (dd, $J$ = 16.0, 8.5 Hz, 1H), 2.29-2.11 (m, 9H), 1.99 - 1.64 (m, AcOH, 45H), 1.63 - 1.45 (m, 19H), 1.41 - 1.14 (m, 18H), 0.87 - 0.77 (m, 12H), 0.72 (d, $J$ = 6.7 Hz, 6H).

**Compound 92:** Arg-Lys-Asp-Val-Tyr-Lys-Glu-Pro-Val-Pro-Gln-Ala-Lys (acetate salt)

High resolution mass spectrometry (Orbitrap-HRMS), molecular formula: $C_{70}H_{116}N_{20}O_{20}$; m/z: 390.2233

([M+4H]$^{4+}$), 519.9619 ([M+3H]$^{3+}$), 779.4393 ([M+2H]$^{2+}$).

$^1$H NMR (600 MHz, D$_2$O) δ 6.96 (d, $J$ = 8.6 Hz, 2H), 6.67 (d, $J$ = 8.5 Hz, 2H), 4.49 (dd, $J$ = 8.4, 5.3 Hz, 1H), 4.38 (t, $J$ = 8.0 Hz, 1H), 4.31 - 4.23 (m, 5H), 4.19 - 4.10 (m, 3H), 4.01 (dd, $J$ = 8.2, 5.1 Hz, 1H), 3.93 - 3.89 (m, 2H), 3.77 - 3.67 (m, 2H), 3.61 - 3.50 (m, 2H), 3.10 - 3.05 (m, 2H), 2.86 - 2.79 (m, 8H), 2.51 (dd, $J$ = 16.0, 5.3 Hz, 1H), 2.42 (dd, $J$ = 15.9, 8.5 Hz, 1H), 2.28 - 2.20 (m, 4H), 2.18 - 2.10 (m, 2H), 1.97 - 1.65 (m, AcOH, 29H), 1.61 - 1.45 (m, 13H), 1.26 (dd, $J$ = 11.1, 7.4 Hz, 10H), 0.83 (dd, $J$ = 19.0, 6.7 Hz, 6H), 0.72 (d, $J$ = 5.6 Hz, 6H).

**Compound 93:** Arg-Lys-Asp-Val-Tyr-Glu-Pro-Val-Pro-Gln-Ala-Lys (acetate salt)

High resolution mass spectrometry (Orbitrap-HRMS), molecular formula: C$_{64}$H$_{104}$N$_{18}$O$_{19}$; m/z: 715.3917 ([M+2H]$^{2+}$).

$^1$H NMR (600 MHz, D$_2$O) δ 6.96 (d, $J$ = 8.6 Hz, 2H), 6.66 (d, $J$ = 8.5 Hz, 2H), 4.48 - 4.42 (m, 3H), 4.26 - 4.12 (m, 6H), 4.02 (dd, $J$ = 8.2, 50 Hz, 1H), 3.95 - 3.89 (m, 2H), 3.77 - 3.68 (m, 1H), 3.56 - 3.47 (m, 2H), 3.43 - 3.36 (m, 1H), 3.10 - 3.04 (m, 2H), 2.87 - 2.80 (m, 6H), 2.54 (dd, $J$ = 16.0, 5.1 Hz, 1H), 2.45 (dd, $J$ = 16.0, 8.4 Hz, 1H), 2.27 (t, $J$ = 7.6 Hz, 2H), 2.17 - 2.08 (m, 4H), 1.98 - 1.66 (m, AcOH, 27H), 1.65 - 1.45 (m, 9H), 1.26 (dd, $J$ = 11.3, 7.4 Hz, 7H), 0.84 (dd, $J$ = 13.0, 6.7 Hz, 6H), 0.70 (dd, $J$ = 6.8, 2.8 Hz, 6H).

**Compound 94:** Arg-Lys-Asp-Val-Tyr-Pro-Val-Pro-Gln (acetate salt)

High resolution mass spectrometry (Orbitrap-HRMS), molecular formula: C$_{50}$H$_{80}$N$_{14}$O$_{14}$; m/z: 367.8727 ([M+3H]$^{3+}$), 551.3051 ([M+2H]$^{2+}$), 1101.6030 ([M+H]$^+$).

$^1$H NMR (600 MHz, D$_2$O) δ 4.45 (dd, $J$ = 8.9, 4.8 Hz, 1H), 4.36 - 4.22 (m, 4H), 4.04 - 3.98 (m, 1H), 3.90 (dd, $J$ = 7.0, 5.8 Hz, 2H), 3.76 (dd, $J$ = 10.1, 6.4 Hz, 1H), 3.69 - 3.63 (m, 1H), 3.60 - 3.43 (m, 2H), 3.09 - 3.05 (m, 2H), 2.95 (dd, $J$ = 14.3, 5.2 Hz, 1H), 2.86 - 2.82 (m, 2H), 2.74 - 2.67 (m, 1H), 2.52 - 2.31 (m, 2H), 2.22 - 2.08 (m, 4H), 1.99 - 1.46 (m, AcOH, 29H), 1.32 - 1.23 (m, 2H), 0.90 - 0.81 (m, 6H), 0.75 (dd, $J$ = 10.3, 6.8 Hz, 2H), 0.67 (dd, $J$ = 17.1, 6.7 Hz, 4H).

**Compound 95:** Gly-Pro-Glu-Thr-Ala-Phe-Leu-Arg-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: C$_{113}$H$_{188}$N$_{34}$O$_{31}$; m/z: 504.6906 ([M+5H]$^{5+}$), 630.6115 ([M+4H]$^{4+}$), 840.4796 ([M+3H]$^{3+}$).

$^1$H NMR (600 MHz, D$_2$O) δ 7.22 (t, $J$ = 7.4 Hz, 2H), 7.16 (t, $J$ = 7.3 Hz, 1H), 7.12 - 7.07 (m, 2H), 4.47 - 4.41 (m, 3H), 4.35 - 4.03 (m, 19H), 4.02 (dd, $J$ = 8.4, 4.8 Hz, 1H), 3.95 (d, $J$ = 3.6 Hz, 1H), 3.86 (s, 1H), 3.75 (s, 1H), 3.68 (t, $J$ = 8.5 Hz, 2H), 3.58 - 3.42 (m, 5H), 3.06 (t, $J$ = 6.9 Hz, 4H), 2.91 - 2.81 (m, 7H), 2.29 - 2.11 (m, 12H), 2.00 - 1.19 (m, AcOH, 80H), 1.14 (d, $J$ = 7.1 Hz, 3H), 1.06 (d, $J$ = 6.3 Hz, 3H), 0.84 (dd, $J$ = 13.7, 6.7 Hz, 6H), 0.81 - 0.76 (m, 9H), 0.72 (d, $J$ = 5.6 Hz, 3H).

**Compound 96:** Gly-Pro-Glu-Thr-Ala-Phe-Leu-Arg-Glu-Pro-Val-Pro-Gln-Ala-Lys (acetate salt)

High resolution mass spectrometry (Orbitrap-HRMS), molecular formula: C$_{74}$H$_{118}$N$_{20}$O$_{22}$; m/z: 547.2972 ([M+3H]$^{3+}$), 820.4424 ([M+2H]$^{2+}$).

$^1$H NMR (600 MHz, D$_2$O) δ 7.21 (dd, $J$ = 8.2, 6.6 Hz, 2H), 7.16 (t, $J$ = 7.3 Hz, 1H), 7.12 - 7.07 (m, 2H), 4.50 - 4.42 (m, 2H), 4.36 - 4.22 (m, 4H), 4.21 - 4.09 (m, 7H), 4.07 - 4.00 (m, 2H), 3.93 (d, $J$ = 16.5 Hz, 1H), 3.84 (d, $J$ = 16.5 Hz, 1H), 3.78 - 3.70 (m, 1H), 3.68 - 3.60 (m, 1H), 3.56 - 3.42 (m, 4H), 3.10 - 2.97 (m, 3H), 2.90 - 2.82 (m, 3H), 2.29 - 2.09 (m, 9H), 2.00 - 1.81 (m, AcOH, 19H), 1.77 - 1.66 (m, 5H), 1.61 - 1.37 (m, 9H), 1.26 (dd, $J$ = 11.9, 7.4 Hz, 5H), 1.14 (d, $J$ = 7.2 Hz, 3H), 1.05 (d, $J$ = 6.4 Hz, 3H), 0.84 (dd, $J$ = 15.5, 6.7 Hz, 6H), 0.77 (d, $J$ = 5.9 Hz, 3H), 0.72 (d, $J$ = 5.7 Hz, 3H).

**Compound 97:** Ser-Ser-Glu-Asp-Ile-Lys-Glu-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: C$_{105}$H$_{179}$N$_{31}$O$_{35}$; m/z: 488.0708 ([M+5H]$^{5+}$), 609.8367 ([M+4H]$^{4+}$), 812.7799 ([M+3H]$^{3+}$).

$^1$H NMR (600 MHz, D$_2$O) δ 4.48 - 4.41 (m, 3H), 4.34 (t, $J$ = 5.4 Hz, 1H), 4.31 (dd, $J$ = 8.4, 5.8 Hz, 1H), 4.28 - 4.22 (m, 3H), 4.22 - 4.09 (m, 11H), 4.04 - 3.88 (m, 5H), 3.81 - 3.72 (m, 3H), 3.71 - 3.62 (m, 2H), 3.59 - 3.43 (m, 3H), 3.07 (t, $J$ = 6.9 Hz, 2H), 2.90 - 2.80 (m, 8H), 2.58 (dd, $J$ = 16.0, 6.3 Hz, 1H), 2.48 (dd, $J$ = 16.0, 7.9 Hz, 1H), 2.29 - 2.09 (m, 13H), 2.00 - 1.47 (m, AcOH, 51H), 1.40 - 1.20 (m, 18H), 1.12 - 1.05 (m, 1H), 0.89 - 0.70 (m, 18H).

**Compound 98:** Ser-Ser-Glu-Asp-Ile-Lys-Glu-Glu-Pro-Val-Pro-Gln-Ala-Lys (acetate salt)

High resolution mass spectrometry (Orbitrap-HRMS), molecular formula: $C_{66}H_{109}N_{17}O_{26}$; m/z: 519.5973 ([M+3H]$^{3+}$), 778.8925 ([M+2H]$^{2+}$).

$^1$H NMR (600 MHz, $D_2O$) δ 4.54 - 4.47 (m, 2H), 4.37 - 4.30 (m, 2H), 4.27 - 4.10 (m, 8H), 4.05 - 3.99 (m, 2H), 3.94 - 3.88 (m, 2H), 3.79 - 3.73 (m, 3H), 3.70 - 3.62 (m, 1H), 3.61 - 3.50 (m, 2H), 2.85 (t, $J$ = 7.6 Hz, 4H), 2.63 (dd, $J$ = 16.2, 6.2 Hz, 1H), 2.51 (dd, $J$ = 16.2, 7.9 Hz, 1H), 2.37 - 2.06 (m, 11H), 1.99 - 1.49 (m, AcOH, 30H), 1.34 - 1.24 (m, 8H), 1.10 - 1.01 (m, 1H), 0.84 (dd, $J$ = 13.8, 6.7 Hz, 6H), 0.77 - 0.71 (m, 6H).

**Compound 99:** Ser-Ser-Glu-Asp-Ile-Lys-Glu-Pro-Val-Pro-Gln (acetate salt)

High resolution mass spectrometry (Orbitrap-HRMS), molecular formula: $C_{52}H_{85}N_{13}O_{21}$; m/z: 614.806 ([M+2H]$^{2+}$), 1228.605 ([M+H]$^+$).

$^1$H NMR (600 MHz, $D_2O$) δ 4.54 - 4.48 (m, 2H), 4.36 (t, $J$ = 5.5 Hz, 1H), 4.34 - 4.24 (m, 3H), 4.23 - 4.18 (m, 2H), 4.12 - 4.05 (m, 2H), 4.01 (d, $J$ = 7.9 Hz, 1H), 3.94 - 3.88 (m, 2H), 3.79 - 3.72 (m, 3H), 3.70 - 3.62 (m, 1H), 3.60 - 3.50 (m, 2H), 2.85 (t, $J$ = 7.6 Hz, 2H), 2.65 (dd, $J$ = 16.3, 6.0 Hz, 1H), 2.53 (dd, $J$ = 16.4, 8.0 Hz, 1H), 2.40 - 2.03 (m, 9H), 2.01 - 1.51 (m, AcOH, 21H), 1.34 - 1.22 (m, 3H), 1.08 - 1.01 (m, 1H), 0.89 - 0.80 (m, 6H), 0.76 - 0.70 (m, 6H).

**Compound 100:** Val-Pro-Pro-Lys-Glu-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{88}H_{150}N_{26}O_{23}$; m/z: 389.0348 ([M+5H]$^{5+}$), 480.0415 ([M+4H]$^{4+}$), 647.7197 ([M+3H]$^{3+}$).

$^1$H NMR (600 MHz, $D_2O$) δ 4.49 - 4.42 (m, 2H), 4.34 - 4.06 (m, 13H), 4.05 - 4.00 (m, 2H), 3.96 (d, $J$ = 7.6 Hz, 1H), 3.80 - 3.73 (m, 1H), 3.69 (d, $J$ = 9.8 Hz, 4H), 3.58 - 3.45 (m, 5H), 3.07 (t, $J$ = 6.9 Hz, 2H), 2.88 - 2.83 (m, 6H), 2.27 (t, $J$ = 7.7 Hz, 3H), 2.20 - 2.11 (m, 9H), 2.01 - 1.45 (m, AcOH, 55H), 1.24 (dd, $J$ = 21.5, 7.2 Hz, 16H), 0.97 (d, $J$ = 6.9 Hz, 3H), 0.88 - 0.78 (m, 15H).

**Compound 101:** Glu-Pro-Val-Pro-Gln-Ala-Lys-Ser-Ser-Glu-Asp-Ile-Lys-Glu (acetate salt)

High resolution mass spectrometry (Orbitrap-HRMS), molecular formula: $C_{66}H_{109}N_{17}O_{26}$, m/z: 519.5978 ([M+3H]$^{3+}$), 778.8933 ([M+2H]$^{2+}$).

$^1$H NMR (600 MHz, $D_2O$) δ 4.50 (dd, $J$ = 8.0, 6.0 Hz, 1H), 4.40 (dd, $J$ = 8.4, 6.4 Hz, 1H), 4.36 - 4.30 (m, 2H), 4.27 - 4.19 (m, 6H), 4.18 - 4.11 (m, 2H), 4.08 (dd, $J$ = 8.8, 4.9 Hz, 1H), 4.01 (d, $J$ = 8.1 Hz, 1H), 3.82 - 3.71 (m, 5H), 3.62 - 3.47 (m, 3H), 2.86 (t, $J$ = 7.5 Hz, 4H), 2.65 (dd, $J$ = 16.4, 6.0 Hz, 1H), 2.51 (dd, $J$ = 16.3, 8.1 Hz, 1H), 2.34 (t, $J$ = 7.2 Hz, 2H), 2.31 - 2.10 (m, 9H), 2.06 - 1.52 (m, AcOH, 28H), 1.35 - 1.23 (m, 8H), 1.08 - 1.00 (m, 1H), 0.86 (dd, $J$ = 11.5, 6.7 Hz, 6H), 0.76 - 0.70 (m, 6H).

**Compound 102:** Glu-Pro-Val-Pro-Gln-Ala-Lys-Val-Pro-Tyr (acetate salt)

High resolution mass spectrometry (Orbitrap-HRMS), molecular formula: $C_{53}H_{82}N_{12}O_{15}$; m/z: 564.3073 ([M+2H]$^{2+}$).

$^1$H NMR (600 MHz, $D_2O$) δ 7.01 - 6.95 (m, 2H), 6.69 (d, $J$ = 8.5 Hz, 2H), 4.40 (dd, $J$ = 8.3, 6.4 Hz, 1H), 4.29 - 4.10 (m, 9H), 3.79 - 3.71 (m, 1H), 3.68 - 3.45 (m, 5H), 2.92 - 2.81 (m, 4H), 2.33 (t, $J$ = 7.3 Hz, 2H), 2.26 (t, $J$ = 7.7 Hz, 2H), 2.20 - 2.13 (m, 2H), 2.06 - 1.52 (m, AcOH, 25H), 1.33 - 1.21 (m, 5H), 0.86 (dd, $J$ = 11.3, 6.7 Hz, 6H), 0.77 (dd, $J$ = 17.6, 6.7 Hz, 6H).

**Compound 103:** Lys-Glu-Pro-Val-Pro-Gln-Ala-Lys-Pro-Arg-Lys-Val-Ala-Ala-Glu (acetate salt)

High resolution mass spectrometry (Orbitrap-HRMS), molecular formula: $C_{73}H_{126}N_{22}O_{21}$; m/z: 824.4799 ([M+2H]$^{2+}$).

$^1$H NMR (600 MHz, $D_2O$) δ 4.49 (dd, $J$ = 9.6, 4.4 Hz, 1H), 4.45 (dd, $J$ = 9.4, 4.9 Hz, 1H), 4.36 - 4.05 (m, 11H), 4.01 - 3.96 (m, 2H), 3.92 - 3.87 (m, 1H), 3.82 - 3.64 (m, 3H), 3.60 - 3.46 (m, 3H), 3.07 (t, $J$ = 7.0 Hz, 2H), 2.88 - 2.83 (m, 6H), 2.29 - 2.09 (m, 9H), 1.99 - 1.46 (m, AcOH, 47H), 1.39 - 1.20 (m, 16H), 0.85 (dd, $J$ = 14.2, 6.7 Hz, 6H), 0.80 (dd, $J$ = 6.7, 3.7 Hz, 6H).

**Compound 104:** Lys-Glu-Pro-Val-Pro-Gln-Ala-Lys-Pro-Arg-Lys-Val-Ala-Ala-Asn (acetate salt)

High resolution mass spectrometry (Orbitrap-HRMS), molecular formula: $C_{72}H_{125}N_{23}O_{20}$; m/z: 408.9938 ([M+4H]$^{4+}$), 544.9891 ([M+3H]$^{3+}$), 816.9801 ([M+2H]$^{2+}$).

$^1$H NMR (600 MHz, $D_2O$) δ 4.49 (dd, $J$ = 9.7, 4.4 Hz, 1H), 4.45 (dd, $J$ = 9.4, 4.8 Hz, 1H), 4.34 - 4.10 (m, 11H), 3.96 (d, $J$ = 7.6 Hz, 1H), 3.92 - 3.84 (m, 1H), 3.78 - 3.66 (m, 3H), 3.62 - 3.43 (m, 3H), 3.07 (t, $J$ = 6.9 Hz, 2H), 2.90 - 2.82 (m, 6H), 2.63 (dd, $J$ = 15.1, 4.9 Hz, 1H), 2.53 (dd, $J$ = 15.1, 8.1 Hz, 1H), 2.30 - 2.11 (m, 7H), 2.03 - 1.42 (m, AcOH, 44H), 1.39 - 1.19 (m, 15H), 0.85 (dd, $J$= 14.0, 6.7 Hz, 6H), 0.80 (dd, $J$= 6.8, 3.0 Hz, 6H).

**Compound 105:** Lys-Gln-Pro-Val-Pro-Gln-Ala-Lys-Pro-Arg-Lys-Val-Ala-Ala-Gln (acetate salt)

High resolution mass spectrometry (Orbitrap-HRMS), molecular formula: $C_{73}H_{128}N_{24}O_{19}$; m/z: 412.2515 ($[M+4H]^{4+}$), 549.3327 ($[M+3H]^{3+}$), 823.4955 ($[M+2H]^{2+}$).
$^1$H NMR (600 MHz, $D_2O$) δ 4.53 (dd, $J$ = 9.0, 5.0 Hz, 1H), 4.44 (dd, $J$ = 9.4, 4.8 Hz, 1H), 4.34 (dd, $J$ = 8.4, 6.0 Hz, 1H), 4.30 - 4.23 (m, 3H), 4.22 - 4.11 (m, 6H), 4.02 (dd, $J$ = 8.4, 4.8 Hz, 1H), 3.96 (d, $J$ = 7.6 Hz, 1H), 3.90 (t, $J$ = 6.7 Hz, 1H), 3.80 - 3.63 (m, 3H), 3.60 - 3.42 (m, 3H), 3.07 (t, $J$ = 6.9 Hz, 2H), 2.89 - 2.82 (m, 6H), 2.34 - 2.24 (m, 4H), 2.22 - 2.07 (m, 5H), 2.01 - 1.45 (m, AcOH, 50H), 1.39 - 1.20 (m, 15H), 0.85 (dd, $J$ = 15.5, 6.7 Hz, 6H), 0.80 (dd, $J$ = 6.8, 4.3 Hz, 6H).

**Compound 106:** Lys-Glu-Pro-Ile-Pro-Gln-Ala-Lys-Pro-Arg-Lys-Val-Ala-Ala-Gln (acetate salt)

High resolution mass spectrometry (Orbitrap-HRMS), molecular formula: $C_{74}H_{129}N_{23}O_{20}$; m/z: 416.0015 ($[M+4H]^{4+}$), 554.3328 ($[M+3H]^{3+}$), 830.9956 ($[M+2H]^{2+}$).
$^1$H NMR (600 MHz, $D_2O$) δ 4.50 (dd, $J$ = 9.6, 4.4 Hz, 1H), 4.45 (dd, $J$ = 9.3, 4.9 Hz, 1H), 4.36 - 4.22 (m, 4H), 4.22 - 4.10 (m, 6H), 4.02 (dd, $J$ = 8.4, 4.9 Hz, 1H), 3.96 (d, $J$ = 7.6 Hz, 1H), 3.92 - 3.84 (m, 1H), 3.82 - 3.74 (m, 1H), 3.73 - 3.63 (m, 2H), 3.61 - 3.41 (m, 3H), 3.07 (t, $J$ = 6.9 Hz, 2H), 2.90 - 2.82 (m, 6H), 2.32 - 2.12 (m, 9H), 2.00 - 1.46 (m, AcOH, 46H), 1.43 - 1.05 (m, 17H), 0.84 (d, $J$ = 6.8 Hz, 3H), 0.80 (dd, $J$ = 6.8, 4.3 Hz, 6H), 0.75 (t, $J$ = 7.4 Hz, 3H).

**Compound 107:** Val-Pro-Gln-Ala (acetate salt)

High resolution mass spectrometry (Orbitrap-HRMS), molecular formula: $C_{18}H_{31}N_5O_6$; m/z: 414.2341 ($[M+1H]^+$).
$^1$H NMR (600 MHz, $D_2O$) δ 4.29 (t, $J$ = 7.6 Hz, 1H), 4.07 (dd, $J$ = 8.5, 6.1 Hz, 1H), 4.01 - 3.91 (m, 2H), 3.61 - 3.53 (m, 1H), 3.46 - 3.41 (m, 1H), 2.24 - 2.10 (m, 4H), 1.95 - 1.69 (m, AcOH, 6H), 1.14 (d, $J$ = 7.3 Hz, 3H), 0.91 (d, $J$ = 7.0 Hz, 3H), 0.80 (d, $J$ = 6.8 Hz, 3H).

**Compound 108:** Val-Pro-Gln-Ala-Lys (acetate salt)

High resolution mass spectrometry (Orbitrap-HRMS), molecular formula: $C_{24}H_{43}N_7O_7$; m/z: 271.6681 ($[M+2H]2+$), 542.3289 ($[M+1H]^+$).
$^1$H NMR (600 MHz, $D_2O$) δ 4.28 (t, $J$ = 7.6 Hz, 1H), 4.13 - 4.06 (m, 2H), 3.99 (d, $J$ = 5.5 Hz, 1H), 3.94 (dd, $J$ = 8.2, 5.1 Hz, 1H), 3.60 - 3.54 (m, 1H), 3.46 - 3.41 (m, 1H), 2.78 (t, $J$ = 7.5 Hz, 2H), 2.21 (t, $J$ = 7.6 Hz, 2H), 2.17 - 2.09 (m, 2H), 1.93 - 1.58 (m, AcOH, 11H), 1.53 - 1.44 (m, 3H), 1.20 (dd, $J$ = 9.7, 7.5 Hz, 5H), 0.91 (d, $J$ = 6.9 Hz, 3H), 0.80 (d, $J$ = 6.9 Hz, 3H).

## Example 2: Experiment on the efficacy of polypeptide compounds in relieving colitis

**Experimental animals:** Wild-type AB strain zebrafishes, bred by natural pair mating. Zebrafishes aged 3 days post fertilization (3 dpf). Zebrafishes were kept in fish farming water at 28°C (water quality: 200 mg of instant sea salt in each 1L of reverse osmosis water, with the conductivity of 450-550 μS/cm; pH of 6.5-8.5; and hardness of 50-100 mg/L CaCOs), the experimental animal use license number is: SYXK (Zhe) 2012-0171, and the breeding management met the requirements of the international AAALAC certification (certification number: 001458).

**Experimental method:**

(1) Grouping: Polypeptide group: prepared into a stock solution of 20.0 mg/mL with ultrapure water and stored at -20°C.

Positive control group: prednisone, as white powder, with batch number C10016501, Shanghai Macklin Biochemical Technology Co., Ltd., stored at 4°C; prepared into a 15.0 mg/mL stock solution with DMSO, and stored in aliquots at -20°C.
Normal control group and model control group.
(2) Experimental method: 3 dpf wild-type AB strain zebrafishes were randomly selected and placed in a 6-well plate, with 30 zebrafishes in each well (experimental group). Except for the normal control group, all other experimental groups were given TNBS in water to establish the zebrafish model of intestinal mucosal injury. After 2 days of treatment at 28°C, TNBS was removed, and polypeptide compound in water (at concentrations shown in Table 4) was given respectively, and for the positive control, prednisone was 15.0 µg/mL. A normal control group (healthy group) and a model control group (intestinal mucosal injury group) were set up at the same time. The capacity of each well (experimental group) was 3 mL. After 2 days of treatment at 28°C, 10 zebrafishes were randomly selected from each experimental group and photographed under a dissecting microscope. NIS-Elements D 3.20 advanced image processing software was used to analyze and collect data, and the intestinal lumen area of the zebrafish was analyzed. The statistical analysis results of this indicator were used to evaluate the efficacy of the samples in relieving colitis. The statistics results were expressed as mean±SE. SPSS26.0 software was used for statistical analysis, and $p < 0.05$ indicates statistically significant difference.

**Experimental results:** The results of the efficacy of the polypeptide compound in relieving colitis are shown in Table 4:

**Table 4 Results of the experimental evaluation of the efficacy of polypeptide compounds in relieving colitis (n=10)**

| Group | Concentration (µg/mL) | Intestinal lumen area (pixels, mean ± SE) |
|---|---|---|
| Normal control group | - | 32743 ± 1560*** |
| Model control group | - | 41868 ± 1671 |
| Prednisone group | 15.0 | 34929 ± 1913* |
| Compound **1** | 250 | 39333 ± 2095 |
| | 500 | 26497 ± 1245*** |
| | 1000 | 26174 ± 1898*** |
| Compound **4** | 250 | 48915 ± 2720 |
| | 500 | 40432 ± 3736 |
| | 1000 | 33664 ± 3758* |
| Compound **5** | 250 | 42960 ± 2661 |
| | 500 | 45713 ± 4481 |
| | 1000 | 33977 ± 3524* |
| Compound **6** | 250 | 53093 ± 3839 |
| | 500 | 45831 ± 3894 |
| | 1000 | 28429 ± 3711** |
| Compound **12** | 250 | 48857 ± 5649 |
| | 500 | 43802 ± 3228 |
| | 1000 | 26727 ± 2106** |
| Compound **15** | 250 | 40719 ± 4647 |
| | 500 | 31319 ± 5274* |
| | 1000 | 30268 ± 2437* |

Compared with the model control group, *$p < 0.05$, **$p < 0.01$, ***$p < 0.001$

According to the data in Table 4, it can be known that the polypeptide compound groups including compounds **1**, **4**, **5**, **6**, **12** and **15** showed significant statistical differences compared with the model control group ($p < 0.05$; $p < 0.01$ ; $p < 0.001$), and the effect was better than that of the prednisone group. The experimental results show that the polypeptide compound provided in the present application has the effect of alleviating colitis, which is specifically manifested in significantly alleviating intestinal lumen dilatation.

The concentration gradient of the test substances set in this experimental system was 250 µg/mL, 500 µg/mL, and 1000 µg/mL. By counting the intestinal lumen area of the zebrafish after intervention in each group, it can be seen that most of the test substances with the effect of relieving colitis had a more obvious effect at a concentration of 1000 µg/mL ($p < 0.05$; $p < 0.01$; $p < 0.001$). When the concentration of the test substance was further increased for intervention, the death phenomenon of zebrafishes was found in the group; therefore, in the subsequent rounds of activity evaluation, each test polypeptide compound was uniformly administered at 1000 μg/mL for intervention. The results are summarized in Table 5.

**Table 5 Summary of the activity of polypeptide compounds in alleviating intestinal lumen dilatation of colitis in zebrafishes**

| Compound No. | Activity in relieving intestinal lumen dilatation | Compound No. | Activity in relieving intestinal lumen dilatation | Compound No. | Activity in relieving intestinal lumen dilatation |
|---|---|---|---|---|---|
| 1 | *** | 37 | + | 73 | ND |
| 2 | ND | 38 | * | 74 | + |
| 3 | ND | 39 | + | 75 | * |
| 4 | * | 40 | + | 76 | + |
| 5 | * | 41 | + | 77 | - |
| 6 | ** | 42 | + | 78 | + |
| 7 | + | 43 | ND | 79 | + |
| 8 | + | 44 | - | 80 | + |
| 9 | * | 45 | # | 81 | + |
| 10 | * | 46 | + | 82 | + |
| 11 | + | 47 | + | 83 | * |
| 12 | ** | 48 | * | 84 | + |
| 13 | - | 49 | * | 85 | * |
| 14 | + | 50 | * | 86 | * |
| 15 | * | 51 | * | 87 | + |
| 16 | + | 52 | + | 88 | * |
| 17 | + | 53 | * | 89 | ** |
| 18 | - | 54 | ** | 90 | * |
| 19 | + | 55 | * | 91 | + |
| 20 | & | 56 | ND | 92 | ** |
| 21 | + | 57 | ** | 93 | * |
| 22 | + | 58 | + | 94 | ** |
| 23 | + | 59 | + | 95 | ** |
| 24 | + | 60 | - | 96 | *** |
| 25 | * | 61 | ND | 97 | ** |
| 26 | * | 62 | * | 98 | ** |
| 27 | + | 63 | *** | 99 | * |
| 28 | + | 64 | + | 100 | *** |
| 29 | * | 65 | * | 101 | ND |
| 30 | + | 66 | * | 102 | + |
| 31 | * | 67 | * | 103 | * |
| 32 | + | 68 | ** | 104 | *** |
| 33 | + | 69 | + | 105 | ** |
| 34 | * | 70 | * | 106 | ** |
| 35 | - | 71 | + | 107 | ND |

(continued)

| Compound No. | Activity in relieving intestinal lumen dilatation | Compound No. | Activity in relieving intestinal lumen dilatation | Compound No. | Activity in relieving intestinal lumen dilatation |
|---|---|---|---|---|---|
| 36 | + | 72 | ** | 108 | ND |

Note: The activity of each compound to relieve intestinal lumen dilatation of colitis in zebrafishes was completed in several batches of trials, and for comparison purposes, its activity against intestinal lumen dilatation was compared with that of the control group of an equivalent number of batches of the experimental model: *p < 0.05, **p < 0.01, ***p < 0.001; ND indicates not detected, "-" indicates absence of activity, "+" indicates some relief of intestinal lumen dilatation, "#" indicates that all the zebrafishes died in the experiment, and "&" indicates that 13 zebrafishes died in the experiment.

According to the data in Table 4 and Table 5, there was a significant statistical difference between the polypeptide groups and the model control group (p<0.05, p<0.01 or p<0.001), and the effect was better than that of the prednisone group. Under the experimental conditions, compounds **1**, **4-6**, **9**, **10**, **12**, **15**, **25**, **26**, **29**, **31**, **34**, **38**, **48-51**, **53-55**, **57**, **62**, **63**, **65-68**, **70**, **72**, **75**, **83**, **85**, **86**, **88-90**, **92-100**, and **103-106** all had a significant effect of alleviating colitis, as manifested by relieving intestinal lumen dilatation (p <0.05); and compounds 7, 8, 11, 14, 16, 17, 19, 21-24, 27, 28, 30, 32, 33, 36, 37, 39-42, 46, 47, 52, 58, 59, 64, 69, 71, 74, 76, 78-82, 84, 87, 91 and 102 had a certain effect of alleviating intestinal lumen dilatation.

## Example 3: Evaluation of the therapeutic effects of polypeptide compounds on TNBS-induced model of acute inflammatory bowel disease (UC) in SD rat

**Objective**: To evaluation the therapeutic effects of polypeptide compounds on TNBS-induced model of acute inflammatory bowel disease in SD rat

## Materials and Methods:

In this experiment, a rat model of ulcerative colitis was established by administering trinitrobenzene sulfonic acid (TNBS) to laboratory animal male SD rats for a single enema intervention. Acute colitis in rats induced by trinitrobenzene sulfonic acid (TNBS) is an immune response induced by cytokines secreted by Thl type cells. The colon of animals in the model control group shows the reactivity of obvious atrophy and intestinal wall thickening, and the ratio of colon weight to length increases significantly. Fissure-like ulcer foci are observed on the surface of the intestinal mucosa, and typical granulomas are observed in the submucosal layer and muscular layer, internally accompanied by infiltration of inflammatory cells mainly composed of lymphocytes and plasma cells. At the same time, there is obvious hyperplasia of small blood vessels and connective tissue (fibroblasts), and crypt structure destruction and bleeding are observed around the ulcer foci. This model is simple to establish and has good reproducibility. It is an experimental animal model widely used in evaluating drugs for the treatment of inflammatory bowel disease. The experimental animals (male SD rats) were randomly divided into 6 groups according to body weight parameters on the first day of the experiment, namely normal control group, model control group, positive drug mesalazine treatment group, compound **1** low-dose treatment group, compound **1** high-dose treatment group, compound **6** low-dose treatment group, compound **6** high-dose treatment group and compound **12** treatment group, with 10 rats in each group. On the day of grouping (D0), except for the normal control group which was given the vehicle, the animals in the remaining experimental groups were given TNBS in ethanol by enema to induce the model; the test article and positive control drug treatments were started on the second day of model induction, with the treatment dose being respectively 3.0 mg/kg and 6.0 mg/kg for compound **1**, respectively 1.4 mg/kg and 2.8 mg/kg for compound **6**, 0.8 mg/kg for compound **12**, and 30.0 mg/kg for positive control drug mesalazine, all of which were administered by enema at a frequency of once a day for 14 consecutive days. During the experimental treatment period (D1-D14), the body weight and general clinical observation indicators (including but not limited to food intake, water intake, activity level and defecation, etc.) of the animals in each experimental group were dynamically collected. At the same time, based on the relevant scoring standards for the disease activity index of ulcerative colitis, the reactivity such as the weight loss degree, stool morphology and trait, and bloody stool of the animals in each experimental group after TNBS induction were scored, and the disease activity index (DAI) of ulcerative colitis was calculated, once a day for 14 consecutive days. At the end point of the experiment, colon tissues of animals in each experimental group were collected, and the length and weight of the colon were measured and then the non-autopsy images were collected. Then the colon was cut open along the intestinal wall longitudinally and autopsy images were collected. Based on the "Criteria for Scoring Gross Morphological Manifestations of the Colon", the gross damage with respect to colon adhesions, colon ulcers and inflammation of animals in each experimental group was observed and scored. Ulcer images were collected under a stereomicroscope and the area of colon ulcer foci was calculated, followed by routine fixation. According to the statistical analysis results of various pharmacodynamic test indicators, the therapeutic effect of the test polypeptide compound on the ulcerative colitis disease model under the set treatment regimen was evaluated.

**Results and conclusions:**

In this experimental research system, the animals in the model control group showed obvious disease characteristics of ulcerative colitis, including weight gain inhibition, soft and loose stools, significantly increased ulcerative colitis disease activity index (DAI) score, and macroscopically observed colon atrophy and typical ulcer foci and colon fusion ulcer foci. The test compound**1** had a significant effect of improving the weight gain inhibition of the model animals under the set treatment regimen, and the degree of improvement was statistically significant compared with the weight change degree of the animals in the model control group in the same period (*P<0.05*), while the test compounds **6** and **12** had no obvious improved therapeutic effect on the weight gain inhibition of the model animals.

**Table 6 Effects of the test polypeptide compounds on changes in the body weight of model animals**

| Group | Grouping day (D0, g) | Experimental day 5 (D5, g) | Experimental endpoint (D14, g) | Weight change range (D0-D14, %) |
|---|---|---|---|---|
| Normal control group | 273.86±7.48 | 281.46±12.97 | 310.67±22.39 | 13.38±6.25 |
| Model control group | 278.66±9.87 | 236.52±14.56*** | 267.73±28.89** | -3.83±10.82*** |
| Mesalazine treatment group | 276.64±7.47 | 234.86±8.07 | 264.43±17.65 | -4.29±7.79 |
| Compound 1 low dose group | 274.40±9.10 | 251.65±18.30# | 296.07±19.58# | 7.96±7.32# |
| Compound 1 high dose group | 279.99±10.57 | 263.90±15.38## | 299.37±14.64## | 6.97±4.73## |
| Compound 6 low dose group | 276.61±9.81 | 243.72±22.18 | 277.96±29.18 | 0.48±9.87 |
| Compound 6 high dose group | 275.55±7.92 | 246.74±22.76 | 279.67±30.93 | 1.50±10.78 |
| Compound 12 treatment group | 276.36±7.47 | 244.90±21.75 | 272.00±33.21 | -1.52±12.20 |

Note: All data were statistically analyzed using mean ± standard deviation (SD). *P<0.05, **P<0.01, ***P<0.001, ****P<0.0001 represent the statistical analysis results of the model control group and the normal control group. #P<0.05, ##P<0.01, represent the statistical analysis results of the test polypeptide compound treatment group and the model control group.

The DAI score results of the experimental period show that the test compounds **1**, **6** and **12** had a significant improvement effect on the disease progression of ulcerative colitis in the model animals under the set treatment regimen. The fecal trait pathological characteristics and weight loss reactivity of the animals in the corresponding treatment groups were significantly alleviated, and the mean DAI scores showed a decrease, which was significantly statistically different from the model control group in the same period (*P<0.05*). The improvement in treatment effect of compound **1** was the most significant, and the treatment effects of the compounds **1** and **6** were significantly dose-dependent.

**Table 7 Effects of the test polypeptide compounds on the DAI scores of model animals**

| Group | Experimental day 1 (D1, point) | Experimental day 3 (D3, point) | Experimental endpoint (D14, point) | $AUC_{(0-14d)}$ (D0-D14) |
|---|---|---|---|---|
| Normal control group | 0.3±0.5 | 0.2±0.4 | 0 | 1.10±0.65 |
| Model control group | 3.8±1.0 | 6.1±1.0 | 2.2±1.3 | 57.00±2.97**** |
| Mesalazine treatment group | 4.2±0.4 | 6.2±0.6 | 2.1±1.6 | 58.75±2.93 |
| Compound 1 low dose group | 3.5±1.3 | 4.9±1.6 | 0.5±0.7 | 37.65±3.60#### |
| Compound 1 high dose group | 3.9±0.6 | 4.3±1.8 | 0.1±0.3 | 3065±293####+++ |
| Compound 6 low dose group | 3.9±0.9 | 5.0±1.7 | 1.3±1.2 | 49.65±3.95#### |
| Compound 6 high dose group | 4.2±0.8 | 4.8±1.0 | 0.7±1.2 | 39.55±3.99####++++ |

(continued)

| Group | Experimental day 1 (D1, point) | Experimental day 3 (D3, point) | Experimental endpoint (D14, point) | $AUC_{(0-14d)}$ (D0-D14) |
|---|---|---|---|---|
| Compound 12 treatment group | 4.1±0.6 | 4.9±1.0 | 1.4±1.8 | 46.40±3.68#### |

Note: All data were statistically analyzed using mean ± standard deviation (SD). *$P$<0.05, **$P$<0.01, ***$P$<0.001, and ****$P$<0.0001 represent the statistical analysis results of the model control group and the normal control group. #$P$<0.05, ##$P$<0.01, ###$P$<0.001, and ####$P$<0.0001 represent the statistical analysis results of the animals in the test polypeptide compound treatment group and the model control group. +$P$<0.05, ++$P$<0.01, +++$P$<0.001, and ++++$P$<0.0001 represent the statistical analysis results between the compound **1 or 6** dose groups.

The results of gross anatomy and colon tissue injury scores at the experimental endpoint show that the test compounds **1**, **6** and **12** had obvious therapeutic effects on TNBS-induced gross colon injury status (colonic adhesion, ulcer and inflammation), colon ulcer focus area and abnormal increase in colon weight/length ratio. The degree of improvement was statistically significant compared with the model control group in the same period (*$P$<0.05*). The therapeutic effect of the compound **1** was the most significant, and its therapeutic effect was significantly dose-dependent.

**Table 8 Effects of the test polypeptide compounds on the gross damage and ulcer area of the colon in model animals**

| Group | Colonic adhesion score (point) | Colon ulcer and inflammation score (point) | Colon ulcer area (cm²) |
|---|---|---|---|
| Normal control group | 0 | 0 | 0 |
| Model control group | 2.80±0.42**** | 4.40±0.70**** | 7.72±4.02**** |
| Mesalazine treatment group | 2.60±0.52 | 4.30±0.95 | 7.50±3.51 |
| Compound 1 low dose group | 1.10±0.57#### | 2.50±0.85 | 3.77±1.91# |
| Compound 1 high dose group | 0.60±0.52#### | 1.40±0.84####++ | 2.15±0.74###+ |
| Compound 6 low dose group | 1.70±067### | 3.00±0.94## | 3.46±2.06## |
| Compound 6 high dose group | 1.30±082#### | 2.50±1.43## | 3.66±2.36# |
| Compound 12 treatment group | 1.20±063#### | 2.30±0.82#### | 3.79±1.63# |

Note: All data were statistically analyzed using mean ± standard deviation (SD). *$P$<0.05, **$P$<0.01, ***$P$<0.001, and ****$P$<0.0001 represent the statistical analysis results of the model control group and the normal control group. #$P$<0.05, ##$P$<0.01, ###$P$<0.001, and ####$P$<0.0001 represent the statistical analysis results of the animals in the test polypeptide compound treatment group and the model control group. +$P$<0.05, ++$P$<0.01, +++$P$<0.001, and ++++$P$<0.0001 represent the statistical analysis results between the compound **1 or 6** dose groups.

**Table 9 Effects of the test polypeptide compounds on the colon weight/length ratio of model animals**

| Group | Colon weight (g) | Colon length (cm) | Colon weight/length ratio (100* g/cm) |
|---|---|---|---|
| Normal control group | 1.90±0.16 | 18.14±1.23 | 10.53±1.01 |
| Model control group | 3.26±0.97*** | 12.76±0.92**** | 25.37±6.35**** |
| Mesalazine treatment group | 3.42±1.15 | 14.76±2.06 | 22.99±5.49 |
| Compound 1 low dose group | 2.48±0.46# | 15.34±1.99## | 16.40±3.29### |
| Compound 1 high dose group | 2.20±0.28## | 16.55±1.67#### | 13.37±1.93####+ |
| Compound 6 low dose group | 2.23±0.39## | 13.37±1.40 | 16.93±3.87## |
| Compound 6 high dose group | 2.41±0.63# | 13.89±1.60 | 17.70±5.71# |

(continued)

| Group | Colon weight (g) | Colon length (cm) | Colon weight/length ratio (100* g/cm) |
|---|---|---|---|
| **Compound 12 treatment group** | 2.10±0.19## | 13.74±1.97 | 15.68±3.14### |

Note: All data were statistically analyzed using mean ± standard deviation (SD). *$P<0.05$, **$P<0.01$, ***$P<0.001$, and ****$P<0.0001$ represent the statistical analysis results of the model control group and the normal control group. #$P<0.05$, ##$P<0.01$, ###$P<0.001$, and ####$P<0.0001$ represent the statistical analysis results of the animals in the test polypeptide compound treatment group and the model control group. +$P<0.05$, ++$P<0.01$, +++$P<0.001$, and ++++$P<0.0001$ represent the statistical analysis results between the compound **1** or **6** dose groups.

In this trial, TNBS was used for intervention by enema in male SD rats to induce colonic lesions and establish a rat ulcerative colitis model that conforms to the clinical characteristics of the disease. The next day after model induction, the treatment with test compounds **1**, **6** and **12** and positive control drug mesalazine was started. During the treatment period (D1-D14), the changes in the weight, the disease activity index (DAI) score of ulcerative colitis, the score of gross colon damage manifestation (colonic adhesion, ulcer and inflammatory response), the ulcer area of the colon tissue and the ratio of colon weight to length of the experimental animals were monitored to evaluate the therapeutic effect of the test articles on the model of TNBS-induced ulcerative colitis in SD rat. The results showed that:

1) After TNBS induction, the experimental animals showed significant manifestations of ulcerative colitis disease, including significant weight loss, significant increase in ulcerative colitis disease activity index score (DAI score), significant increase in colon adhesion and ulcer score, increase in colon tissue ulcer area and significant increase in colon weight/length ratio, indicating that the rat ulcerative colitis model suitable for the pharmacodynamic study of this experiment was successfully established.
2) The test compound **1** had a significant improvement effect on the weight loss response of the model animals under the set treatment regimen, and the degree of improvement was statistically significant compared with the model control group during the same period (*P<0.05*), while the test compounds **6**, **12** and the positive control drug mesalazine did not show a significant weight improvement effect.
3) The test compounds **1**, **6** and **12** had a significant improvement effect on the disease progression of ulcerative colitis in model animals under the set treatment regimen, and their DAI scores showed a significant decrease compared with the model control group during the same period. The therapeutic effect of the test compound **1** was the most significant. At the same time, the treatment improvement effect of the test compounds **1** and **6** was significantly dose-dependent, while the positive control drug mesalazine had no significant effect on the disease symptoms of ulcerative colitis in model animals.
4) The test compounds **1**, **6** and **12** had significant therapeutic effects on the gross damage manifestation of the colon in model animals under the set treatment regimen. Compared with the model control group in the same period, their colon adhesion score, colon ulcer and inflammation score and colon tissue ulcer area all showed a significant decrease, and the degree of improvement was statistically significant (*P<0.05*). The therapeutic effect of the test compound **1** was the most significant, and its improved therapeutic effect was significantly dose-dependent.
5) The test compounds **1**, **6** and **12** had a significant improvement effect on the colon atrophy process of model animals under the set treatment regimen. Compared with the model control group in the same period, their colon weight/length ratio was significantly decreased (*P<0.05*).

It can be seen that this experiment established a rat model of ulcerative colitis that conforms to the clinical characteristics of the pathogenesis, and the results of the efficacy evaluation in this model show that the test compounds **1**, **6** and **12** all had significant therapeutic effects.

**Example 4: Study on the effect of polypeptide compounds on the proliferation of CaCo-2 cells in vitro**

The following cell lines were used to study in vitro proliferation of the polypeptide compounds:

**Table 10 Cell line information**

| Serial No. | Cell line | Cancer type | Growth characteristics | Culture medium |
|---|---|---|---|---|
| 1 | CaCo-2 | Colorectal cancer | Adherence | (MEM+0.01mM NEAA)+20%FBS |

Experimental method:

Cells will be thrawed and maintained according to the culture conditions shown in Table 10.

Day 1 (cell plating): Cells in the logarithmic growth phase were collected, resuspended, counted using a cell counter and detected for activity. The cell suspension was diluted according to the required plating density and 90 μL of the cell suspension was added into each well of a 96-well plate (the plating density of cell would be adjusted based on historical data or density optimization experiments). While plating, three replicate wells were plated for each cell as T0 plates. All 96-well plates were placed in a 37°C, 5% CO2 thermostatic incubator overnight.

Day 2 (medium change): The medium with the corresponding serum concentration was replaced according to the platemap, 90μL per well. Day 2: The T0 plate was read and treated with the test compound. 10 μL of culture medium was added into each well of the T0 plate to make up the total volume to 100 μL. 50 μL of CellTiter-Glo® Reagent was added to each well. The system was mixed and shaken for 5 min to fully lyse the cells. The system was incubated at room temperature for 10 min to stabilize the luminescent signal (note: temperature, cell density, and edge effects can all lead to uneven luminescent signals). Fluorescent signals were detected using EnVision Multi Label Reader. The test compound was configured according to the corresponding molecular weight, C1=91.08μM, diluted C2=9.108μM, and C3=0.9108μM. Cisplatin was diluted as shown in Table 11 to prepare a 10X compound working solution. 10 μL of working solution (10X) was added to each well, so that the final volume per well of all plates was 100 μL. The cells were cultured in a 37°C, 5% CO2 incubator.

On the 5th day, treatment with the test compound was performed for 72 h (plate reading): whether the status of the drug-treated group and the control group is normal was observed under a microscope. 50 μL of CTG reagent was added to each well. The system was mixed on a shaker for 5 min to allow cells to fully lyse. The system was incubated at room temperature for 10 min to stabilize the luminescent signal. Fluorescent signals were read using EnVision Multi Label Reader.

Table 11 Cisplatin concentration after dilution

| | C1-1 | C2-1 | C3-1 | C4-1 | C5-1 | C6-1 | C7-1 | C8-1 | C9-1 | C10-1 |
|---|---|---|---|---|---|---|---|---|---|---|
| Concentration (μM) | 1000 | 333.33 | 111.11 | 37.04 | 12.35 | 4.12 | 1.37 | 0.46 | 0.15 | 0 |
| Solution volume (μL) | 90 | 90 | 90 | 90 | 90 | 90 | 90 | 90 | 90 | 90 |
| Broth volume (μL) | 209.7 | 209.7 | 209.7 | 209.7 | 209.7 | 209.7 | 209.7 | 209.7 | 209.7 | 209.7 |

$$\text{Cell viability (\%)} = \text{Lum}_{\text{test compound}} / \text{Lum}_{\text{solvent control}} \times 100\%$$

Experimental results: The statistical results of the proliferation-promoting activity of the selected polypeptide compounds are shown in Table 12:

| Compound No. | Proliferation-promoting activity |
|---|---|
| **1** | Cell viability > 110% |
| **20** | / |
| **44** | / |
| **45** | / |

The concentration gradient of the administered test substance set in this experimental system was set as C1=91.08μM, diluted C2=9.108μM, and C3=0.9108μM, with 0.88% FBS as the reference. The experimental results show that the polypeptide compound 1 of the present application has the activity of promoting the proliferation of Caco-2 cells, which is specifically manifested as an increase in cell viability after intervention.

In summary, the above-mentioned polypeptide compound of the present invention has a short peptide chain and is absorbed faster and better. The experimental results show that the polypeptide compound provided in the present application has the activity of alleviating the intestinal lumen dilatation of colitis in zebrafishes; and has a significant therapeutic effect in the trial for rat model of acute inflammatory bowel disease model. The polypeptide compound of the present invention has a significant effect of alleviating colitis and can be used to prepare drugs for treating enteritis, especially drugs for ulcerative colitis.

Although the present invention discloses the above Examples, the embodiments of the present invention are not limited to the above Examples, and any other changes, modifications, replacements, combinations, and simplifications that do not deviate from the present invention should be considered as equivalents and are included in the scope of protection of the present invention.

**Claims**

1. A compound of formula (I) or a physiologically compatible salt thereof, wherein the compound of formula (I) is as follows:

$$H\text{-}X_a\text{-}Z_1\text{-}Z_2\text{-}Z_3\text{-}Z_4\text{-}X_b\text{-}OH \qquad \textbf{(I)}$$

wherein

$Z_1$ is Pro, Ala, Gly, D-Pro or is absent;
$Z_2$ is Val, Ala, D-Val, Ile or is absent;
$Z_3$ is Pro, Ala, Gly, D-Pro or is absent;
$Z_4$ is Gln, Ala, Glu, Ile, D-Gln or is absent;
provided that at most 2 of $Z_1$, $Z_2$, $Z_3$ and $Z_4$ are absent.
$X_a$ is a sequence containing 0-10 amino acid residues; and
$X_b$ is a sequence containing 0-9 amino acid residues.

2. The compound according to 1 or a physiologically compatible salt thereof, wherein 2 of $Z_1$, $Z_2$, $Z_3$ and $Z_4$ are absent; or $Z_1$ and $Z_2$ are absent; or $Z_3$ and $Z_4$ are absent; or 1 of $Z_1$, $Z_2$, $Z_3$ and $Z_4$ is absent; or $Z_1$ is absent; or $Z_4$ is absent; or 0 of $Z_1$, $Z_2$, $Z_3$ and $Z_4$ is absent.

3. The compound according to 1 or a physiologically compatible salt thereof, wherein 0 of $Z_1$, $Z_2$, $Z_3$ and $Z_4$ is absent.

4. The compound according to 1 or a physiologically compatible salt thereof, wherein $Z_1$ is Pro; $Z_2$ is Val; $Z_3$ is Pro; and $Z_4$ is Gln; or $Z_1$ is D-Pro; $Z_2$ is Val; $Z_3$ is Pro; and $Z_4$ is Gln; or $Z_1$ is Pro; $Z_2$ is Val; $Z_3$ is Ala; and $Z_4$ is Gln; or $Z_1$ is Pro; $Z_2$ is Ile; $Z_3$ is Pro; and $Z_4$ is Gln; or $Z_1$ is Pro; $Z_2$ is Val; $Z_3$ is Pro; and $Z_4$ is absent; or $Z_1$ is Pro; $Z_2$ is Ala; $Z_3$ is Pro; and $Z_4$ is Gln; or $Z_1$ is Gly; $Z_2$ is Val; $Z_3$ is Pro; and $Z_4$ is Gln; or $Z_1$ is Pro; $Z_2$ is Val; $Z_3$ is Gly; and $Z_4$ is Gln; or $Z_1$ is Ala; $Z_2$ is Val; $Z_3$ is Pro; and $Z_4$ is Gln; or $Z_1$ is Pro; $Z_2$ is Val; $Z_3$ is Pro; and $Z_4$ is Glu; or $Z_1$ is Pro; $Z_2$ is Val; $Z_3$ is D-Pro; and $Z_4$ is Gln; or $Z_1$ is Pro; $Z_2$ is Val; $Z_3$ is Pro; and $Z_4$ is D-Gln.

5. The compound according to 1 or a physiologically compatible salt thereof, wherein $Z_1$ is Pro; $Z_2$ is Val; $Z_3$ is Pro; and $Z_4$ is Gln.

6. The compound according to any one of claims 1-5 or a physiologically compatible salt thereof, wherein

$X_a$ is $X_{a10}\text{-}X_{a9}\text{-}X_{a8}\text{-}X_{a7}\text{-}X_{a6}\text{-}X_{a5}\text{-}X_{a4}\text{-}X_{a3}\text{-}X_{a2}\text{-}X_{a1}\text{-}*$, wherein * indicates the position linked to $Z_1\text{-}Z_2\text{-}Z_3\text{-}Z_4$, wherein
$X_{a10}$ is Gly or is absent,
$X_{a9}$ is Gly, Pro, Ser or is absent,
$X_{a8}$ is Pro, Glu, Ser or is absent,
$X_{a7}$ is Arg, Glu, Thr, Ser or is absent,
$X_{a6}$ is Lys, Thr, Ala, Asp, Glu, Arg, Ser or is absent,
$X_{a5}$ is Asp, Ala, Val, Arg, Phe, Ile, Pro, Lys, Glu or is absent,
$X_{a4}$ is Val, Phe, Pro, Pyro-Glu, Lys, Leu, Ile, Ala, Asp or is absent,
$X_{a3}$ is Tyr, Leu, Pro, Asp, Arg, Glu, Lys, Tys, Val, Ile or is absent,
$X_{a2}$ is D-Lys, Lys, Ala, Arg, Val, Glu, Tyr or is absent, and
$X_{a1}$ is Glu, Ala, D-Glu, Tyr, Gln, Asp, Asn or is absent.

7. The compound according to 6 or a physiologically compatible salt thereof, wherein $X_a$ is $X_{aa}\text{-}X_{a2}\text{-}X_{a1}\text{-}*$, wherein * indicates the position linked to $Z_1\text{-}Z_2\text{-}Z_3\text{-}Z_4$, $X_{a2}\text{-}X_{a1}$ is Lys-Glu-*, Arg-Glu-*, Val-Tyr-*, Glu-Glu-*, Lys-Gln-*, Lys-D-Glu-* or Tyr-Glu-*, and $X_{aa}$ is $X_{a10}\text{-}X_{a9}\text{-}X_{a8}\text{-}X_{a7}\text{-}X_{a6}\text{-}X_{a5}\text{-}X_{a4}\text{-}X_{a3}$-, wherein $X_{a10}$ is Gly or is absent, $X_{a9}$ is Gly, Pro, Ser or is absent, $X_{a8}$ is Pro, Glu, Ser or is absent, $X_{a7}$ is Arg, Glu, Thr, Ser or is absent, $X_{a6}$ is Lys, Thr, Ala, Asp, Glu, Arg, Ser or is absent, $X_{a5}$ is Asp, Ala, Val, Arg, Phe, Ile, Pro, Lys, Glu or is absent, $X_{a4}$ is Val, Phe, Pro, Pyro-Glu, Lys, Leu, Ile, Ala, Asp or is absent, and $X_{a3}$ is Tyr, Leu, Pro, Asp, Arg, Glu, Lys, Tys, Val, Ile or is absent.

8. The compound according to 7 or a physiologically compatible salt thereof, wherein $X_{aa}$ is Arg-Lys-Asp-Val-Tyr-, Gly-Pro-Glu-Thr-Ala-Phe-Leu-, Val-Pro-Pro-, Pyro-Glu-Leu-, Arg-Lys-Asp-, Gly-Pro-Glu-Thr-Ala-Phe-Leu-Arg-, Ser-Ser-Glu-Asp-Ile-Lys-Glu-, Ser-Ser-Glu-Asp-Ile-Lys-, Val-Pro-Tys-, Pro-Ala-Tys-, Arg-Lys-Asp-Val-, Ser-Ser-Glu-Asp-Ile-, or is absent, wherein the rightmost connector - indicates linkage to -$X_{a2}$-$X_{a1}$.

9. The compound according to 7 or a physiologically compatible salt thereof, wherein $X_a$ is $X_{aa}$-$X_{a2}$-$X_{a1}$-*, wherein * indicates the position linked to $Z_1$-$Z_2$-$Z_3$-$Z_4$, wherein $X_{a2}$-$X_{a1}$ is Lys-Glu-*, and $X_{aa}$ is as defined above.

10. The compound according to 6 or a physiologically compatible salt thereof, wherein $X_a$ is Glu-*, Ala-*, D-Glu-*, Asp-*, Asn-*, Lys-Glu-*, D-Lys-Glu-*, Ala-Glu-*, Lys-Gln-*, Lys-D-Glu-*, Lys-Ala-*, Arg-Lys-Asp-Val-Tyr-Lys-Glu-*, Gly-Pro-Glu-Thr-Ala-Phe-Leu-Arg-Glu-*, Val-Pro-Pro-Lys-Glu-*, Pyro-Glu-Leu-Lys-Glu-*, Arg-Lys-Asp-Val-Tyr-*, Gly-Pro-Glu-Thr-Ala-Phe-Leu-Arg-Lys-Glu-*, Ser-Ser-Glu-Asp-Ile-Lys-Glu-LysGlu-*, Ser-Ser-Glu-Asp-Ile-Lys-Glu-Glu-*, Val-Pro-Tys-Lys-Glu-*, Pro-Ala-Tys-Lys-Glu-*, Arg-Lys-Asp-Val-Tyr-Glu-*, Ser-Ser-Glu-Asp-Ile-Lys-Glu-* or is absent, wherein * indicates the position linked to Z1-Z2-Z3-Z4.

11. The compound according to any one of claims 1-10 or a physiologically compatible salt thereof, wherein

   $X_b$ is **-$X_{b1}$-$X_{b2}$-$X_{b3}$-$X_{b4}$-$X_{b5}$-$X_{b6}$-$X_{b7}$-$X_{b8}$-$X_{b9}$, wherein ** indicates the position linked to $Z_1$-$Z_2$-$Z_3$-$Z_4$, wherein
   $X_{b1}$ is Ala, D-Ala or is absent,
   $X_{b2}$ is Lys, Ala, D-Lys, Arg or is absent,
   $X_{b3}$ is Pro, Gly, D-Pro, Ser, Val, Ala or is absent,
   $X_{b4}$ is Arg, Ala, Ser, Pro, D-Arg or is absent,
   $X_{b5}$ is Lys, Ala, D-Lys, Glu, Tyr or is absent,
   $X_{b6}$ is Val, Asp, D-Val, Ala or is absent,
   $X_{b7}$ is Ala, D-Ala, Ile or is absent,
   $X_{b8}$ is Ala, D-Ala, Lys or is absent, and
   $X_{b9}$ is Gln, Ala, Glu, Asn, D-Gln or is absent.

12. The compound according to claim 11 or a physiologically compatible salt thereof, wherein $X_b$ is **-$X_{b1}$-$X_{b2}$-$X_{b3}$-$X_{b4}$-$X_{b5}$-$X_{b6}$-$X_{b7}$-$X_{b8}$-$X_{b9}$, wherein ** indicates the position linked to $Z_1$-$Z_2$-$Z_3$-$Z_4$, wherein

   $X_{b1}$ is Ala or D-Ala,
   $X_{b2}$ is Lys, Ala or D-Lys,
   $X_{b3}$ is Pro, Gly, or Ala,
   $X_{b4}$ is Arg or D-Arg,
   $X_{b5}$ is Lys or is absent,
   $X_{b6}$ is Val, D-Val, Ala or is absent,
   $X_{b7}$ is Ala is absent,
   $X_{b8}$ is Ala or is absent, and
   $X_{b9}$ is Gln, Asn, D-Gln or is absent.

13. The compound according to claim 11 or 12 or a physiologically compatible salt thereof, wherein $X_b$ is **-$X_{b1}$-$X_{b2}$-$X_{bb}$, wherein ** indicates the position linked to $Z_1$-$Z_2$-$Z_3$-$Z_4$, $X_{b1}$-$X_{b2}$- is **-Ala-Lys-, **-Ala-Lys-, **-Ala-D-Lys-, **-Ala-Ala- or **-D-Ala-Lys-, $X_{bb}$ is -$X_{b3}$-$X_{b4}$-$X_{b5}$-$X_{b6}$-$X_{b7}$-$X_{b8}$-$X_{b9}$, wherein $X_{b3}$ is Pro, Gly, D-Pro, Ser, Val, Ala or is absent, $X_{b4}$ is Arg, Ala, Ser, Pro, D-Arg or is absent, $X_{b5}$ is Lys, Ala, D-Lys, Glu, Tyr or is absent, $X_{b6}$ is Val, Asp, D-Val, Ala or is absent, $X_{b7}$ is Ala, D-Ala, Ile or is absent, $X_{b8}$ is Ala, D-Ala, Lys or is absent, and $X_{b9}$ is Gln, Ala, Glu, Asn, D-Gln or is absent; or $X_{b3}$ is Pro, Gly, or Ala, $X_{b4}$ is Arg or D-Arg, $X_{b5}$ is Lys or is absent, $X_{b6}$ is Val, D-Val, Ala or is absent, $X_{b7}$ is Ala or is absent, $X_{b8}$ is Ala or is absent, and $X_{b9}$ is Gln, Asn, D-Gln or is absent.

14. The compound according to claim 13 or a physiologically compatible salt thereof, wherein $X_{bb}$ is -Pro-Arg-Lys-Val, -Pro-Arg-Lys, -Pro-Arg, -Ala-Gln, -Ala-Ala, -Pro, -Lys-Val, -Val-Pro-Tyr, -Arg-Lys, -Val-Ala, -Pro-Arg-Lys-Val-Ala, -Lys-Val-Ala-Ala-Gln, -Pro-Arg-Lys-Val-Ala-Ala-Gln, -Pro-A rg-Lys-Val-Ala-Ala-Gln, -Pro-Ala-Lys-Val-Ala-Ala-Gln, -Pro-Arg-Ala-Val-Ala-Ala-Gln, -Pro-Arg-Lys-Val-Ala-Ala-Ala, -Gly-Arg-Lys-Val-Ala-Ala-Gln, -D-Pro-Arg-Lys-Val-Ala-Ala-Gln, -Pro-Arg-D-Lys-Val-Ala-Ala-Gln, -Pro-Arg-Lys-Val-D-Ala-Ala-Gln, -Pro-Arg-Lys-Val-Ala-D-Ala-Gln, -Ser-Ser-Glu-Asp-Ile-Lys-Glu, -Pro-Arg-Lys-Val-Ala-Ala-Asn, -Pro-Arg-Lys-Val-Ala-Ala-Gln, -Pro-Arg-Lys-D-Val-Ala-Ala-Gln, -Ala-Arg-Lys-Val-Ala-Ala-Gln, -Pro-Arg-Lys-Ala-Ala-Ala-Gln, -Pro-Arg-Lys-Val-Ala-Ala-Gln, -Pro-D-Arg-Lys-Val-Ala-Ala-Gln, -Pro-Arg-Lys-Val-Ala-Ala-D-Gln or is absent, wherein the leftmost connector - indicates linkage to $X_{b1}$-$X_{b2}$.

**15.** The compound according to claim 13 or a physiologically compatible salt thereof, wherein $X_b$ is $^{**}$-$X_{b1}$-$X_{b2}$-$X_{bb}$, wherein $^{**}$ indicates the position linked to $Z_1$-$Z_2$-$Z_3$-$Z_4$, $X_{b1}$-$X_{b2}$- is $^{**}$-Ala-Lys-, and $X_{bb}$ is as defined above.

**16.** The compound according to claim 1 or a physiologically compatible salt thereof, wherein $X_b$ is $^{**}$-Ala-, $^{**}$-Ala-Lys, $^{**}$-Ala-D-Lys-, $^{**}$-Ala-Ala-, $^{**}$-D-Ala-Lys, $^{**}$-Ala-Arg, $^{**}$-Ala-Lys-Pro-Arg-Lys-Val, $^{**}$-Ala-Lys-Pro-Arg-Lys, $^{**}$-Ala-Lys-Pro-Arg, $^{**}$-Val-Ala-Ala-Gln, $^{**}$-Lys-Val-Ala-Ala, $^{**}$-Ala-Lys-Pro, $^{**}$-Pro-Arg-Lys-Val, $^{**}$-Ala-Lys-Val-Pro-Tyr, $^{**}$-Lys-Pro-Arg-Lys, $^{**}$-Arg-Lys-Val-Ala, $^{**}$-Ala-Lys-Pro-Arg-Lys-Val-Ala, $^{**}$-Pro-Arg-Lys-Val-Ala-Ala-Gln, $^{**}$-Ala-Lys-Pro-Arg-Lys-Val-Ala-Ala-Gln, $^{**}$-Ala-Ala-Pro-Arg-Lys-Val-Ala-Ala-Gln, $^{**}$-Ala-Lys-Pro-Ala-Lys-Val-Ala-Ala-Gln, $^{**}$-Ala-Lys-Pro-Arg-Ala-Val-Ala-Ala-Gln, $^{**}$-Ala-Lys-Pro-Arg-Lys-Val-Ala-Ala-Ala, $^{**}$-Ala-Lys-Gly-Arg-Lys-Val-Ala-Ala-Gln, $^{**}$-Ala-Lys-D-Pro-Arg-Lys-Val-Ala-Ala-Gln, $^{**}$-Ala-Lys-Pro-Arg-D-Lys-Val-Ala-Ala-Gln, $^{**}$-Ala-Lys-Pro-Arg-Lys-Val-D-Ala-Ala-Gln, $^{**}$-Ala-Lys-Pro-Arg-Lys-Val-Ala-D-Ala-Gln, $^{**}$-Ala-Lys-Ser-Ser-Glu-Asp-Ile-Lys-Glu, $^{**}$-Ala-Lys-Pro-Arg-Lys-Val-Ala-Ala-Asn, $^{**}$-Ala-D-Lys-Pro-Arg-Lys-Val-Ala-Ala-Gln, $^{**}$-Ala-Lys-Pro-Arg-Lys-D-Val-Ala-Ala-Gln, $^{**}$-Ala-Lys-Ala-Arg-Lys-Val-Ala-Ala-Gln, $^{**}$-Ala-Lys-Pro-Arg-Lys-Ala-Ala-Ala-Gln, $^{**}$-D-Ala-Lys-Pro-Arg-Lys-Val-Ala-Ala-Gln, $^{**}$-Ala-Lys-Pro-D-Arg-Lys-Val-Ala-Ala-Gln, $^{**}$-Ala-Lys-Pro-Arg-Lys-Val-Ala-Ala-D-Gln, or is absent, wherein $^{**}$ indicates the position linked to $Z_1$-$Z_2$-$Z_3$-$Z_4$.

**17.** The compound according to claim 1 or a physiologically compatible salt thereof, wherein the compound is selected from:

Lys-Glu-Pro-Val-Pro-Gln-Ala-Lys-Pro-Arg-Lys-Val-Ala-Ala-Gln (Compound 1);

Lys-Glu-Pro-Val-Pro-Gln-Ala-Lys-Pro-Arg-Lys (Compound 2);

Lys-Glu-Pro-Val-Pro-Gln-Ala-Lys-Pro-Arg (Compound 3);

Lys-Glu-Pro-Val-Pro-Gln-Ala-Lys (Compound 4);

Pro-Arg-Lys-Val-Ala-Ala-Gln (Compound 5);

Glu-Pro-Val-Pro-Gln-Ala-Lys (Compound 6);

Lys-Glu-Pro-Val (Compound 7);

Val-Ala-Ala-Gln (Compound 8);

Glu-Pro-Val-Pro (Compound 9);

Lys-Pro-Arg-Lys (Compound 10);

Ala-Lys-Pro-Arg (Compound 11);

Pro-Val-Pro-Gln (Compound 12);

Val-Pro-Gln-Ala (Compound 13);

Pro-Gln-Ala-Lys (Compound 14);

Arg-Lys-Val-Ala (Compound 15);

Lys-Val-Ala-Ala (Compound 16);

Gln-Ala-Lys-Pro (Compound 17);

Pro-Arg-Lys-Val (Compound 18);

Ala-Glu-Pro-Val-Pro-Gln-Ala-Lys-Pro-Arg-Lys-Val-Ala-Ala-Gln (Compound 19);

Lys-Ala-Pro-Val-Pro-Gln-Ala-Lys-Pro-Arg-Lys-Val-Ala-Ala-Gln (Compound 20);

Lys-Glu-Ala-Val-Pro-Gln-Ala-Lys-Pro-Arg-Lys-Val-Ala-Ala-Gln            (Compound 21);

Lys-Glu-Pro-Val-Ala-Gln-Ala-Lys-Pro-Arg-Lys-Val-Ala-Ala-Gln            (Compound 22);

Lys-Glu-Pro-Val-Pro-Ala-Ala-Lys-Pro-Arg-Lys-Val-Ala-Ala-Gln            (Compound 23);

Lys-Glu-Pro-Val-Pro-Gln-Ala-Ala-Pro-Arg-Lys-Val-Ala-Ala-Gln            (Compound 24);

Lys-Glu-Pro-Ala-Pro-Gln-Ala-Lys-Pro-Arg-Lys-Val-Ala-Ala-Gln            (Compound 25);

Lys-Glu-Pro-Val-Pro-Gln-Ala-Lys-Ala-Arg-Lys-Val-Ala-Ala-Gln            (Compound 26);

Lys-Glu-Pro-Val-Pro-Gln-Ala-Lys-Pro-Ala-Lys-Val-Ala-Ala-Gln            (Compound 27);

Lys-Glu-Pro-Val-Pro-Gln-Ala-Lys-Pro-Arg-Ala-Val-Ala-Ala-Gln            (Compound 28);

Lys-Glu-Pro-Val-Pro-Gln-Ala-Lys-Pro-Arg-Lys-Ala-Ala-Ala-Gln            (Compound 29);

Lys-Glu-Pro-Val-Pro-Gln-Ala-Lys-Pro-Arg-Lys-Val-Ala-Ala-Ala            (Compound 30);

Lys-Glu-Gly-Val-Pro-Gln-Ala-Lys-Pro-Arg-Lys-Val-Ala-Ala-Gln            (Compound 31);

Lys-Glu-Pro-Val-Gly-Gln-Ala-Lys-Pro-Arg-Lys-Val-Ala-Ala-Gln            (Compound 32);

Lys-Glu-Pro-Val-Pro-Gln-Ala-Lys-Gly-Arg-Lys-Val-Ala-Ala-Gln            (Compound 33);

Lys-Glu-Pro-Val-Gly-Gln-Ala-Lys-Gly-Arg-Lys-Val-Ala-Ala-Gln            (Compound 34);

Lys-Glu-Gly-Val-Gly-Gln-Ala-Lys-Gly-Arg-Lys-Val-Ala-Ala-Gln            (Compound 35);

Lys-Glu-Gly-Val-Pro-Gln-Ala-Lys-Gly-Arg-Lys-Val-Ala-Ala-Gln            (Compound 36);

Lys-Glu-Gly-Val-Gly-Gln-Ala-Lys-Pro-Arg-Lys-Val-Ala-Ala-Gln            (Compound 37);

Glu-Pro-Val-Pro-Gln-Ala-Lys-Pro-Arg-Lys            (Compound 38);

Pro-Val-Pro-Gln-Ala            (Compound 39);

Pro-Val-Pro-Glu-Ala            (Compound 40);

Pro-Val-Pro-Ile-Ala            (Compound 41);

Pro-Val-Pro-Ala            (Compound 42);

Glu-Pro-Val-Pro-Gln-Ala            (Compound 43);

Pro-Val-Pro-Gln-Ala-Lys            (Compound 44);

Glu-Pro-Val-Pro-Gln-Ala-Arg            (Compound 45);

Ala-Pro-Val-Pro-Gln-Ala-Lys            (Compound 46);

Glu-Pro-Val-Pro-Ala-Ala-Lys            (Compound 47);

Glu-Pro-Val-Pro-Gln-Ala-Ala            (Compound 48);

Asp-Pro-Val-Pro-Gln-Ala-Lys            (Compound 49);

Asn-Pro-Val-Pro-Gln-Ala-Lys                (Compound 50);

Glu-Pro-Val-Pro-Gln-Ala-Lys-Pro-Arg-Lys-Val-Ala                (Compound 51);

Glu-Pro-Ala-Pro-Gln-Ala-Lys                (Compound 52);

Glu-Ala-Val-Pro-Gln-Ala-Lys                (Compound 53);

Glu-Pro-Val-Ala-Gln-Ala-Lys                (Compound 54);

Glu-Pro-Val-Pro-Glu-Ala-Lys                (Compound 55);

Glu-Pro-Val-Pro-Gln-Ala-Lys-Pro-Arg-Lys-Val                (Compound 56);

Glu-Pro-Val-Pro-Gln-Ala-Lys-Pro-Arg                (Compound 57);

Glu-Pro-Val-Pro-Gln-Ala-Lys-Pro                (Compound 58);

Pro-Val-Pro-Gln-Ala-Lys-Pro-Arg                (Compound 59);

Pro-Val-Pro-Gln-Ala-Lys-Pro                (Compound 60);

D-Lys-Glu-Pro-Val-Pro-Gln-Ala-Lys-Pro-Arg-Lys-Val-Ala-Ala-Gln                (Compound 61);

Lys-D-Glu-Pro-Val-Pro-Gln-Ala-Lys-Pro-Arg-Lys-Val-Ala-Ala-Gln                (Compound 62);

Lys-Glu-D-Pro-Val-Pro-Gln-Ala-Lys-Pro-Arg-Lys-Val-Ala-Ala-Gln                (Compound 63);

Lys-Glu-Pro-D-Val-Pro-Gln-Ala-Lys-Pro-Arg-Lys-Val-Ala-Ala-Gln                (Compound 64);

Lys-Glu-Pro-Val-D-Pro-Gln-Ala-Lys-Pro-Arg-Lys-Val-Ala-Ala-Gln                (Compound 65);

Lys-Glu-Pro-Val-Pro-D-Gln-Ala-Lys-Pro-Arg-Lys-Val-Ala-Ala-Gln                (Compound 66);

Lys-Glu-Pro-Val-Pro-Gln-D-Ala-Lys-Pro-Arg-Lys-Val-Ala-Ala-Gln                (Compound 67);

Lys-Glu-Pro-Val-Pro-Gln-Ala-D-Lys-Pro-Arg-Lys-Val-Ala-Ala-Gln                (Compound 68);

Lys-Glu-Pro-Val-Pro-Gln-Ala-Lys-D-Pro-Arg-Lys-Val-Ala-Ala-Gln                (Compound 69);

Lys-Glu-Pro-Val-Pro-Gln-Ala-Lys-Pro-D-Arg-Lys-Val-Ala-Ala-Gln                (Compound 70);

Lys-Glu-Pro-Val-Pro-Gln-Ala-Lys-Pro-Arg-D-Lys-Val-Ala-Ala-Gln                (Compound 71);

Lys-Glu-Pro-Val-Pro-Gln-Ala-Lys-Pro-Arg-Lys-D-Val-Ala-Ala-Gln                (Compound 72);

Lys-Glu-Pro-Val-Pro-Gln-Ala-Lys-Pro-Arg-Lys-Val-D-Ala-Ala-Gln                (Compound 73);

Lys-Glu-Pro-Val-Pro-Gln-Ala-Lys-Pro-Arg-Lys-Val-Ala-D-Ala-Gln                (Compound 74);

Lys-Glu-Pro-Val-Pro-Gln-Ala-Lys-Pro-Arg-Lys-Val-Ala-Ala-D-Gln                (Compound 75);

Glu-Gly-Val-Pro-Gln-Ala-Lys                (Compound 76);

Glu-Pro-Val-Gly-Gln-Ala-Lys                (Compound 77);

D-Glu-Pro-Val-Pro-Gln-Ala-Lys                (Compound 78);

Glu-D-Pro-Val-Pro-Gln-Ala-Lys (Compound 79);

Glu-Pro-D-Val-Pro-Gln-Ala-Lys (Compound 80);

Glu-Pro-Val-D-Pro-Gln-Ala-Lys (Compound 81);

Glu-Pro-Val-Pro-D-Gln-Ala-Lys (Compound 82);

Glu-Pro-Val-Pro-Gln-D-Ala-Lys (Compound 83);

Glu-Pro-Val-Pro-Gln-Ala-D-Lys (Compound 84);

Lys-Glu-Pro-Val-Pro (Compound 85);

Glu-Pro-Val-Pro-Gln (Compound 86);

Lys-Glu-Pro-Val-Pro-Gln (Compound 87);

Val-Pro-Tyr-Lys-Glu-Pro-Val-Pro-Gln-Ala-Lys-Pro-Arg-Lys-Val-Ala-Ala-Gln (Compound 88);

Pyro-Glu-Leu-Lys-Glu-Pro-Val-Pro-Gln-Ala-Lys-Pro-Arg-Lys-Val-Ala-Ala-Gln (Compound 89);

Pro-Ala-Tyr-Lys-Glu-Pro-Val-Pro-Gln-Ala-Lys-Pro-Arg-Lys-Val-Ala-Ala-Gln (Compound 90);

Arg-Lys-Asp-Val-Tyr-Lys-Glu-Pro-Val-Pro-Gln-Ala-Lys-Pro-Arg-Lys-Val-Ala-Ala-Gln (Compound 91);

Arg-Lys-Asp-Val-Tyr-Lys-Glu-Pro-Val-Pro-Gln-Ala-Lys (Compound 92);

Arg-Lys-Asp-Val-Tyr-Glu-Pro-Val-Pro-Gln-Ala-Lys (Compound 93);

Arg-Lys-Asp-Val-Tyr-Pro-Val-Pro-Gln (Compound 94);

Gly-Pro-Glu-Thr-Ala-Phe-Leu-Arg-Lys-Glu-Pro-Val-Pro-Gln-Ala-Lys-Pro-Arg-Lys-Val-Ala-Ala-Gln (Compound 95);

Gly-Pro-Glu-Thr-Ala-Phe-Leu-Arg-Glu-Pro-Val-Pro-Gln-Ala-Lys (Compound 96);

Ser-Ser-Glu-Asp-Ile-Lys-Glu-Lys-Glu-Pro-Val-Pro-Gln-Ala-Lys-Pro-Arg-Lys-Val-Ala-Ala-Gln (Compound 97);

Ser-Ser-Glu-Asp-Ile-Lys-Glu-Glu-Pro-Val-Pro-Gln-Ala-Lys (Compound 98);

Ser-Ser-Glu-Asp-Ile-Lys-Glu-Pro-Val-Pro-Gln (Compound 99);

Val-Pro-Pro-Lys-Glu-Pro-Val-Pro-Gln-Ala-Lys-Pro-Arg-Lys-Val-Ala-Ala-Gln (Compound 100);

Glu-Pro-Val-Pro-Gln-Ala-Lys-Ser-Ser-Glu-Asp-Ile-Lys-Glu (Compound 101);

Glu-Pro-Val-Pro-Gln-Ala-Lys-Val-Pro-Tyr (Compound 102);

Lys-Glu-Pro-Val-Pro-Gln-Ala-Lys-Pro-Arg-Lys-Val-Ala-Ala-Glu (Compound 103);

Lys-Glu-Pro-Val-Pro-Gln-Ala-Lys-Pro-Arg-Lys-Val-Ala-Ala-Asn      (Compound 104);

Lys-Gln-Pro-Val-Pro-Gln-Ala-Lys-Pro-Arg-Lys-Val-Ala-Ala-Gln      (Compound 105);

Lys-Glu-Pro-Ile-Pro-Gln-Ala-Lys-Pro-Arg-Lys-Val-Ala-Ala-Gln      (Compound 106);

Val-Pro-Gln-Ala      (Compound 107);

or

Val-Pro-Gln-Ala-Lys      (Compound 108).

**18.** The compound according to claim 1 or a physiologically compatible salt thereof, wherein the compound is selected from:

Lys-Glu-Pro-Val-Pro-Gln-Ala-Lys-Pro-Arg-Lys-Val-Ala-Ala-Gln      (Compound 1);

Lys-Glu-D-Pro-Val-Pro-Gln-Ala-Lys-Pro-Arg-Lys-Val-Ala-Ala-Gln      (Compound 63);

Gly-Pro-Glu-Thr-Ala-Phe-Leu-Arg-Glu-Pro-Val-Pro-Gln-Ala-Lys      (Compound 96);

Val-Pro-Pro-Lys-Glu-Pro-Val-Pro-Gln-Ala-Lys-Pro-Arg-Lys-Val-Ala-Ala-Gln      (Compound 100);

Lys-Glu-Pro-Val-Pro-Gln-Ala-Lys-Pro-Arg-Lys-Val-Ala-Ala-Asn      (Compound 104);

Glu-Pro-Val-Pro-Gln-Ala-Lys      (Compound 6);

Pro-Val-Pro-Gln      (Compound 12);

Glu-Pro-Val-Ala-Gln-Ala-Lys      (Compound 54);

Glu-Pro-Val-Pro-Gln-Ala-Lys-Pro-Arg      (Compound 57);

Lys-Glu-Pro-Val-Pro-Gln-Ala-D-Lys-Pro-Arg-Lys-Val-Ala-Ala-Gln      (Compound 68);

Lys-Glu-Pro-Val-Pro-Gln-Ala-Lys-Pro-Arg-Lys-D-Val-Ala-Ala-Gln      (Compound 72);

Pyro-Glu-Leu-Lys-Glu-Pro-Val-Pro-Gln-Ala-Lys-Pro-Arg-Lys-Val-Ala-Ala-Gln      (Compound 89);

Arg-Lys-Asp-Val-Tyr-Lys-Glu-Pro-Val-Pro-Gln-Ala-Lys      (Compound 92);

Arg-Lys-Asp-Val-Tyr-Pro-Val-Pro-Gln      (Compound 94);

Gly-Pro-Glu-Thr-Ala-Phe-Leu-Arg-Lys-Glu-Pro-Val-Pro-Gln-Ala-Lys-Pro-Arg-Lys-Val-Ala-Ala-Gln      (Compound 95);

Ser-Ser-Glu-Asp-Ile-Lys-Glu-Lys-Glu-Pro-Val-Pro-Gln-Ala-Lys-Pro-Arg-Lys-Val-Ala-Ala-Gln      (Compound 97);

Ser-Ser-Glu-Asp-Ile-Lys-Glu-Glu-Pro-Val-Pro-Gln-Ala-Lys      (Compound 98);

Lys-Gln-Pro-Val-Pro-Gln-Ala-Lys-Pro-Arg-Lys-Val-Ala-Ala-Gln      (Compound 105);

Lys-Glu-Pro-Ile-Pro-Gln-Ala-Lys-Pro-Arg-Lys-Val-Ala-Ala-Gln      (Compound 106);

Lys-Glu-Pro-Val-Pro-Gln-Ala-Lys        (Compound 4);

Pro-Arg-Lys-Val-Ala-Ala-Gln        (Compound 5);

Glu-Pro-Val-Pro        (Compound 9);

Lys-Pro-Arg-Lys        (Compound 10);

Arg-Lys-Val-Ala        (Compound 15);

Lys-Glu-Pro-Ala-Pro-Gln-Ala-Lys-Pro-Arg-Lys-Val-Ala-Ala-Gln        (Compound 25);

Lys-Glu-Pro-Val-Pro-Gln-Ala-Lys-Ala-Arg-Lys-Val-Ala-Ala-Gln        (Compound 26);

Lys-Glu-Pro-Val-Pro-Gln-Ala-Lys-Pro-Arg-Lys-Ala-Ala-Ala-Gln        (Compound 29);

Lys-Glu-Gly-Val-Pro-Gln-Ala-Lys-Pro-Arg-Lys-Val-Ala-Ala-Gln        (Compound 31);

Lys-Glu-Pro-Val-Gly-Gln-Ala-Lys-Gly-Arg-Lys-Val-Ala-Ala-Gln        (Compound 34);

Glu-Pro-Val-Pro-Gln-Ala-Lys-Pro-Arg-Lys        (Compound 38);

Glu-Pro-Val-Pro-Gln-Ala-Ala        (Compound 48);

Asp-Pro-Val-Pro-Gln-Ala-Lys        (Compound 49);

Asn-Pro-Val-Pro-Gln-Ala-Lys        (Compound 50);

Glu-Pro-Val-Pro-Gln-Ala-Lys-Pro-Arg-Lys-Val-Ala        (Compound 51);

Glu-Ala-Val-Pro-Gln-Ala-Lys        (Compound 53);

Glu-Pro-Val-Pro-Glu-Ala-Lys        (Compound 55);

Lys-D-Glu-Pro-Val-Pro-Gln-Ala-Lys-Pro-Arg-Lys-Val-Ala-Ala-Gln        (Compound 62);

Lys-Glu-Pro-Val-D-Pro-Gln-Ala-Lys-Pro-Arg-Lys-Val-Ala-Ala-Gln        (Compound 65);

Lys-Glu-Pro-Val-Pro-D-Gln-Ala-Lys-Pro-Arg-Lys-Val-Ala-Ala-Gln        (Compound 66);

Lys-Glu-Pro-Val-Pro-Gln-D-Ala-Lys-Pro-Arg-Lys-Val-Ala-Ala-Gln        (Compound 67);

Lys-Glu-Pro-Val-Pro-Gln-Ala-Lys-Pro-D-Arg-Lys-Val-Ala-Ala-Gln        (Compound 70);

Lys-Glu-Pro-Val-Pro-Gln-Ala-Lys-Pro-Arg-Lys-Val-Ala-Ala-D-Gln        (Compound 75);

Glu-Pro-Val-Pro-Gln-D-Ala-Lys        (Compound 83);

Lys-Glu-Pro-Val-Pro        (Compound 85);

Glu-Pro-Val-Pro-Gln        (Compound 86);

Val-Pro-Tyr-Lys-Glu-Pro-Val-Pro-Gln-Ala-Lys-Pro-Arg-Lys-Val-Ala-Ala-Gln        (Compound 88);

Pro-Ala-Tyr-Lys-Glu-Pro-Val-Pro-Gln-Ala-Lys-Pro-Arg-Lys-Val-Ala-Ala-Gln        (Compound 90);

Arg-Lys-Asp-Val-Tyr-Glu-Pro-Val-Pro-Gln-Ala-Lys        (Compound 93);

Ser-Ser-Glu-Asp-Ile-Lys-Glu-Pro-Val-Pro-Gln        (Compound 99);

or

Lys-Glu-Pro-Val-Pro-Gln-Ala-Lys-Pro-Arg-Lys-Val-Ala-Ala-Glu        (Compound 103).

19. Use of the compound according to any one of claims 1-18 or a physiologically compatible salt thereof in the preparation of a drug for treating enteritis.

20. The use according to claim 19, wherein the enteritis includes specific enteritis and nonspecific enteritis; further, the specific enteritis includes inflammatory bowel disease, necrotic enteropathy and dysbacteriosis enteritis; and further, the inflammatory bowel disease includes ulcerative colitis.

21. A pharmaceutical composition comprising the compound according to any one of claims 1-18 or a physiologically compatible salt thereof, and a pharmaceutically acceptable excipient.

**Fig. 1**

**Fig. 2**

## INTERNATIONAL SEARCH REPORT

International application No.

**PCT/CN2023/073068**

### A. CLASSIFICATION OF SUBJECT MATTER

C07K 14/00(2006.01)i;C07K 5/00(2006.01)i;C07K 7/00(2006.01)i;C07K
11/00(2006.01)i;A61K38/00(2006.01)i;A61P1/00(2006.01)i;A61P1/04(2006.01)i

According to International Patent Classification (IPC) or to both national classification and IPC

### B. FIELDS SEARCHED

Minimum documentation searched (classification system followed by classification symbols)

IPC: C07K A61K A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统, China Patent Biological Sequence Search System, Genbank, EMBL, STN and search terms: 炎症性肠病, inflammatory bowel disease, IBD, Lys-Glu-Pro-Val-Pro-Gln-Ala-Lys-Pro-Arg-Lys-Val-Ala-Ala-Gln, KEPVPQAKPRKVAAQ

### C. DOCUMENTS CONSIDERED TO BE RELEVANT

| Category* | Citation of document, with indication, where appropriate, of the relevant passages | Relevant to claim No. |
|---|---|---|
| A | CN 108210886 A (SHANGHAI FENGJIN BIOMEDICAL TECHNOLOGY CO., LTD.) 29 June 2018 (2018-06-29)<br>see entire document, in particular the claims and embodiments | 1-21 (in part) |
| A | CN 108478800 A (SHEN, Dong) 04 September 2018 (2018-09-04)<br>see entire document, in particular the claims and embodiments | 1-21 (in part) |
| A | CN 110090295 A (WUHAN KANGLITONG TECHNOLOGY CO., LTD.) 06 August 2019 (2019-08-06)<br>see entire document, in particular the claims and embodiments | 1-21 (in part) |
| A | CN 111542335 A (STEMRIM INC; UNIVERSITY OSAKA) 14 August 2020 (2020-08-14)<br>see entire document, in particular the claims and embodiments | 1-21 (in part) |
| A | CN 112679589 A (SHANGHAI LONGXIN BIOMEDICAL TECHNOLOGY CO., LTD.) 20 April 2021 (2021-04-20)<br>see entire document, in particular the claims and embodiments | 1-21 (in part) |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 April 2023** | **28 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.

**PCT/CN2023/073068**

**C.     DOCUMENTS CONSIDERED TO BE RELEVANT**

| Category* | Citation of document, with indication, where appropriate, of the relevant passages | Relevant to claim No. |
|---|---|---|
| A | CN 112812158 A (SHANGHAI LONGXIN BIOMEDICAL TECHNOLOGY CO., LTD.) 18 May 2021 (2021-05-18)<br>    see entire document, in particular the claims and embodiments | 1-21 (in part) |
| A | CN 112851774 A (SHANGHAI LONGXIN BIOMEDICAL TECHNOLOGY CO., LTD.) 28 May 2021 (2021-05-28)<br>    see entire document, in particular the claims and embodiments | 1-21 (in part) |

Form PCT/ISA/210 (second sheet) (July 2022)

**INTERNATIONAL SEARCH REPORT**

International application No.

**PCT/CN2023/073068**

| Box No. III    Observations where unity of invention is lacking (Continuation of item 3 of first sheet) |
| --- |

This International Searching Authority found multiple inventions in this international application, as follows:

Inventions 1-91: claims 1-21 (in part)

relates to compounds 1, 4-12, 14-17, 19, 21-34, 36-42, 46-55, 57-59, 62-72, 74-76, 78-100 and 102-106, and corresponding salts, pharmaceutical compositions and pharmaceutical use.

First, the embodiments of the description of the present application verify that compounds 1, 4-6, 9, 10, 12, 15, 25, 26, 29, 31, 34, 38, 48-51, 53-55, 57, 62, 63, 65-68, 70, 72, 75, 83, 85, 86, 88-90, 92-100 and 103-106 all have the efficacy of significantly relieving colitis, and the specific expression is to relieve intestinal cavity expansion (P ≤ 0.05); and compounds 7, 8, 11, 14, 16, 17, 19, 21-24, 27, 28, 30, 32, 33, 36, 37, 39-42, 46, 47, 52, 58, 59, 64, 69, 71, 74, 76, 78-82, 84, 87, 91 and 102 have a certain effect of relieving intestinal cavity expansion (see description, embodiments). Therefore, 91 compounds have the effect, which form 91 groups of inventions. Although partially common amino acid sequences exist in polypeptides of inventions 1-91, on one hand, according to what is recited in the description of the present application and the prior art, it is difficult for a person skilled in the art to expect that the above-mentioned common amino acid sequence parts are essential to the common performance or effects of treating enteritis, and the present application cannot prove that the joint effect of these similar peptides is brought by these partially common amino acid sequences. Therefore, partially same amino acid sequence segments between peptide monomers do not constitute the specific technical feature of the multiple groups of peptide monomer inventions mentioned above that makes a contribution over the prior art. On the other hand, the common feature of the polypeptides of inventions 1-91 is "treating enteritis"; however, the above-mentioned common feature is disclosed in D1 (CN 111542335 A, 14 August 2020, see claims). Therefore, the technical solutions of inventions 1-91 do not belong to a single general inventive concept, do not have any technical relationship therebetween, do not have the same or corresponding special technical features, lack unity of invention, and thus do not comply with PCT Rules 13.1 and 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-21 (in part, relating to compound 1)**

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

INTERNATIONAL SEARCH REPORT
Information on patent family members

International application No.

**PCT/CN2023/073068**

| Patent document cited in search report | | | Publication date (day/month/year) | Patent family member(s) | | | Publication date (day/month/year) |
|---|---|---|---|---|---|---|---|
| CN | 108210886 | A | 29 June 2018 | None | | | |
| CN | 108478800 | A | 04 September 2018 | None | | | |
| CN | 110090295 | A | 06 August 2019 | None | | | |
| CN | 111542335 | A | 14 August 2020 | JPWO | 2019107530 | A1 | 26 November 2020 |
| | | | | US | 2020384074 | A1 | 10 December 2020 |
| | | | | US | 11298403 | B2 | 12 April 2022 |
| | | | | EP | 3718561 | A1 | 07 October 2020 |
| | | | | EP | 3718561 | A4 | 21 July 2021 |
| | | | | WO | 2019107530 | A1 | 06 June 2019 |
| CN | 112679589 | A | 20 April 2021 | None | | | |
| CN | 112812158 | A | 18 May 2021 | None | | | |
| CN | 112851774 | A | 28 May 2021 | None | | | |

Form PCT/ISA/210 (patent family annex) (July 2022)